# EUROPEAN PATENT APPLICATION

(11) **EP 1 787 979 A1**
(43) Date of publication of application: **23.05.2007**
(21) Application number: 05781598.7
(22) Date of filing: 05.09.2005
(51) Int. Cl.: C07C 311/00

(54) **SULFONAMIDE DERIVATIVE SELECTIVELY INHIBITING MMP-13**

(30) Priority: 06.09.2004 JP 2004258306
(71) Applicant: Shionogi Co., Ltd., Osaka-shi, Osaka 541-0045 (JP)
(72) Inventor: WATANABE, Fumihiko c/o Shionogi & Co., Ltd., Osaka 553 0002 (JP); TAMURA, Yoshinori c/o Shionogi & Co., Ltd., Osaka 5530 002 (JP); YOSHIKAWA, Naoki c/o Shionogi & Co., Ltd., Osaka 55300 02 (JP)
(74) Representative: Vossius & Partner
(86) International application number: PCT/JP2005/016231
(87) International publication number: WO 2006/028038

(57) **Abstract**

It is considered that MMP-13 inhibitors contribute to the treatment or prevention against the diseases caused by or related to activity of MMP-13, especially osteoarthritis (OA). Therefore, the development of MMP-13 inhibitors has been desired.

A compound represented by the general formula (I): 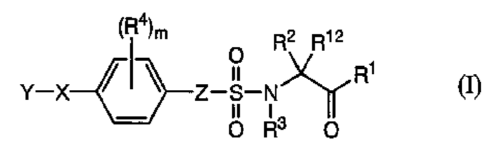 wherein Z is 1,4-phenylene and the like; R¹ is hydroxy and the like; R² is hydrogen atom, optionally substituted lower alkyl, and the like; R¹² is hydrogen atom; or R² and R¹² taken together with the adjacent carbon atom may form a ring; R³ is hydrogen atom, optionally substituted lower alkyl, and the like; R⁴ is halogen, lower alkyl, and the like; m is 0, 1, or 2; X is a bond, -C≡C-, and the like; Y is optionally substituted phenyl, optionally substituted naphthyl, and the like, its optically active substance, their pharmaceutically acceptable salt, or a solvate thereof.

## Description

### Technical Field

This invention relates to sulfonamide derivatives selectively inhibiting MMP-13.

### Background Art

An extracellular matrix, consisting of collagen, fibronectin, laminin, proteoglycan, etc., has a function to support tissues, and plays a role in proliferation, differentiation, adhesion, and the like in cells. Metalloproteinases, which have a metal ion in the active center, especially matrix metalloproteinases (MMP), are concerned with the degradation of the extracellular matrix. Many types of MMP, from MMP-1 (collagenase type I) to MMP-23, have been reported as enzymes working for the growth, remodeling of tissues, etc. under usual physiological conditions. It is reported, however, that the progression of various kinds of diseases involving breakdown or fibrosis of tissues (e.g., osteoarthritis, rheumatoid arthritis, corneal ulceration, periodontitis, metastasis and invasion of tumor, and virus infection (HFV infection)) is related with increase of the expression or activity of the above-mentioned enzyme.
Sulfonamide derivatives having an inhibitory activity against MMP are described in Patent Document 1, Patent Document 2, Patent Document 3, Non Patent Document 1, and the like.
Sulfonamide derivatives having an inhibitory activity against MMP-12 and having a naphthyl group as a substituent are described in Patent Document 4.
Thiazole or oxazole sulfonamide derivatives having a naphthyl group as a substituent are described in Patent Documents 5.
Thiazole or oxazole sulfonamide derivatives having a naphthyl group as a substituent are described in Patent Documents 6.
Sulfonamide derivatives having an inhibitory activity against aggrecanase and having a naphthyl group as a substituent are described in Patent Document 7.
Sulfonamide derivatives having an inhibitory activity against aggrecanase and an inhibitory activity against MMP-13 are described in Patent Document 8.
Patent Document 1: International Publication WO 97/27174
Patent Document 2: International Publication WO 99/04780
Patent Document 3: International Publication WO 00/15213
Patent Document 4: International Publication WO 01/83431
Patent Document 5: International Publication WO 02/28844
Patent Document 6: International Publication WO 01/83461
Patent Document 7: International Publication WO 03/080042
Patent Document 8: Japanese Patent Publication (Kokai) 2000-319250
Non Patent Document 1: Tamura Y., et al., J. Med. Chem., 41(4), 640-649, 1998.

### Disclosure of Invention

### Problems to be solved by the Invention

It is considered that MMP-13 inhibitors contribute to the treatment or prevention against the diseases caused by or related to activity of MMP-13, especially osteoarthritis (OA). Therefore, the development of MMP-13 inhibitors has been desired.

### Means to Solve the Problems

In the above situation, the inventors of the present invention have found that certain biaryl sulfonamide derivatives have a selective inhibitory activity against MMP-13, and have accomplished the present invention.

### Effect of the invention

The present invention relates to 1) A compound represented by the general formula (I): 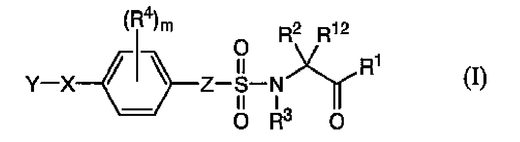 wherein Z is 1,4-phenylene or thiophen-2,5-diyl;
R¹ is hydroxy, lower alkyloxy, or -NHOH;
R² is hydrogen atom, optionally substituted lower alkyl, optionally substituted aralkyl, optionally substituted heteroarylalkyl, optionally substituted aryl, or optionally substituted heteroaryl;
R¹² is hydrogen atom; or
R² and R¹² taken together with the adjacent carbon atom may form a 5 to 6 membered non-aromatic carbocyclic group or a 5 to 6 membered non-aromatic heterocyclic group;
R³ is hydrogen atom, optionally substituted lower alkyl, optionally substituted aralkyl, optionally substituted heteroarylalkyl, optionally substituted aryl, or optionally substituted heteroaryl;
R⁴ is halogen, lower alkyl, cycloalkyl, lower alkenyl, lower alkynyl, lower alkyloxy, lower alkenyloxy, lower alkylthio, halo(lower)alkyl, halo(lower)alkyloxy, halo(lower)alkylthio, hydroxy, hydroxy(lower)alkyl, carboxy, lower alkyloxycarbonyl, lower alkylsulfonyl, acyl, acyloxy, nitro, cyano, optionally substituted amino, or optionally substituted aminocarbonyl;
m is 0, 1, or 2;
X is a bond, -CH₂CH₂-, -CH=CH-, -C≡C-, -(CH₂)ₚ1-C≡C-, -X¹-C(=O)-N(R⁵)-, -X²-N(R⁶)-C(=O)-, -X³-O-X⁴-, -SO-N(R⁹)-, or -N(R¹⁰)-C(=O)-N(R¹¹)-;
X¹ and X² are each independently a bond, -CH=CH-, or -C=C-;
X³ and X⁴ are each independently a bond or -(CH₂)ₚ²-;
R⁵, R⁶, R⁹, R¹⁰, and R¹¹ are each independently hydrogen atom or lower alkyl;
p¹ and p² are each independently an integer from 1 to 5;
Y is optionally substituted aryl, optionally substituted heteroaryl, an optionally substituted non-aromatic carbocyclic group, or an optionally substituted non-aromatic heterocyclic group; provided Y is not unsubstituted phenyl when R² is isopropyl or benzyl and X is a bond; Y is not unsubstituted naphthyl when R² is methyl or isopropyl and X is a bond; and Y is not unsubstituted naphthyl when R² is benzyl and X is -C≡C-;
its optically active substance, their pharmaceutically acceptable salt, or a solvate thereof.

In more detail, the invention relates to the following 2) to 34).
2) A compound according to 1) wherein the compound is represented by the general formula (II): 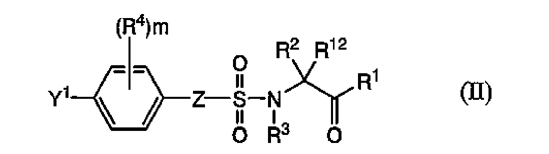 wherein R¹, R², R³, R⁴, R¹², Z, and m are as defined in 1);
Y¹ is a group represented by the formula: 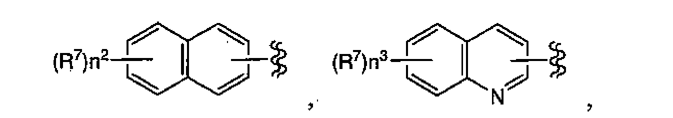 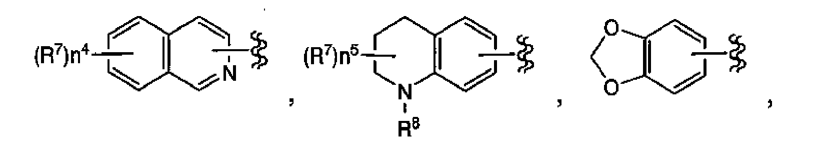 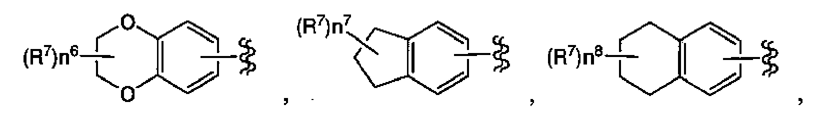 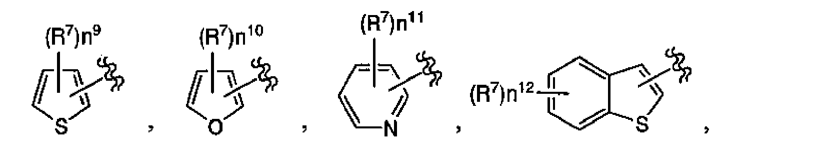 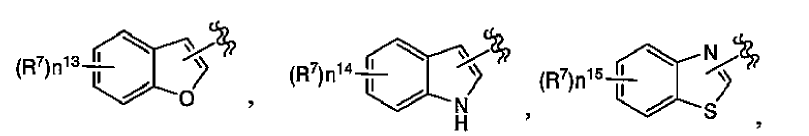 or 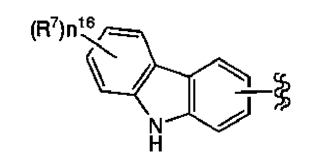 wherein R⁷ is halogen, lower alkyl, lower alkyloxy, cycloalkyl, lower alkenyl, lower alkynyl, lower alkenyloxy, lower alkylthio, halo(lower)alkyl, halo(lower)alkyloxy, halo(lower)alkylthio, hydroxy, hydroxy(lower)alkyl, carboxy, lower alkyloxycarbonyl, lower alkylsulfonyl, acyl, acyloxy, mercapto, nitro, cyano, optionally substituted amino, optionally substituted aminocarbonyl, optionally substituted aryl, optionally substituted heteroaryl, optionally substituted aryloxy, optionally substituted aralkyloxy, lower alkyloxy(lower)alkyl, optionally substituted aryloxy(lower)alkyl, optionally substituted ureide, optionally substituted pyrolizinyl, optionally substituted morpholinyl, or optionally substituted piperidyl,;
R⁸ is hydrogen atom or lower alkyl;
n² to n¹⁶ are each independently an integer from 0 to 3;
provided n² is an integer from 1 to 3 when R² is methyl or isopropyl,
its optically active substance, their pharmaceutically acceptable salt, or a solvate thereof.
When Y¹ is a condensed cyclic group, any atoms constituting a cyclic group of Y¹ and a carbon atom consisting a benzene ring can be bonded at any possible position. Further, Y¹ may be substituted with R⁷ as a substituent for Y¹ at any possible position.

3) A compound according to 1) wherein the compound is represented by the general formula (III): 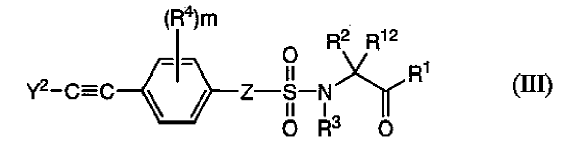 wherein R¹, R², R³, R⁴, R¹², Z, and m are as defined in 1);
Y² is a group represented by the formula: 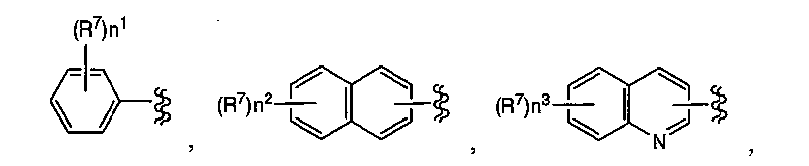 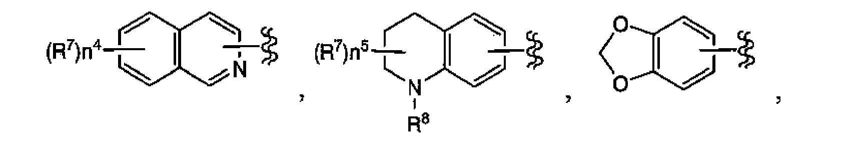 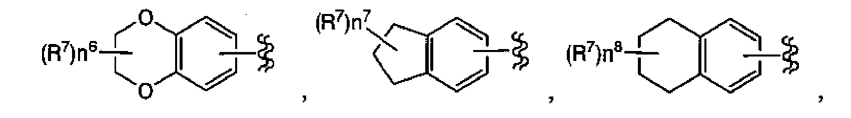 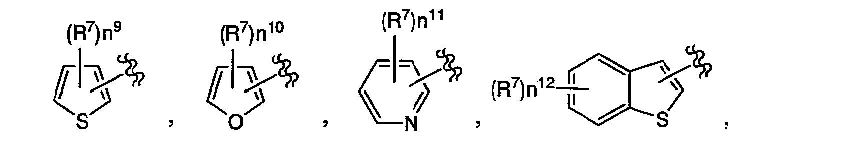 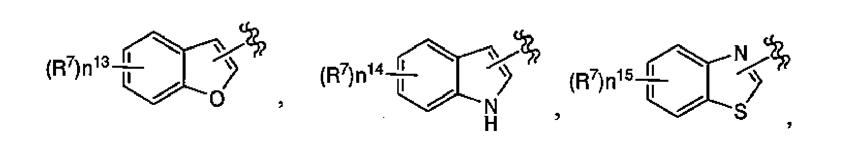 or 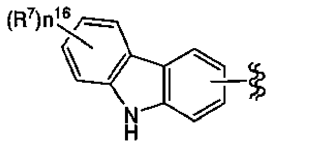 wherein R⁷, R⁸, and n¹ to n¹⁶ are as defined in 2);
provided n² is an integer from 1 to 3 when R² is benzyl,
its optically active substance, their pharmaceutically acceptable salt, or a solvate thereof.
When Y² is a condensed ring, any atoms consisting a cyclic group of Y² and a carbon atom attached to Y² can be bonded at any possible position. Further Y² may be substituted with R⁷ as a substituent for Y² at any possible position.

4) A compound according to 1) wherein the compound is represented by the general formula (IV): 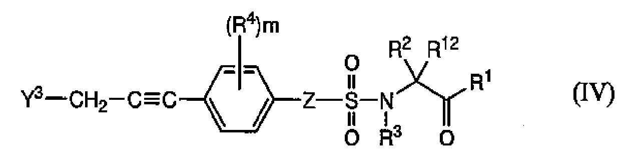 wherein R¹, R², R³, R⁴, R¹², Z, and m are as defined in 1);
Y³ is a group represented by the formula: 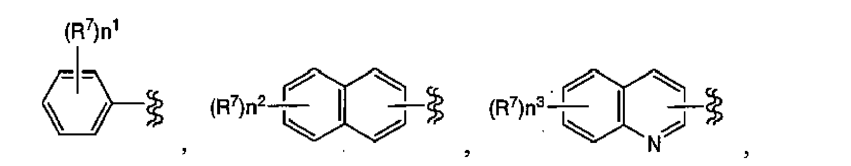 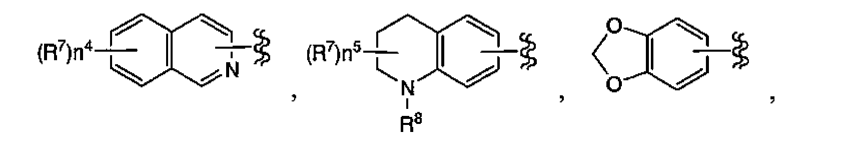 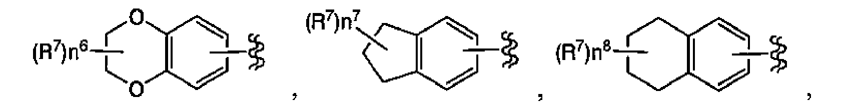 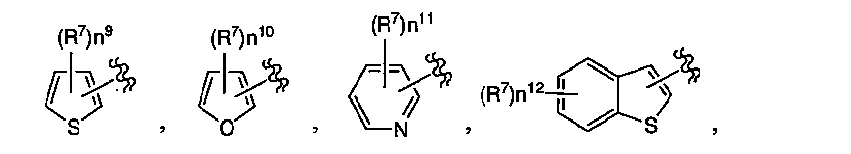 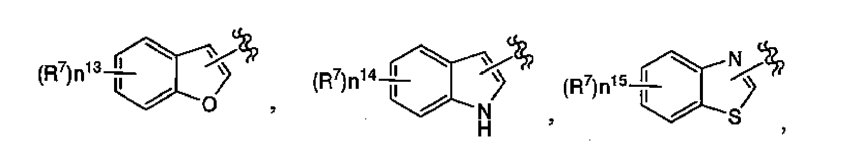 or 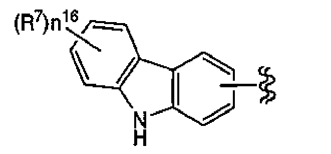 wherein R⁷, R⁸, and n¹ to n¹⁶ are as defined in 2),
its optically active substance, their pharmaceutically acceptable salt, or a solvate thereof.
When Y³ is a condensed ring, any atoms consisting a cyclic group of Y³ and a carbon atom of -(CH₂)p- group can be bonded at any possible position. Further Y³ may be substituted with R⁷ as a substituent for Y³ at any possible position.

5) A compound according to 1) wherein the compound is represented by the general formula (V): 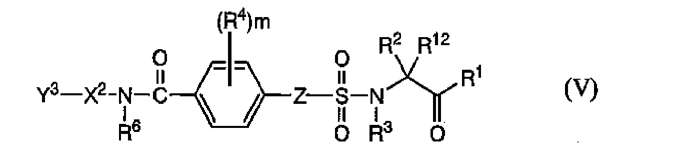 wherein R¹, R², R³, R⁴, R⁶, R¹², X², Z, and m are as defined in 1);
and Y³ is as defined in 4),
its optically active substance, their pharmaceutically acceptable salt, or a solvate thereof.
6) A compound according to 1) wherein the compound is represented by the general formula (VI): 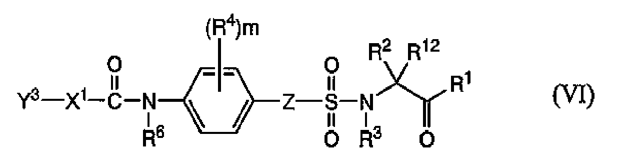 wherein R¹, R², R³, R⁴, R⁶, R¹², X¹, Z, and m are as defined in 1);
   and Y³ is as defined in 4),
   its optically active substance, their pharmaceutically acceptable salt, or a solvate thereof
7) A compound according to 1) wherein the compound is represented by the general formula (VII): 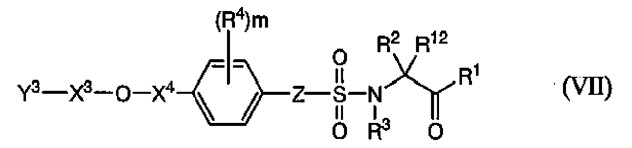 wherein R¹, R², R³, R⁴, R¹², X³, X⁴, Z, and m are as defined in 1);
   and Y³ is as defined in 4),
   its optically active substance, their pharmaceutically acceptable salt, or a solvate thereof
8) A compound according to 1) wherein the compound is represented by the general formula (VIII): 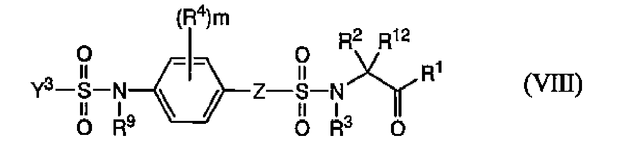 wherein R¹, R², R³, R⁴, R⁹, R¹², Z, and m are as defined in 1);
   and Y³ is as defined in 4),
   its optically active substance, their pharmaceutically acceptable salt, or a solvate thereof
9) A compound according to 1) wherein the compound is represented by the general formula (IX): 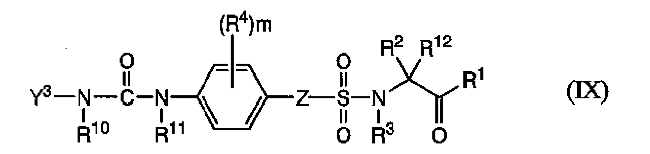 wherein R¹, R², R³, R⁴, R¹⁰, R¹¹, R¹², Z, and m are as defined in 1);
   and Y³ is as defined in 4),
   its optically active substance, their pharmaceutically acceptable salt, or a solvate thereof
10) A compound according to 1) wherein the compound is represented by the general formula (X): 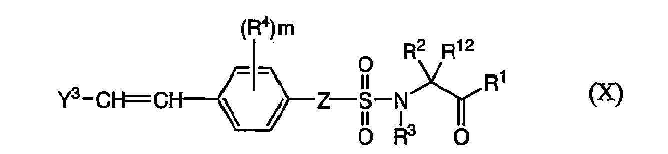 wherein R¹, R², R³, R⁴, R¹², Z, and m are as defined in 1);
   and Y³ is as defined in 4),
   its optically active substance, their pharmaceutically acceptable salt, or a solvate thereof
11) A compound according to 1) wherein the compound is represented by the general formula (XI): 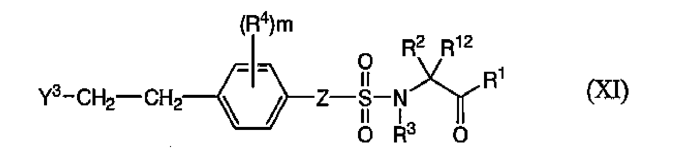 wherein R¹, R², R³, R⁴, R¹², Z, and m are as defined in 1);
   and Y³ is as defined in 4),
   its optically active substance, their pharmaceutically acceptable salt, or a solvate thereof
12) A compound according to any one of 1) to 11) wherein R¹ is hydroxy, its optically active substance, their pharmaceutically acceptable salt, or a solvate thereof.
13) A compound according to any one of 1) to 12) wherein R² is hydrogen atom, methyl, ethyl, n-propyl, isopropyl, n-butyl, s-butyl, isobutyl, t-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, mdol-3-ylmethyl, thiazol-4-ylmethyl, carboxymethyloxybenzyl, or cyclopropylmethyl;
   R¹² is hydrogen atom; or
   R² and R¹² taken together with the adjacent carbon atom may form a group represented by the formula: 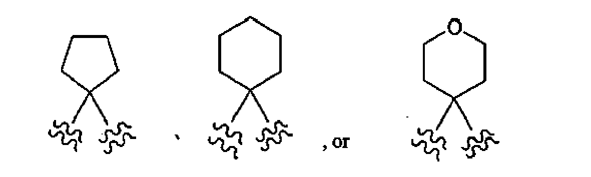 its optically active substance, their pharmaceutically acceptable salt, or a solvate thereof.
14) A compound according to any one of 1) to 13) wherein R² is methyl, isobutyl, hydroxymethyl, carboxymethyl, 4-fluorophenyl, 4-methyloxyphenyl, benzyl, or cyclopropylmethyl,
   its optically active substance, their pharmaceutically acceptable salt, or a solvate thereof.
15) A compound according to any one of 1) to 14) wherein Z is 1,4-phenylene, its optically active substance, their pharmaceutically acceptable salt, or a solvate thereof.
16) A compound according to any one of 1) to 14) wherein Z is thiophen-2,5-diyl, its optically active substance, their pharmaceutically acceptable salt, or a solvate thereof.
17) A compound according to any one of 1) to 16) wherein R⁴ is halogen; and m is 0 or 1, its optically active substance, their pharmaceutically acceptable salt, or a solvate thereof.
18) A compound according to any one of 1) to 17) wherein m is 0, its optically active substance, their pharmaceutically acceptable salt, or a solvate thereof.
19) A compound according to any one of 3) and 12) to 18) wherein Y² is a group represented by the formula: 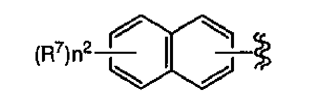 wherein R⁷ and n² are as defined in 2),
   its optically active substance, their pharmaceutically acceptable salt, or a solvate thereof.
20) A compound according to any one of 3) and 12) to 18) wherein Y² is a group represented by the formula: 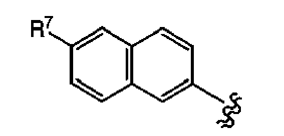 wherein R⁷ is lower alkyloxy,
   its optically active substance, their pharmaceutically acceptable salt, or a solvate thereof.
21) A compound according to any one of 4) to 18) wherein Y³ is a group represented by the formula: 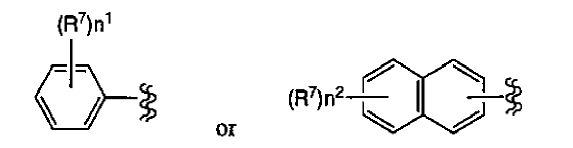 wherein R⁷, n¹, and n² are as defined in 2),
   its optically active substance, their pharmaceutically acceptable salt, or a solvate thereof.
22) A compound according to any one of 4) to 18) wherein Y³ is a group represented by the formula: 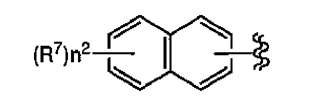 wherein R⁷ and n² are as defined in 2),
   its optically active substance, their pharmaceutically acceptable salt, or a solvate thereof.
23) A compound according to any one of 4) to 18) wherein Y³ is a group represented by the formula: 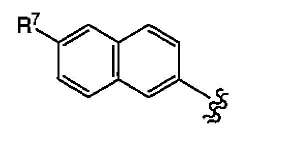 wherein R⁷ is hydrogen atom or lower alkyloxy,
   its optically active substance, their pharmaceutically acceptable salt, or a solvate thereof.
24) A compound according to any one of 5) and 12) to 18) wherein X² is a bond; and R⁶ is hydrogen atom, its optically active substance, their pharmaceutically acceptable salt, or a solvate thereof.
25) A compound according to any one of 6) and 12) to 18) wherein X¹ is a bond; and R⁶ is hydrogen atom, its optically active substance, their pharmaceutically acceptable salt, or a solvate thereof.
26) A compound according to any one of 7) and 12) to 18) wherein X³ is a bond; and X⁴ is -CH₂-; or X³ is -CH²- and X⁴ is a bond, its optically active substance, their pharmaceutically acceptable salt, or a solvate thereof.
27) A compound according to any one of 8) and 12) to 18) wherein R⁹ is hydrogen atom, its optically active substance, their pharmaceutically acceptable salt, or a solvate thereof.
28) A compound according to any one of 9) and 12) to 18) wherein R¹⁰ is hydrogen atom; and R¹¹ is hydrogen atom, its optically active substance, their pharmaceutically acceptable salt, or a solvate thereof.
29) A compound according to any one of 1) to 28) wherein R³ is hydrogen atom, its optically active substance, their pharmaceutically acceptable salt, or a solvate thereof.
30) A pharmaceutical composition which contains a compound of any one of 1) to 29) as an active ingredient.
31) An MMP-13 inhibitor which contains a compound of any one of 1) to 29) as an active ingredient.
32) An agent for treating osteoarthritis which contains a compound of any one of 1) to 29) as an active ingredient.
33) A method for treating a disease caused by or related to MMP-13 of a mammal comprising administering to said mammal including human a therapeutically effective amount of a compound of any one of 1) to 29).
34) Use of a compound of any one of 1) to 29) for the preparation of a medicament for treating a disease caused by or related to MMP-13.

In the present specification, the term "lower alkyl" employed alone or in combination with other terms means a straight- or branched chain monovalent hydrocarbon group having 1 to 8 carbon atom(s). Examples of the alkyl include methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl and the like. C₁ to C₆ alkyl is preferred. C₁ to C₃ alkyl is more preferred.
In the present specification, the term "cycloalkyl" includes cycloalkyl group having 3 to 8 carbon atoms. Examples of cycloalkyl group include cyclopropyl, cyclobutyl, cyclopentyl, cyclohexyl, cycloheptyl, cyclooctyl and the like. C₃ to C₆ cycloalkyl is preferred.
In the present specification, the term "lower alkenyl" employed alone or in combination with other terms means a straight- or branched chain monovalent hydrocarbon group having 2 to 8 carbon atoms and at least one double bond. Examples of the alkenyl include vinyl, allyl, propenyl, crotonyl, isopentenyl, a variety of butenyl isomers and the like. C₂ to C₆ alkenyl is preferred. C₂ to C₄ alkenyl is more preferred.
In the present specification, the term "cycloalkenyl" includes cycloalkenyl group having 3 to 8 carbon atoms and at least one double bond in the ring. Examples of cycloalkenyl group include cyclopropenyl, cyclobutenyl, cyclopentenyl, cyclohexenyl, cycloheptenyl, cyclooctenyl and the like. C₃ to C₆ cycloalkenyl is preferred.
In the present specification, the term "lower alkynyl" means a straight- or branched chain monovalent hydrocarbon group having 2 to 8 carbon atoms and at least one triple bond. The alkynyl may contain (a) double bond(s). Examples of the alkynyl include ethynyl, 2-propynyl, 3-butynyl, 4-pentynyl, 5-hexynyl, 6-heptynyl, 7-octynyl and the like. C₂ to C₆ alkynyl is preferred. C₂ to C₄ alkynyl is more preferred.
In the present specification, the term "cycloalkynyl" includes cycloalkynyl group having 4 to 8 carbon atoms and at least one triple bond in the ring. Examples of cycloalkynyl group include cyclobutynyl, cyclopentynyl, cyclohexynyl, cycloheptynyl, cyclooctynyl and the like. C₃ to C₆ cycloalkynyl is preferred.

In the present specification, the term "aryl" employed alone or in combination with other terms includes a monocyclic aromatic hydrocarbon group or a fused aromatic hydrocarbon group condensed two or three of the aromatic rings. Examples of the aryl include phenyl, 1-naphthyl, 2-naphthyl, anthryl and the like.
Preferable is phenyl as "aryl" for R².
Preferable is phenyl as "aryl" for R³.
Preferable is phenyl as "aryl" for R⁷.
Preferable are phenyl or naphthyl as "aryl" for Y.
In the present specification, the term "naphthyl" includes 1-naphthyl and 2-naphthyl.
In the present specification, the term "aralkyl" herein used means the above mentioned "lower alkyl" substituted one or more with the above mentioned "aryl" at any possible position. Examples of the aralkyl are benzyl, phenylethyl (e.g., 2-phenylethyl), phenylpropyl (e.g., 3-phenylpropyl), naphthylmethyl (e.g., I-naphthylmethyl and 2-naphthylmethyl), anthrylmethyl (e.g., 9-anthrylmethyl), biphenylmethyl (e.g., 4-biphenylmethyl), and the like. Benzyl and phenylethyl are preferred.
Preferable are phenyl C₁ to C₆ alkyl and naphthyl C₁ to C₆ alkyl as "aralkyl" for R². More preferable is C₁ to C₆ alkyl and naphthyl C₁ to C₆ alkyl. Examples are benzyl, phenylethyl, 1-naphthylmethyl, and 2-naphthylmethyl.
Preferable is phenyl C₁ to C₃ alkyl as "aralkyl" for R³. Benzyl is more preferable.

In the present specification, the term "heteroaryl" employed alone or in combination with other terms includes a 5 to 6 membered aromatic heterocyclic group which contains one or more hetero atoms selected from the group consisting of oxygen, sulfur, and nitrogen atoms in the ring and may be fused with cycloalkyl, aryl, non-aromatic heterocyclic group, and other heteroaryl at any possible position. Examples of the heteroaryl are pyrrolyl (e.g., 1-pyrrolyl, 2-pyrrolyl, 3-pyrrolyl), furyl (e.g., 2-furyl, 3-furyl), thienyl (e.g., 2-thienyl, 3-thienyl), imidazolyl (e.g., 2-imidazolyl, 4-imidazolyl), pyrazolyl (e.g., 1-pyrazolyl, 3-pyrazolyl), isothiazolyl (e.g., 3-isothiazolyl), isoxazolyl (e.g., 3-isoxazolyl), oxazolyl (e.g., 2-oxazolyl), thiazolyl (e.g., 2-thiazolyl), pyridyl (e.g., 2-pyridyl, 3-pyridyl, 4-pyridyl), pyrazinyl (e.g., 2-pyrazinyl), pyrimidinyl (e.g., 2-pyrimidinyl, 4-pyrimidinyl), pyridazinyl (e.g., 3-pyridazinyl), tetrazolyl (e.g., 1H-tetrazolyl), oxadiazolyl (e.g., 1,3,4-oxadiazolyl), thiadiazolyl (e.g., 1,3,4-thiadiazolyl), indolizinyl (e.g., 2-indolizinyl, 6-indolizinyl), isoindolyl (e.g., 2-isoindolyl), indolyl (e.g., 1-indolyl, 2-indolyl, 3-indolyl), indazolyl (e.g., 3-indazolyl), purinyl (e.g., 8-purinyl), quinolizinyl (e.g., 2-quinolizinyl), isoquinolyl (e.g., 3-isoquinolyl), quinolyl (e.g., 2-quinolyl, 5-quinolyl), phthalazinyl (e.g., 1-phthalazinyl), naphthyridinyl (e.g., 2-naphthyridinyl), quinoxanyl (e.g., 2-quinoxanyl), quinazolinyl (e.g., 2-quinazolinyl), cinnolinyl (e.g., 3-cinnolinyl), pteridinyl (e.g., 2-pteridinyl), carbazolyl (e.g., 2-carbazolyl, 3-carbazolyl), phenanthridinyl (e.g., 2-phenanthridinyl, 3-phenanthridinyl), acridinyl (e.g., 1-acridinyl, 2-acridinyl), dibenzofuranyl (e.g., 1-dibenzofuranyl, 2-dibenzofuranyl), benzimidazolyl (e.g., 2-benzimidazolyl), benzisoxazolyl (e.g., 3-benzisoxazolyl), benzoxazolyl (e.g., 2-benzoxazolyl), benzoxadiazolyl (e.g., 4-benzoxadiazolyl), benzisothiazolyl (e.g., 3-benzisothiazolyl), benzothiazolyl (e.g., 2-benzothiazolyl, 5-benzothiazolyl), benzofuryl (e.g., 3-benzofuryl), benzothienyl (e.g., 2-benzothienyl) and the like.
Preferable are indolyl, imidazolyl, furyl, thienyl, and the like as "heteroaryl" for R².
Preferable are pyridyl, thienyl, furyl, imidazolyl, and the like as "heteroaryl" for R³.
Preferable are pyridyl, thienyl, furyl, indolyl, benzothienyl, dibenzofuryl, benzimidazolyl, benzothiazolyl, carbazolyl, quinolyl, and the like as "heteroaryl" for Y.

In the present specification, the term "heteroarylalkyl" herein used includes the above mentioned "lower alkyl" substituted one or more with the above mentioned "heteroaryl" at any possible position.
Examples of the heteroarylalkyl are oxazolylmethyl (e.g., 2-oxazolylmethyl, 4-oxazolylmethyl), thiazolylmethyl (e.g., 2-thiazolylmethyl, 4-thiazolylmethyl), oxazolylethyl (e.g., 5-oxazolyl-2-ethyl), thiazolylethyl (e.g., 5-thiazolyl-2-ethyl), benzoxazolylmethyl (e.g., benzoxazol-2-ylmethyl), benzothiazolylmethyl (e.g., benzothiazol-2-ylmethyl), indolylmethyl (e.g., indol-3-ylmethyl), imidazolylmethyl (e.g., 4-imidazalylznethyl), indazolylmethyl (e.g., 1-indazolylmethyl), benzotriazolylmethyl (e.g., 1-benzotriazolylmethyl), benzoquinolylmethyl (e.g., 2-benzoquinolylmethyl), benzimidazolylmethyl (e.g., benzimidazol-2-ylmethyl), pyridylmethyl (e.g., 4-pyridylmethyl), furylmethyl (e.g., furan-2-ylmethyl), thienylmethyl (e.g., thiophen-2-ylmethyl) and the like.
Preferable are indolylmethyl (e.g., indol-3-ylmethyl), imidazolylmethyl (e.g., imidazol-5-ylmethyl), benzoxazolylmethyl (e.g., benzoxazol-2-ylmethyl), benzothiazolylmethyl (e.g., benzothiazol-2-ylmethyl), benzimidazolylmethyl (e.g., benzimidazol-2-ylmethyl), thienylmethyl (e.g., thiophen-2-ylmethyl), thiazolylmethyl (e.g., 2-thiazolylmethyl, 4-thiazolylmethyl) and the like as "heteroarylalkyl" for R².
Preferable are indolylmethyl (e.g., indol-3-ylmethyl), imidazolylmethyl (e.g., imidazol-5-yhnethyl), thienylmethyl (e.g., thiophen-2-ylmethyl), thiazolylmethyl (e.g., 2-thiazolylmethyl, 4-thiazolylmethyl) and the like as "heteroarylalkyl" for R³.

In the present specification, the term "non-aromatic carbocyclic group" employed alone or in combination with other terms includes cycloalkyl, cycloalkenyl, cycloalkynyl, or a condensed cyclic group thereof with above mentioned "aryl".
Examples of the non-aromatic carbocyclic group are cyclopentyl, cyclohexyl, indanyl, tetrahydronaphthyl and the like.
Preferable are cycloalkane, cycloalkene, or cycloalkyne as "non-aromatic carbocyclic group" when R² and R¹² taken together with the adjacent carbon atom form a 5 to 6 membered non-aromatic carbocyclic group. Preferable are cyclopentane, cyclohexane, and the like.
In the present specification, the term "non-aromatic heterocyclic group" employed alone or in combination with other terms includes a 5 to 7 membered non-aromatic ring which contains one or more hetero atoms selected from the group consisting of oxygen, sulfur, and nitrogen atoms in the ring or a condensed ring group which is formed with two or more of them, or a condensed ring group which is formed with above mentioned "ring" and above mentioned "aryl". Examples of the non-aromatic heterocyclic group are pyrrolidinyl (e.g., 1-pyrrolidinyl, 2-pyrrolidinyl), pyrrolinyl (e.g., 3-pyrrolinyl), imidazolidinyl (e.g., 2-imidazolidinyl), imidazolinyl (e.g., imidazolinyl), pyrazolidinyl (e.g., 1-pyrazolidinyl, 2-pyrazolidinyl), pyrazolinyl (e.g., pyrazolinyl), piperidinyl (piperidino, 2-piperidinyl), piperazinyl (e.g., 1-piperazinyl), indolynyl (e.g., 1-indolynyl), isoindolinyl (e.g., isoindolinyl), morpholinyl (e.g., morpholino, 3-morpholinyl), tetrahydroquinolyl, tetrahydrofuryl, tetrahydropyranyl, 1,3-benzodioxolanyl, 1,4-benzodioxanyl, 1-benzoxolanyl and the like.
Preferable are tetrahydropyran and the like as "non-aromatic heterocyclic group" when R² and R¹² taken together with the adjacent carbon atom form a 5 to 6 membered non-aromatic heterocyclic group..

In the present specification, the term "halogen" herein used means fluoro, chloro, bromo, and iodo. Fluoro, chloro, and bromo are preferred.
In the present specification, the term "lower alkyloxy" herein used are methyloxy, ethyloxy, n-propyloxy, isopropyloxy, n-butyloxy, isobutyloxy, s-butyloxy, t-butyloxy, n-pentyloxy, n-hexyloxy and the like. Preferable are methyloxy, ethyloxy, n-propyloxy, isopropyloxy, n-butyloxy, s-butyloxy, isobutyloxy, and t-butyloxy. Preferable is C₁ to C₆ alkyloxy. More preferable is C₁ to C₃ alkyloxy.
In the present specification, the term "lower alkenyloxy" herein used are vinyloxy, allyloxy, propenyloxy, 3-butenyloxy, 2-butenyloxy (crotonylaxy, isocrotonyloxy), 1-butenyloxy, isopentenyloxy and the like. Preferable is C₂ to C₃ alkenyloxy.
In the present specification, the term "lower alkylthio" herein used are methylthio, ethylthio, propylthio, isopropylthio, butylthio, s-butylthio, isobutylthio, t-butylthio and the like. Preferable is C₁ to C₃ alkylthio.

In the present specification, the term "halo(lower)alkyl" employed alone or in combination with other terms includes the above-mentioned "lower alkyl" which is substituted with the above mentioned "halogen" at 1 to 8 positions, preferably, at 1 to 5. Examples of the halo(lower)alkyl are trifluoromethyl, trichloromethyl, difluoroethyl, trifluoroethyl, dichloroethyl, trichloroethyl, and the like. Preferable is C₁ to C₃ lower alkyl" which is substituted with the above mentioned "halogen" at 1 to 5 positions. More preferable is trifluoromethyl.
In the present specification, preferable of the term "halo(lower)alkyloxy" are C₁ to C₃ alkyloxy substitute with the above mentioned "halogen" at 1 to 5 positions. More preferable is trifluoromethyloxy and the like.
In the present specification, preferable of the term "halo(lower)alkylthio" are C₁ to C₃ alkylthio substitute with the above mentioned "halogen" at 1 to 5 positions. More preferable is trifluoromethylthio and the like.
In the present specification, the term "hydroxy(lower)alkyl" includes the above-mentioned "lower alkyl" which is substituted with hydroxyl group. Examples of the hydroxy(lower)alkyl are hydroxymethyl, 1-hydroxyethyl, 2-hydroxyethyl, 1-hydroxypropyl, 2-hydroxypropyl, 3-hydroxypropyl, and the like. Preferable are hydroxymethyl, 1-hydroxyethyl, and 2-hydroxyethyl.
In the present specification, examples of the term "lower alkyloxycarbonyl" herein used are methyloxycarbonyl, ethyloxycarbonyl, n-propyloxycarbonyl, isopropyloxycarbonyl, and the like.
In the present specification, examples of the term "lower alkylsulfonyl" herein used are methylsulfonyl, ethylsulfonyl, and the like. Preferable is methylsulfonyl.
In the present specification, examples of the term "aralkyloxy" herein used are phenyl C₁ to C₃ alkyloxy, naphthyl C₁ to C₃ alkyloxy and the like. Preferable is benzyloxy.
In the present specification, the term "lower alkyloxy(lower)alkyl" includes the above-mentioned "lower alkyl" which is substituted with the above mentioned "lower alkyloxy". Examples of the lower alkyloxy(lower)alkyl are methyloxymethyl, ethyloxymethyl, propyloxymethyl, 1-methyloxyethyl, 2-methyloxyethyl, 1-methyloxypropyl, 2-methyloxypropyl, 3-methyloxypropyl, and the like. Preferable is methyloxymethyl.
In the present specification, examples of the term "aryloxy(lower)alkyl" herein used are phenyloxymethyl, 1-phenyloxyethyl, 2-phenyloxyethyl, 1-naphthyloxymethyl, 2-naphthyloxymethyl, and the like.
In the present specification, the term "acyl" employed alone or in combination with other terms includes alkylcarbonyl in which alkyl group is the above mentioned "lower alkyl" or arylcarbonyl in which aryl group is the above mentioned "aryl". Examples of the acyl are acetyl, propyonyl, benzoyl, and the like. "Lower alkyl" and "aryl" may be substituted respectively with substituents mentioned below.
In the present specification, examples of the term "acyloxy" herein used are acetyloxy, propyonyloxy, benzoyloxy, and the like.

In the present specification, the term "optionally substituted amino" employed alone or in combination with other terms includes amino, or amino substituted with one or two of the above mentioned "lower alkyl", "aralkyl", "heteroarylalkyl" or "acyl". Preferable are unsubstituted amino or amino substituted with one or two of groups selected with C₁ to C₆ alkyl, phenyl C₁ to C₃ alkyl, pyridyl C₁ to C₃ alkyl, C₁ to C₆ alkylcarbonyl, and arylcarbonyl. Examples are amino, methylamino, dimethylamino, ethylmethylamino, diethylamino, benzylamino, acetylamino, benzoylamino, and the like. Preferable are amino, methylamino, dimethylamino, ethylmethylamino, diethylamino, and acetylamino.
In the present specification, preferable are unsubstituted aminocarbonyl or aminocarbonyl optionally substituted with one or two of C₁ to C₆ alkyl as "optionally substituted aminocarbonyl". Examples are aminocarbonyl, methylaminocarbonyl, dimethylaminocarbonyl, ethylmethylaminocarbonyl, diethylaminocarbonyl, and the like. Preferable are aminocarbonyl and dimethylaminocarbonyl.

In the present specification, the substituents of "optionally substituted lower alkyl" are lower alkylthio, cycloalkyl, carboxy, lower alkyloxy, hydroxy, mercapto, halogen, nitro, cyano, lower alkyloxycarbonyl, halo(lower)alkyl, halo(lower)alkyloxy, optionally substituted amino, optionally substituted aminocarbonyl, acyl, acyloxy, non-aromatic heterocyclic group optionally substituted with substituents selected from Substituents group A, aryloxy, aralkyloxy, lower alkylsulfonyl, guanidino, azo group, ureide optionally substituted with substituents selected from Substituents group A, carbamoyl, and the like. These substituents are able to locate at one or more of any possible positions.
Substituents group A: lower alkyl, cycloalkyl, lower alkenyl, lower alkynyl, hydroxy, lower alkyloxy, mercapto, lower alkylthio, lower alkenyloxy, halogen, nitro, cyano, carboxy, lower alkyloxycarbonyl, halo(lower)alkyl, halo(lower)alkyloxy, halo(lower)alkylthio, optionally substituted amino, optionally substituted aminocarbonyl, acyl, acyloxy, aryl, heteroaryl, non-aromatic heterocyclic group, aralkyl, lower alkylsulfonyl, guanidino, azo group, and ureide.
Preferred substituents of "optionally substituted lower alkyl" for R² are lower alkyloxy, lower alkylthio, cycloalkyl, carboxy, halogen, hydroxy, carbamoyl, and mercapto.
Preferred substituents of "optionally substituted lower alkyl" for R³ are hydroxy, lower alkyloxy, and non-aromatic heterocyclic group optionally substituted with substituents selected from Substituents group A.

In the present specification, the substituents of "optionally substituted aryl", "optionally substituted heteroaryl", "optionally substituted aralkyl", and "optionally substituted heteroarylalkyl" for R² and R⁸ are lower alkyl optionally substituted with substituents selected from Substituents group A, cycloalkyl, lower alkenyl, lower alkynyl, hydroxy, lower alkyloxy, mercapto, lower alkylthio, lower alkenyloxy, halogen, nitro, cyano, carboxy, lower alkyloxycarbonyl, halo(lower)alkyl, halo(lower)alkyloxy, halo(lower)alkylthio, optionally substituted amino, optionally substituted aminocarbonyl, acyl, acyloxy, aryl optionally substituted with substituents selected from Substituents group A, heteroaryl optionally substituted with substituents selected from Substituents group A, non-aromatic heterocyclic group optionally substituted with substituents selected from Substituents group A, aralkyl optionally substituted with substituents selected from Substituents group A, lower alkylsulfonyl, guanidino, azo group, ureide optionally substituted with substituents selected from Substituents group A, and the like. These substituents are able to locate at one or more of any possible positions.
Substituents group A: lower alkyl, cycloalkyl, lower alkenyl, lower alkynyl, hydroxy, lower alkyloxy, mercapto, lower alkylthio, lower alkenyloxy, halogen, nitro, cyano, carboxy, lower alkyloxycarbonyl, halo(lower)alkyl, halo(lower)alkyloxy, halo(lower)alkylthin, optionally substituted amino, optionally substituted aminocarbonyl, acyl, acyloxy, aryl, heteroaryl, non-aromatic heterocyclic group, aralkyl, lower alkylsulfonyl, guanidino, azo group, and ureide.
Preferred substituents of "optionally substituted aryl" for R² is hydroxy.
Preferred substituents of "optionally substituted aryl" for R³ are hydroxy, lower alkyloxy, halogen, or halo(lower)alkyl.
Preferred substituents of "optionally substituted heteroaryl" for R² are hydroxy or halogen.
Preferred substituents of "optionally substituted heteroaryl" for R³ are hydroxy, lower alkyloxy, halogen, or halo(lower)alkyl.
Preferred substituents of "optionally substituted aralkyl" for R² are hydroxy, halogen, nitro, lower alkyloxy, halo(lower)alkyl, or earboxy(lower)alkyl.
Preferred substituents of "optionally substituted aralkyl" for R³ are hydroxy, lower alkyloxy, halogen, halo(lower)alkyl, or nitro.
Preferred substituents of "optionally substituted heteroarylalkyl" for R² are halogen or hydroxy.
Preferred substituents of "optionally substituted heteroarylalkyl" for R³ are hydroxy, lower alkyloxy, halogen, or halo(lower)alkyl.
Substituents of "optionally substituted aryl", "optionally substituted heteroaryl", "optionally substituted aryloxy", "optionally substituted aralkyloxy", "optionally substituted lower alkyloxy(lower)alkyl", "optionally substituted aryloxy(lower)alkyl", "optionally substituted ureide", "optionally substituted pyrolizinyl", "optionally substituted morpholinyl", and "optionally substituted piperidinyl" for R⁷ herein used are lower alkyl, cycloalkyl, lower alkenyl, lower alkynyl, hydroxy, lower alkyloxy, mercapto, lower alkylthio, lower alkenyloxy, halogen, nitro, cyano, carboxy, lower alkyloxycarbonyl, halo(lower)alkyl, halo(lower)alkyloxy, halo(lnwer)alkylthio, optionally substituted amino, optionally substituted aminocarbonyl, acyl, acyloxy, aryl, heteroaryl, non-aromatic heterocyclic group, aralkyl, lower alkylsulfonyl, guanidino, azo group, and ureide.

Substituents of "optionally substituted aryl", "optionally substituted heteroaryl", "optionally substituted non-aromatic carbocyclic group", and "optionally substituted non-aromatic heterocyclic group" for Y herein used are halogen, lower alkyl, lower alkyloxy, cycloalkyl, lower alkenyl, lower alkynyl, lower alkenyloxy, lower alkylthio, halo(lower)alkyl, halo(lower)alkyloxy, halo(lower)alkylthio, hydroxy, hydroxy(lower)alkyl, mercapto, carboxy, lower alkyloxycarbonyl, lower alkylsulfonyl, acyl, acyloxy, nitro, cyano, optionally substituted amino, optionally substituted aminocarbonyl, optionally substituted aryl, optionally substituted heteroaryl, optionally substituted aryloxy, optionally substituted aralkyloxy, lower alkyloxy(lower)alkyl, optionally substituted aryloxy(lower)alkyl, optionally substituted ureide, optionally substituted pyrolizinyl, optionally substituted morpholinyl, and optionally substituted piperidinyl. These substituents are able to locate at one or more of any possible positions. Substituents of "optionally substituted aryl", "optionally substituted heteroaryl", "optionally substituted aryloxy", "optionally substituted aralkyloxy", "optionally substituted lower alkyloxy(lower)alkyl", "optionally substituted aryloxy(lower)alkyl", "optionally substituted ureide", "optionally substituted pyrolizinyl", "optionally substituted morpholinyl", and "optionally substituted piperidinyl" for the above mentioned substituents are lower alkyl, cycloalkyl, lower alkenyl, lower alkynyl, hydroxy, lower alkyloxy, mercapto, lower alkylthio, lower alkenyloxy, halogen, nitro, cyano, carboxy, lower alkyloxycarbonyl, hala(lower)alkyl, halo(lower)alkyloxy, halo(lower)alkylthio, optionally substituted amino, optionally substituted aminocarbonyl, acyl, acyloxy, aryl, heteroaryl, non-aromatic heterocyclic group, aralkyl, lower alkylsulfonyl, guanidino, azo group, and ureide.

The compounds of the present invention have specific inhibitory activities against the MMP-13 and are useful for the treating or preventing agent for diseases caused by MMP-13. Furthermore, it has relatively low protein binding ratio, high oral absorbability, and low toxicity. Therefore, it is excellent for medicament.

### Best Mode for Carrying Out the Invention

The compounds of the present invention are able to be synthesized in accordance with the methods described in WO97/27174, WO00/46189, etc., and well known methods. Methods A to C are illustrated as the synthetic methods for the compound of the present invention as follow. 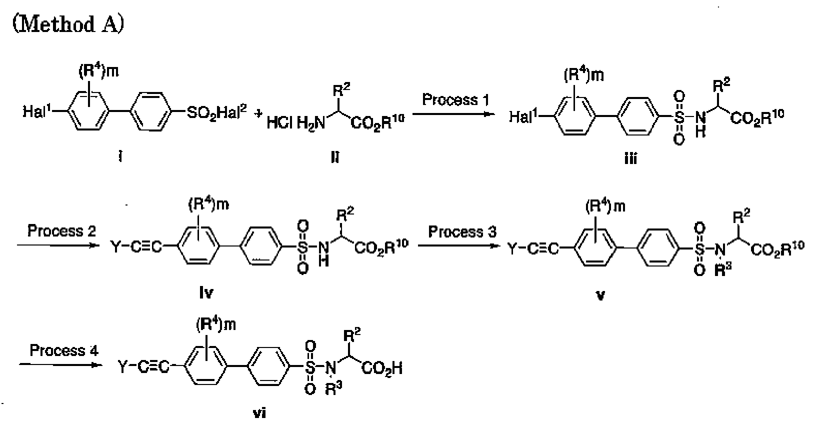 wherein R², R³, R⁴, Y, and m are as defined above; R¹⁰ is a carboxy protecting group; and Hall and Hal² are each independently halogen.

### (Process 1)

Some of amino acids represented by the formula (ii) or its acidic salts (e.g., hydrochloride, p-toluenesulfonate, and trifluoroacetate) which are starting materials are commercially available. The other are able to be synthesized in accordance with a method described in Zikkenkagakukoza, vol. 22, IV (nihonkagakukai), J. Med. Chem. 38, 1689-1700, 1995, Gary M. Ksander et. al., etc.
Some of sulfonating agents (e.g., sulfonyl halide) are commercially available and the other are synthesized in accordance with a method described Shin-zikkenkagakukoza, vol. 14, 1787, 1978, Synthesis 852-854, 1986, etc.
A carboxyl protective group is exemplified by esters (e.g., methyl ester, tert-butyl ester and benzyl ester). Deprotection of this protective group may be carried out by hydrolysis with acid (e.g., hydrochloride and trifluoroacetic acid) or base (e.g., sodium hydroxide) depending on the type of the group, or by catalytic reduction (e.g., under 10% palladium-carbon catalyst condition).
Preferable solvents for this sulfonylation are dimethylformamide, tetrahydrofuran, dioxane, dimethylsulfoxide, acetonitrile, water, mixed solvents thereof, or mixed solvent of water insoluble solvent and above mentioned solvent.
A base to be used in this sulfonylation is exemplified by organic bases such as triethylamine, N-methylmorpholine, etc. and inorganic bases such as sodium hydroxide, potassium hydroxide, potassium carbonate, and the like. IJsually this reaction can be carried out at ice-cooling to room temperature. When R² and R⁴ contain a functional group(s) possibly interfering this sulfonylation (e.g., hydroxy, mercapto, amino, and guanidino), it can previously be protected in accordance with a method described in "Protective Groups in Organic Synthesis" (Theodora W. Green (John Wiley & Sons)) and then deprotected at an appropriate process.

### (Process 2)

The compound (iii) is reacted with an optionally substituted phenyl, an optionally substituted naphthyl derivative, and the like having an ethynyl group such as ethynylbenzene, ethynylnaphtalene, etc. in a solvent such as dimethylformamide, toluene, xylene, benzene, tetrahydrofuran etc. in the presence of a palladium catalyst (e.g., Pd(Ph₃P)₂Cl₂ etc.), a divalent copper reagent (e.g., CuI etc.), and an organic base (e.g., triethylamine, diisopropylethylamine, etc.) to give a desired compound (Sonogashira reaction).
This reaction is carried out at room temperature to 100 °C, preferably room temperature to 80 °C . This reaction is completed for 3 to 30 hours, preferably 10 to 20 hours. When optionally substituted aryl or optionally substituted heteroaryl has a substituent(s) interfering this reaction, the substituent(s) can previously be protected in accordance with a method of " Protective Groups in Organic Synthesis " (Theodora W. Green (John Wiley & Sons)), and then deprotected at an appropriate step.

### (Process 3)

The compound (iv) is reacted with alkyl halide (e.g., methyl iodide, and ethyl iodide, etc.), aralkyl halide (e.g., benzyl chloride, and benzyl bromide, etc.), and the like in dimethylformamide, tetrahydrofuran, dioxane, and the like at a temperature range of ice-cooling to 80 °C, preferably ice-cooling to room temperature, for 3-30 hours, preferably 10-20 hours to give the desired N-R³ derivative.

### (Process 4)

The compound (v) is deprotected in accordance with usual methods to give a compound represented by the formula (III).

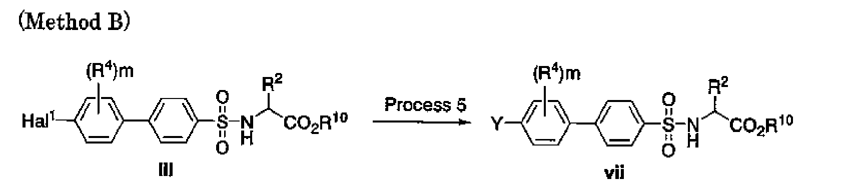

wherein R², R³, R⁴, Y, m, R¹⁰, and Hall are as defined above.
The compounds represented by the formula (II) are able to be synthesized in accordance with above described Process 5, instead of Process 2 of Method A, and following Processes 3 and 4 of Method A.

### (Process 5)

The compound (iii) is reacted with an optionally substituted phenyl, an optionally substituted naphthyl derivative, etc. having a B(OH)₂ group (otherwise B(Et)₂) such as phenylboronic acid, naphthylboronic acid, etc. in a solvent such as dimethylformamide, toluene, xylene, benzene, tetrahydrofuran etc. in the presence of a palladium catalyst (e.g., Pd(PhsP)₄ etc.) and a base (e.g., potassium carbonate, calcium carbonate, triethylamine, sodium methoxide etc.) to give the desired compound (vii) (Suzuki reaction).
This reaction is carried out at room temperature to 100 °C, preferably room temperature to 80 °C. This reaction is completed for 5 to 50 hours, preferably 15 to 30 hours. When optionally substituted phenyl or optionally substituted naphthyl has a substituent(s) interfering this reaction, the substituent(s) can previously be protected in accordance with a method of " Protective Groups in Organic Synthesis " (Theodora W. Green (John Wiley & Sons)) and then deprotected at an appropriate step.

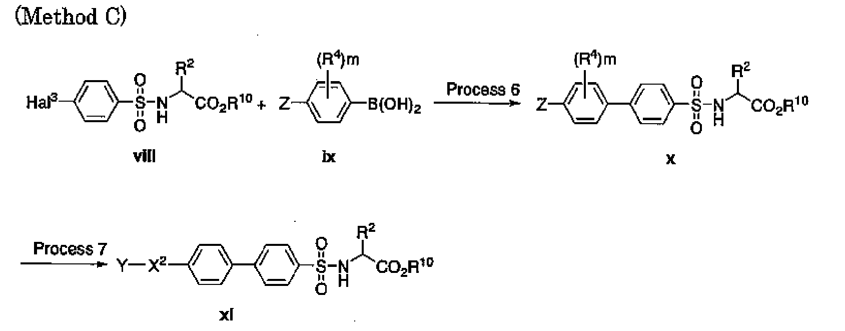

wherein R², R³, R⁴, Y, m, and R¹⁰ are as defined above; Hal³ is halogen; Z is amino or carboxy; and X² is -CONH- or -NHCO-.

### (Process 6)

This process may be carried out to use Suzuki reaction in accordance with the method of above mentioned Process 5.

### (Process 7)

The compound (x) is reacted with an optionally substituted phenyl, an optionally substituted naphthyl derivative, and the like having an acid halide group (otherwise an activate ester) such as benzoyl chloride etc. in a solvent such as dimethylformamide, tetrahydrofuran, dioxane, dimethylsulfoxide, acetonitrile, xylene, toluene, benzene, dichloromethane, etc. in the presence of a base (e.g., triethylamine, N-methylmorpholine, potassium carbonate etc.) to give the desired compound (xi).

This reaction is carried out at a temperature under ice-cooling to 100 °C, preferably room temperature to 60 °C, and is completed for 3 to 30 hours, preferably 10 to 25 hours.
The compounds represented by the formula (V) and (VI) are able to be synthesized to carry out following Processes 3 and 4 of Method A.

Therapeutic effects of the compounds of the present invention for osteoarthritis and adjuvant arthritis may be confirmed by the methods as follows.
A) Osteoarthritis
Guinea pig osteoarthritis model is prepared according to the procedure reported by Meacock et. al. (J. Exp. Path. 71: 279-293, 1990), by medial meniscectomy and collateral ligament transection on the right knee of 12 weeks old female Hartley guinea pigs (Charles River Japan). 0.5% methylcellulose or compounds (30 mg/kg) are orally administered once daily from the following day of the surgery for 10 days.
On the next day of final administration, femoral chondyles and tibial plateaus of right knees are removed. The surfaces of the tibial plateau are stained with India ink (Kuretake), and then photographed using a digital camera (Nikon). Whole area and stained area of the medial plateaus are measured by using a computer aided image analyzing system (WinRoof, MITANI CORPORATION).
(Lesion area (%) = Stained area / Whole area X 100)
In a similar way, oral administration to 12 weeks old female NZW rabbits (Kitayama Labes) or 12 weeks old female SD rats (Clea Japan) for 6 weeks also shows that the present invention compounds are effective on the treatment of osteoarthritis.

B) Adjuvant arthritis
Adjuvant arthritis is induced by subcutaneous injection of Mycobacterium butyricum into the right foot paw of 7 weeks old female Lewis rats (Japan Charles River) according to the procedures reported by Fretcher et al. (J. Pharmacol. Exp. Ther. 284(2): 714-721). Vehicle (0.5% methylcellulose solution) or a test compound (30 mg/kg) is orally administered once a day from the following day of the surgery for 21 days. Hind paw volume is measured using a hydroplethysmometer (Shionogi, Japan) on Day 21 after induction of arthritis. And then spleen and thymus are taken and weighed. Both of hind legs are photographed by X-lay (OHMIC), and destruction level of articulation of X-lay image is scored from 0 (normal) to 3 (perfect destruction of bone and cartilage) in blind manner.

The term "solvate" in the present invention herein used includes a solvate with an organic solvent(s), a hydrate and the like. These hydrates can coordinate with any water molecules.
The term "compound of the present invention" herein used includes a pharmaceutically acceptable salt or its solvate. The salt is exemplified by a salt with alkali metals (e.g., lithium, sodium, potassium, and the like), alkaline earth metals (e.g., magnesium, calcium, and the like), ammonium, organic bases, amino acids, mineral acids (e.g., hydrochloric acid, hydrobromic acid, phosphoric acid, sulfuric acid, and the like), or organic acids (e.g., acetic acid, citric acid, maleic acid, fumaric acid, benzene sulfonic acid, p-toluenesulfonic acid, and the like). These salts and solvates can be formed by the usual method.
The compound of the present invention is not restricted to any particular isomers but includes all possible isomers (e.g. optically active substance) and racemic modifications.
The compound of the present invention has selective and excellent inhibitory activities against MMP-13, as described in the following test example.
Definitely, the compounds of the present invention are useful in the treatment of diseases such as osteoarthritis, rheumatoid arthritis, adjuvant arthritis, periodontitis, hepatocirrhosis, osteoporosis, deossification, gingivitis, colorectal cancer, squamous cell carcinoma, epithelial cancer, prostatic adenoma, ovarioncus, glossoncus, endometrioma, and gastric cancer.
When the compound of the present invention is administered to a person for the treatment of the above diseases, it can be administered orally as powder, granules, tablets, capsules, pilulae, and liquid medicines, or parenterally as injections, suppositories, percutaneous formulations, insufflation, and the like. An effective dose of the compound is formulated by being mixed with appropriate medicinal admixtures such as excipient, binder, penetrant, disintegrators, lubricant, and the like if necessary. Parenteral injections are prepared by sterilizing the compound together with an appropriate carrier.
The dosage varies with the conditions of the patients, administration route, their age, and body weight. In the case of oral administration, the dosage can generally be between 0.1 to 100 mg/kg/day, and preferably 0.1 to 20 mg/kg/day for adult.
The following examples and test examples are provided to further illustrate the present invention and are not to be constructed as limiting the scope thereof.

### Example

Abbreviations described below are used in the following examples.
Me: methyl
Et: ethyl
Pr: propyl
Bu: butyl
Bn: benzyl

### Example 1

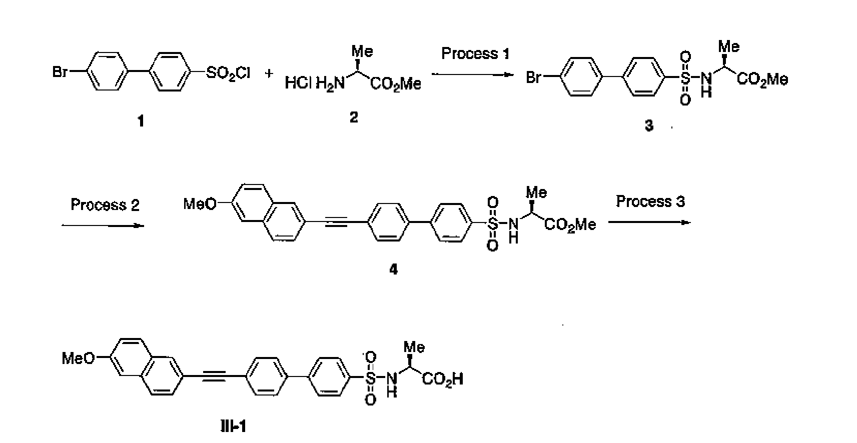

### Process 1

To a solution of 4'-bromabiphenyl-4-sulfanyl chloride (1) (3.32 g, 11.0 mmol) and L-alanine methyl ester hydrochloride (2) (1.54 g, 29.6 mmol) in tetrahydrofuran (30 mL) were added N-methylmorpholine (2.75 mL, 25.0 mmol) under ice-cooling. The mixture was stirred overnight at room temperature. The reaction mixture was poured into ice-2 mol/L hydrochloric acid and extracted with ethyl acetate. The organic layer was washed with saturated aqueous sodium hydrogen carbonate solution and saturated aqueous sodium chloride solution successively, and then dried over anhydrous sodium sulfate. The organic layer was concentrated under reduced pressure. The residue was subjected to silica gel chromatography, and the fraction eluted with hexane/ethyl acetate =1/1 was collected. Recrystallization from ethyl acetate/hexane gave target compound (3) (3.57 g, yield 89.6 %) with a melting point of 118-120 °C.
IR (KBr, vmax cm⁻¹) 3267, 1738, 1433, 1345, 1168, 1133, 815, 602, 573
¹H NMR (CDCl₃, δ ppm): 1.42 (d, J = 7.2 Hz, 3H), 3.55 (s, 3H), 4.04 (m, 1H), 5.26 (d, J = 8.1 Hz, 1H), 7.46 (d, J = 8.1 Hz, 2H), 7.61 (d, J = 8.1 Hz, 2H), 7.67 (d, J = 8.1 Hz, 2H), 7.91 (d, J = 8.1 Hz, 2H)
[α]_{D}- 22.3 ± 1.2° (c = 0.507, DMSO, 24°C)
Elemental analysis for C₁₆H₁₆BrNO₄S
Calcd.:C;48.25, H;4.05, Br;20.06, N;3.52, S;8.05
Found:C;48.37, H;4.06, Br;20.08, N;3.35, S;7.92

Process 2
To a solution of compound (3) (597 mg, 1.50 mmol) in dimethylformamide (6 mL) was added 2-ethynyl-6-methoxynaphtalene (328 mg, 1.80 mmol), bis(triphenylphosphine)palladium(II) chloride (105 mg, 0.15 mmol), copper iodide(I) (57 mg, 0.30 mmol), and triethylamine (0.63 ml, 4.50 mmol). The mixture was degassed under an argon atmosphere sufficiently. The reaction mixture was stirred overnight at 50 °C. The reaction mixture was poured into ice-2 mol/L hydrochloric acid and extracted with ethyl acetate. The organic layer was washed with saturated aqueous sodium hydrogen carbonate solution and saturated aqueous sodium chloride solution successively, and then dried over anhydrous sodium sulfate. The organic layer was concentrated under reduced pressure. The residue was washed with methanol and the crude crystal was filtered. Recrystalization from acetone gave target compound (4) (426 mg, yield 56.8 %) with a melting point of 214-216 °C.
IR (KBr, v max cm⁻¹) 3255, 1705, 1337,1318,1169, 991, 858, 820
¹H NMR (DMSO-d₆,δ ppm): 1.20 (d, J = 7.2 Hz, 3H), 3.44 (s, 3H), 3.90 (s, 3H), 3.91 (m, 1H), 7.24 (dd, J = 2.4, 8.7 Hz, 1H), 7.38 (d, J = 2.4 Hz, 1H), 7.59 (dd, J = 1.8, 8.7 Hz, 1H), 7.71 (d, J = 8.4 Hz, 2H), 7.82-7.92 (m, 6H), 7.95 (d, J = 8.4 Hz, 2H), 8.14 (s, 1H), 8.42 (br s, 1H)
[α]_{D} - 18.4 ± 1.1° (c = 0.511, DMSO, 24 °C)
Elemental analysis for C₂₉H₂₅NO₅S
Calcd.:C;69.72, H;5.04, N;2.80, S;6.42
Found:C;70.01, H;5.03, N;2.65, S;6.30

Process 3
To a solution of compound (4) (380 mg, 0.761 mmol) in dimethylsulfoxide (6.8 mL) was added 1 mol/L aqueous sodium hydroxide solution (2.28 mL) at room temperature. The mixture was stirred overnight at room temperature. The crystallized sodium salt was filtered and washed with ethyl acetate. The crystal was poured into ice·2mol/L hydrochloric acid and extracted with ethyl acetate and tetrahydrofuran. The organic layer was washed with saturated aqueous sodium chloride solution and dried over anhydrous sodium sulfate.
The organic layer was concentrated under reduced pressure. Recrystalization of the residue from acetone/hexane gave a target compound (III-1, 377 mg, yield 94.7 %) with a decomposition point over 285 °C.
IR (KBr,vmax cm⁻¹) 3251, 1712, 1333, 1256, 1167, 1150, 1099, 858, 820
¹H NMR (DMSO-d₆,δppm): 1.19 (d, J = 7.2 Hz, 3H), 3.82 (m, 1H), 3.90 (s, 3H), 7.24 (dd, J = 2.4, 8.7 Hz, 1H), 7.38 (d, J = 2.4 Hz, 1H), 7.59 (dd, J = 1.5, 8.7 Hz, 1H), 7.71 (d, J = 8.4 Hz, 2H), 7.81-7.91 (m, 6H), 7.94 (d, J = 8.4 Hz, 2H), 8.14 (s, 1H), 8.22 (br s, 1H), 12.61 (br s, 1H)
[α]_{D} - 4.0 ± 0.9° (c = 0.503, DMSO, 24 °C)
Elemental analysis for C₂₈H₂₃NO₅S·0.5H₂O
Calcd.:C;68.00, H;4.89, N;2.83, S;6.48
Found:C;68.01, H;5.08, N;2.55, S;6.30

### Example 2

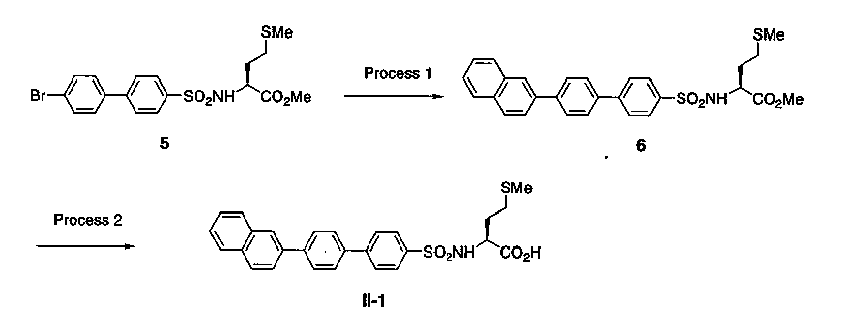

Process 1
To a solution of compound (5) (448 mg, 0.98 mmol) in toluene (10 mL) was added 2-naphthylboronic acid (206 mg, 1.17 mmol) and 2M aqueous sodium carbonate solution (1.5 mL). The mixture was degassed under an argon atmosphere sufficiently. And then to the mixture was added tetrakis (triphenylphosphine) palladium (116 mg, 0.098 mmol). The mixture was degassed under an argon atmosphere sufficiently again and stirred at 90°C for 4 hours. The reaction mixture was poured into ice water and extracted with ethyl acetate. The organic layer was washed with saturated aqueous sodium chloride solution successively and dried over anhydrous sodium sulfate. The organic layer was concentrated under reduced pressure. Recrystalization of the residue from acetone/hexane gave target compound (6) (293 mg, yield 59 %) with a melting point of 148-150°C.
IR (KBr, v max cm⁻¹) 3275, 1742, 1594, 1342, 1217, 1093, 1093, 814
¹H NMR (DMSO-d₆, δ ppm): 1.76-1.93 (m, 2H), 1.94 (s, 3H), 2.34-2.50 (m, 2H), 3.44 (s, 3H), 4.02 (m, 1H), 7.54-7.58 (m, 2H), 7.84-8.07 (m, 12H), 8.33 (s, 1H), 8.47 (d, J = 9.0 Hz, 1H)
[α]_{D} - 6.5 ± 0.9° (c = 0.512, DMSO, 24°C)
Elemental analysis for C₂₂H₂₂N₂O₄S
Calcd.:C;64.37, H;5.40, N;6.82, S;7.81
Found:C;64.33, H;5.43, N;6.62, S;7.46

Process 2
To a solution of compound (6) (278 mg, 0.550 mmol) in dimethylsulfoxide(4.2 mL) was added I mol/L aqueous sodium hydroxide solution(1.4 mL) at room temperature. The reaction mixture was stirred at room temperature overnight. The crystallized sodium salt was filtered and washed with ethyl acetate. The crystal was poured into ice-2mol/L hydrochloric acid and extracted with ethyl acetate and tetrahydrofuran. The organic layer was washed with saturated aqueous sodium chloride solution and dried over anhydrous sodium sulfate. The organic layer was concentrated under reduced pressure. Recrystalization of the residue from acetone/hexane gave the target compound (II-1: 210mg, yield 78 %) with a melting point of 243-245°C.
IR (KBr,vmax cm⁻¹) 3420, 1751, 1652, 1594, 1527, 1490, 1325, 11.58, 1089, 819
¹H NMR (DMSO-d₆, δ ppm): 1.74-1.94 (m, 2H), 1.93 (s, 3H), 2.31-2.50 (m, 2H), 3.93 (m, 1H), 7.53-7.58 (m, 2H), 7.86-8.06 (m, 12H), 8.28 (d, J = 9.0 Hz, 1H), 8.33(s, 1H), 12.81 (br s, 1H)
[α]_{D} + 2.6 ± 0.9° (c= 0.501, DMSO, 24 °C)
Elemental analysis for C₂₈H₂₄N₂O₅S
Calcd.:C;67.18, H;4.83, N;5.60, S;6.41 Found:C;66.88, H;4.81, N;5.67, S;6.32
**Example 3**

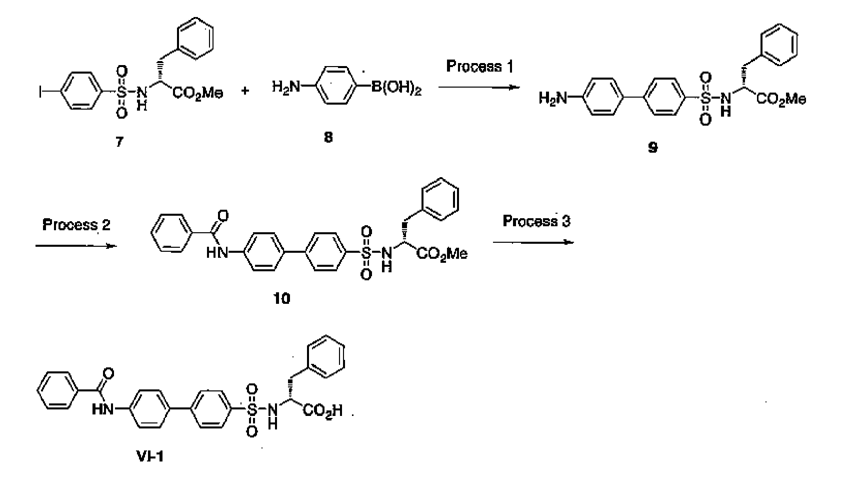

Process 1
To a solution of compound (7) (668 mg, 1.50 mmol) in tetrahydrofuran (3 mL) and toluene (3 mL) was added 4-aminophenylboronic acid (8) (246 mg, 1.80 mmol) and 2M aqueous sodium carbonate solution (3 mL). The mixture was degassed under an argon atmosphere sufficiently. And then to the mixture was added tetrakis (triphenylphosphine) palladium (87 mg, 0.075 mmol). The mixture was degassed under an argon atmosphere sufficiently again and stirred at 90 °C for 6 hours. The reaction mixture was poured into ice water and extracted with ethyl acetate. The organic layer was washed with saturated aqueous sodium chloride solution successively and dried over anhydrous sodium sulfate. The organic layer was concentrated under reduced pressure. The residue was subjected to silica gel chromatography, and the fraction eluted with chloroform/ethyl acetate =2/1 was collected.
Recrystalization from ethyl acetate/hexane gave target compound (9) (423 mg, yield 68.7 %) with a melting point of 148-150 °C.
IR (KBr, v max cm⁻¹) 3462, 3374, 3285, 1742, 1589, 1330, 1310, 1158, 1092, 819
¹H NMR (DMSO-d₆,δppm): 2.77 (dd, J = 8.7, 13.5 Hz, 1H), 2.91 (dd, J = 6.6, 13.5 Hz, 1H=, 3.33 (s, 3H), 3.95 (m, 1H), 5.41 (s, 2H), 6.66 (d, J = 8.4 Hz, 2H), 7.08-7.25 (m, 5H), 7.55 (d, J = 8.4 Hz, 2H), 7.62 (d, J = 8.4 Hz, 2H), 7.43 (d, J = 8.4 Hz, 2H), 8.43 (br s, 1H)
[α]_{D} - 6.5 ± 0.9° (c = 0.508, DMSO, 24 °C)
Elemental analysis for C₂₂H₂₂N₂O₄S
Caled.:C;64.37, H;5.40, N;6.82, S;7.81
Found:C;64.09, H;5.40, N;6.71, S;7.66

Process 2
To a solution of compound (9) (300 mg, 0.731 mmol) in tetrahydrofuran (10 mL) was added N-methylmorpholine (0.12 mL, 1.10 mmol) and benzoyl chloride (93 µL, 0.804 mmol) successively under ice-cooling. The mixture was stirred at room temperature for 2 hours. The reaction mixture was poured into ice-2mol/L hydrochloric acid, and extracted with ethyl acetate and tetrahydrofuran. The organic layer was washed with saturated aqueous sodium hydrogen carbonate solution and saturated aqueous sodium chloride solution successively, and dried over anhydrous sodium sulfate. The organic layer was concentrated under reduced pressure. Recrystalization of the residue from tetrahydrofuran/hexane gave target compound (10) (353 mg, yield 93.8 %) with a melting point of 253-255 °C.
IR (KBr, v- max cm⁻¹) 3277, 1742, 1651, 1592, 1525, 1517, 1331, 1156, 1094, 818
¹H NMR (DMSO-d₆, δ ppm): 2.79 (dd, J = 9.0, 13.5 Hz, 1H), 2:94 (dd, J = 6.3, 13.5 Hz, 1H), 3.36 (s, 3H), 3.99 (m, 1H), 7.10-7.25 (m, 5H), 7.52-7.66 (m, 5H), 7.72-7.80 (m, 4H), 7.91-8.02 (m, 4H), 8.54 (d, J = 9.0 Hz, 1H), 10.42 (s, 1H)
[α]_{D} - 6.2 ± 0.9° (c = 0.500, DMSO, 24 °C)
Elemental analysis for C₂₉H₂₆N₂O₅S
Calcd.:C;67.69, H;5.09, N;5.44, S;6.23
Found:C;67.45, H;5.14, N;5.49, S;6.25

Process 3
To a solution of compound (10) (310 mg, 0.602 mmol) in dimethylsulfoxide(5.4 mL) was added Imol/L aqueous sodium hydroxide solution (1.81 mL) at room temperature. The mixture was stirred at room temperature overnight. The crystallized sodium salt was filtered and washed with ethyl acetate. The crystal was poured into ice·2mol/L hydrochloric acid and extracted with ethyl acetate and tetrahydrofuran. The organic layer was washed with saturated aqueous sodium chloride solution, and dried over anhydrous sodium sulfate. The organic layer was concentrated under reduced pressure. Recrystalization of the residue from acetone/hexane gave target compound (VI-1, 242 mg, yield 80.3 %) with a melting point of 243-245 °C.
IR (KBr, v max cm⁻¹) 3420, 1751, 1652, 1594, 1527, 1490, 1325, 1158, 1089, 819
¹H NMR (DMSO-d₆, δ ppm): 2.74 (dd, J = 9.0, 13.5 Hz, 1H), 2.96 (dd, J = 5.1, 13.5 Hz, 1H), 3.91 (m,1H), 7.10-7.24 (m, 5H), 7.51-7.66 (m, 5H), 7.69-7.79 (m, 4H), 7.91-8.03 (m, 4H), 8.31 (d, J = 9.0 Hz,1H), 10.41 (s, 1H), 12.74 (br s, 1H)
[α]_{D} + 2.6 ± 0.9° (c = 0.501, DMSO, 24 °C)
Elemental analysis for C₂₈H₂₄N₂O₅S
Calcd.:C;67.18, H;4.83, N;5.60, S;6.41 Found:C;66.88, H;4.81, N;5.67, S;6.32

### Example 4

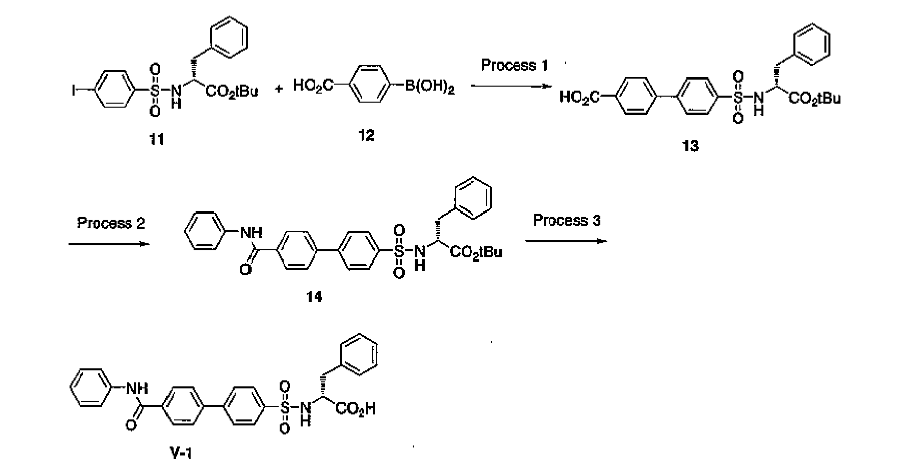

Process 1
To a solution of compound (11) (731 mg, 1.50 mmol) in dimethoxyethane(6 mL) and ethanol (1.5 mL) was added 4-carboxyphenylboronic acid (12) (299 mg, 1.80 mmol) and 2M aqueous sodium carbonate solution (3 mL). The mixture was degassed under an argon atmosphere sufficiently. And then to the mixture was added tetrakis (triphenylphosphine) palladium (87 mg, 0.075 mmol). The mixture was degassed under an argon atmosphere sufficiently again and stirred at 90 °C overnight. The reaction mixture was poured into ice-2mol/L hydrochloric acid, and extracted with ethyl acetate. The organic layer was washed with saturated aqueous sodium chloride solution, and dried over anhydrous sodium sulfate. The organic layer was concentrated under reduced pressure. The residue was subjected to silica gel chromatography, and the fraction eluted with chloroform/methanol =10/1 was collected.
Recrystalization from acetone/hexane gave target compound (13) (422 mg, yield 58.4 %).
¹H NMR (DMSO-d₆, δ ppm): 1.10 (s, 9H), 2.80 (dd, J = 7.8, 13.5 Hz, 1H), 2.89 (dd, J = 6.9, 13.5 Hz, 1H), 3.90 (m, 1H), 7.12-7.28 (m, 5H), 7.72-7.88 (m, 6H), 8.06 (d, J = 8.1 Hz, 2H), 8.51 (d, J = 9.0 Hz, 1H), 14.60 (br s, 1H)

Process 2
To a solution of compound (13) (115 mg, 0.239 mmol) in tetrahydrofuran (2 mL) was added oxalyl chloride (60 µL, 0.693 mmol) and dimethylformamide (0.1 mL) under ice-cooling. The mixture was stirred at room temperature for 1 hour. And then to a solution of aniline (33 µL, 0.359 mmol) and N-methylmorpholine (0.26 mL, 2.39 mmol) in tetrahydrofuran (2 mL) was added the acyl chloride solution prepared as above under ice-cooling. The mixture was stirred at room temperature for 2 hours. The reaction mixture was poured into ice-2mol/L hydrochloric acid, and extracted with ethyl acetate. The organic layer was washed with saturated aqueous sodium hydrogen carbonate solution and saturated aqueous sodium chloride solution successively, and dried over anhydrous sodium sulfate. The organic layer was concentrated under reduced pressure. The residue was subjected to silica gel chromatography, and the fraction eluted with chloroform/ethyl acetate =10/1 was collected. Recrystalization from acetone/hexane gave target compound (14) (120 mg, yield 90.2 %).
¹H NMR (DMSO-d₆, δ ppm): 1.11 (s, 9H), 2.79 (dd, J = 8.4, 13.5 Hz, 1H), 2.89 (dd, J = 6.6, 13.5 Hz, 1H), 3.91 (m, 1H), 7.08-7.28 (m, 6H), 7.33-7.43 (m, 2H), 7.73-7.92 (m, 8H), 8.09 (d, J = 8.4 Hz, 2H), 8.50 (br s, 1H), 10.32 (s, 1H)

Process 3
To a suspension of compound (14) (110 mg, 0.198 mmol) in methylene chloride (2 mL) was added trifluoroacetic acid (2 mL) at room temperature. The mixture was stirred at room temperature for 2.5 hours. The reaction mixture concentrated under reduced pressure. Recrystalization of the residue from acetone/hexane gave the target compound (V-1, 95 mg, yield 95.9 %) with a melting point of 232-234 °C.
IR (KBr, v max cm⁻¹) 3336, 1731, 1647, 1598, 1537, 1442, 1327, 1158, 1094, 699
¹H NMR (DMSO-d₆, δ ppm): 2.75 (dd, J = 9.3, 13.8 Hz, 1H), 2.97 (dd, J = 5.7, 13.8 Hz, 1H), 3.92 (m, 1H), 7.09-7.22 (m, 6H), 7.33-7.42 (m, 2H), 7.66 (d, J = 8.4 Hz, 2H), 7.77-7.84 (m, 4H), 7.88 (d, J = 8.4 Hz, 2H), 8.10 (d, J = 8.4 Hz, 2H), 8.36 (d, J = 8.7 Hz, 1H), 10.33 (s, 1H), 12.80 (br s, 1H)
[α]_{D} +3.2 ± 0.9° (c = 0.504, DMSO, 24 °C)
Elemental analysis for C₂₈H₂₄N₂O₅S·H₂O
Caled.:C;66.00, H;4.95, N;5.50, S;6.29 Found:C;66.07, H;4.86, N;5.44, S;6.09

### Example 5

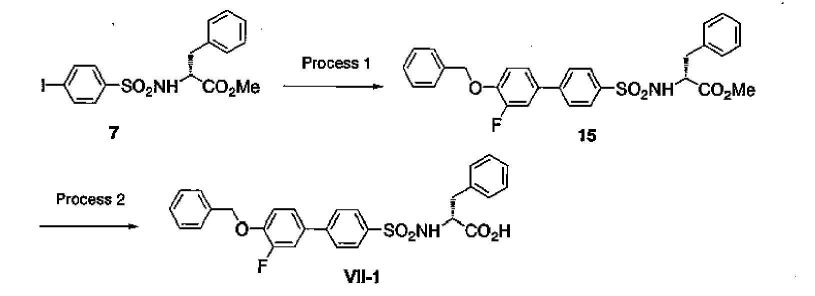

### Process 1

To a solution of compound (7) (500 mg, 1.12 mmol) in toluene (5 mL)was added 4-benzyloxy-3-fluorobronic acid (332 mg, 1.34 mmol) and 2M aqueous sodium carbonate solution (1.1 mL). The mixture was degassed under an argon atmosphere sufficiently. And then to the mixture was added palladium acetate (12.6 mg, 0.056 mmol) and triphenylphosphine (29.2 mg, 0.168 mmol). The mixture was degassed under an argon atmosphere sufficiently again and stirred under reflux for 5 hours. The reaction mixture was poured into ice water, and extracted with ethyl acetate. The organic layer was washed with saturated aqueous sodium chloride solution successively, and dried over anhydrous sodium sulfate. The organic layer was concentrated under reduced pressure. The residue was subjected to silica gel chromatography, and the fraction eluted with toluene/acetone =8/1 was collected. Recrystalization from acetone/hexane gave target compound (15) (453 mg, yield 78 %).
IR (KBr, v max cm⁻¹) 3275, 1742, 1594, 1342, 1217, 1093, 1093, 814
¹H NMR (DMSO-d₆, δ ppm): 2.80 (dd; J = 7.8, 13.5 Hz, 1H), 2.89 (dd, J = 6.9, 13.5 Hz, 1H), 3.90 (m, 1H), 5.25 (s, 2H), 7.12-7.28 (m, 5H), 7.72-7.88 (m, 11H), 7.66 (d, J = 8.1 Hz, 1H), 8.81 (br s, 1H)
[α]_{D} - 6.9 ± 0.9° (c = 0.512, DMSO, 24 °C)
Elemental analysis for C₂₉H₂₆FNO₅S
Calcd.:C;67.04, H;5.04, F;3.66, N;2.70, S;6.17
Found:C;67.24, H;5.14, F;3.69, N;2.71, S;6.28

Process 2
To a solution of compound (15) (431 mg, 0.830 mmol) in dimethylsulfoxide(6.3 mL) was added I mol/L aqueous sodium hydroxide solution (2.1 mL) at room temperature. The mixture was stirred at room temperature overnight. The crystallized sodium salt was filtered and washed with ethyl acetate. The crystal was poured into ice-2mol/L hydrochloric acid and extracted with ethyl acetate and tetrahydrofuran. The organic layer was washed with saturated aqueous sodium chloride solution, and dried over anhydrous sodium sulfate. The organic layer was concentrated under reduced pressure. Recrystalization of the residue from ethyl acetate/hexane gave target compound (VII-1: 351mg, yield 84%) with a melting point of 155-157 °C.
IR(KBr,vmax cm⁻¹) 3420, 1751, 1652, 1594, 1527, 1490, 1325, 1158, 1089, 819
¹H NMR (DMSO-d₆, δ ppm): 2.80 (dd, J = 7.8, 13.5 Hz, 1H), 2.99 (dd, J = 6.9, 13.5 Hz, 1H), 3.95 (m, 1H), 5.25 (s, 2H), 7.12-7.50 (m, 16H), 7.61 (d, J = 12.6 Hz, 1H), 8.61 (br s, 1H), 12.86 (br s, 1H)
[α]_{D} + 2.6 ± 0.9° (c= 0.501, DMSO, 24 °C)
Elemental analysis for C₂₈H₂₄FNO₅S
Calcd.:C;66.62, H;4.78, F;3.76, N;2.77, S;6.34
Found:C;66.62, H;4.78, F;3.76, N;2.77, S;6.34

According to above mentioned Examples 1 to 5, the following compounds were synthesized.

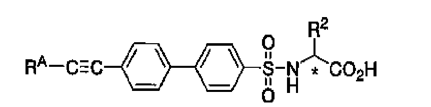

**Table 1**

| Compound No. | R² | R^{A} | * | 1H-NMR (DMSD-d₆) |
|---|---|---|---|---|
| III-2 | Me | 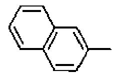 | R | |
| III-3 | Me | 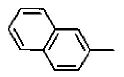 | S | 1.19 (d, J=7.2 Hz, 3H), 3.82 (m, 1H), 7.56-7.63 (m, 2H), 7.65 (dd, J=1.2, 8.4 Hz, 1H), 7.74 (d, J=8.4 Hz, 2H), 7.82-8.02 (m, 9H), 8.13-8.32 (m, 2H), 12.66 (br s, 1H) |
| III-4 | i-Pr | 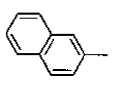 | R | 0.82 (d, J=6.6 Hz, 3H), 0.85 (d, J=6.9 Hz, 3H), 1.96 (m, 1H), 3.56 (m, 1H), 7.55-7.68 (m, 3H), 7.74 (d, J=8.4 Hz, 2H), 7.82-8.02 (m, 9H), 8.11 (d, J=10.2 Hz, 1H), 8.23 (s, 1H), 12.65 (br s, 1H) |
| III-5 | i-Pr | 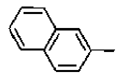 | S | |
| III-6 | i-Bu | 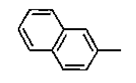 | R | 0.71 (d, J=6.0 Hz, 3H), 0.82 (d, J=6.6 Hz, 3H), 1.42-1.46 (m, 2H), 1.60 (m, 1H), 3.71 (m, 1H), 7.55-7.68 (m, 3H), 7.74 (d, J=7.5 Hz, 2H), 7.80-7.90 (m, 4H), 7.90-8.03 (m, 5H), 8.17-8.29 (m, 2H), 12.62 (br s, 1H) |
| III-7 | i-Bu | 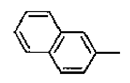 | S | 0.71 (d, J=6.0 Hz, 3H), 0.83 (d, J=6.6 Hz, 3H), 1.42-1.47 (m, 2H), 1.60 (m, 1H), 3.70 (m, 1H), 7.56-7.68 (m, 3H), 7.74 (d, J=7.5 Hz, 2H), 7.80-7.90 (m, 4H), 7.90-8.04 (m, 5H), 8.17-8.29 (m, 2H), 12.62 (br s, 1H) |
| III-8 | s-Bu | 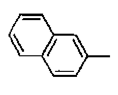 | S | 0.77 (t, J=7.5 Hz, 3H), 0.82 (d, J=6.9 Hz, 3H), 1.13 (m, 1H), 1.38 (m, 1H), 1.70 (m, 1H),3.61 (dd, J=6.6, 9.0 Hz, 1H), 7.55-7.67 (m, 3H), 7.74 (d, J=8.1 Hz, 2H), 7.83-7.89 (m, 4H), 7.91-8.01 (m, 5H), 8.13 (d, J=9.0 Hz, 1H), 8.23 (s, 1H), 12.61 (br s, 1H) |
| III-9 | MeS(CH₂)₂- | 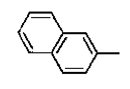 | R | 1.68-1.92 (m, 2H), 1.93 (s, 3H), 2.27-2.48 (m, 2H), 3.91 (m, 1H), 7.55-7.63 (m, 2H), 7.65 (d, J=8.7 Hz, 1H), 7.74 (d, J=8.1 Hz, 2H), 7.82-7.90 (m, 4H), 7.91-8.02 (m, 5H), 8.23 (s, 1H), 8.25 (br s, 1H), 12.70 (br s, 1H) |
| III-10 | MeS(CH₂)₂- | 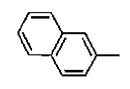 | S | 1.68-1.92 (m, 2H), 1.93 (s, 3H), 2.28-2.47 (m, 2H), 3.90 (m, 1H), 7.56-7.62 (m, 2H), 7.65 (dd, J=1.5, 8.4 Hz, 1H), 7.74 (d, J=8.1 Hz, 2H), 7.83-7.88 (m, 4H), 7.92-8.00 (m, 5H), 8.23 (s, 1H), 8.27 (br s, 1H) |
| III-11 | Bn | 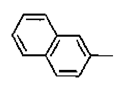 | R | 2.75 (dd, J=9.0, 13.8Hz, 1H), 2.97 (dd, J=6.0, 13.8Hz, 1H), 3.92 (m, 1H), 7.15-7.2D (m, 5H), 7.57-7.66 (m, 5H), 7.73-7.83 (m, 6H), 7.95-8.00 (m, 3H), 8.23 (s, 1H), 8.36 (d, J=8.7Hz, 1H), 12.80 (br, 1H) |

**Table 2**

| Compound No. | R² | R^{A} | * | 1H-NMR (DMSO-d₆) |
|---|---|---|---|---|
| III-12 | Bn | 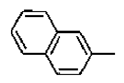 | S | 2.75 (dd, J=9.3, 13.5 Hz, 1H), 2.97 (dd, J=5.4, 13.5 Hz, 1H), 3.92 (m, 1H), 7.10-7.24 (m, 5H), 7.56-7.69 (m, 5H), 7.72-7.85 (m, 6H), 7.94-8.02 (m, 3H), 8.23 (s, 1H), 8.38 (d, J=9.0 Hz, 1H), 12.80 (br s, 1H) |
| III-13 | 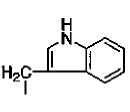 | 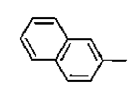 | R | 2.88 (dd, J=8.4, 14.1Hz, 1H), 3.08 (dd, J=6.0, 15.3Hz, 1H), 3.93 (m, 1H), 6.92 (m, 1H), 6.99 (m, 1H), 6.99 (m, 1H). 7.09 (d, J=2.1Hz, 1H), 7.22 ((d, J=8.1Hz, 1H), 7.30 (d, J=7.8Hz, 1H), 7.57-7.81 (m, 11H), 7.97-8.00 (m, 3H), 8.23 (s, 1H), 8.32 (d, J=8.1Hz, 1H), 10.80 (s, 1H), 12.50 (br, 1H) |
| III-14 | 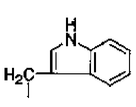 | 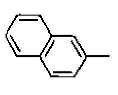 | S | 1.70~1.90 (m, 2H), 1.93 (s; 3H), 2.30~2.43 (m, 2H), 3.90 (s, 4H), 7.23 (dd, J=2.4, S 9.9Hz, 1H), 7.38 (d, J=2.1Hz), 7.59 (dd, J=1.5, 8.4Hz, 1H), 7.71 (d, J=8.4Hz, 2H), 7.81~7.96 (m, 8H), 8.14 (s.1H), 8.25 (br, 1H), 12.40 (br, 1H) |
| III-15 | 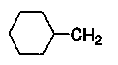 | 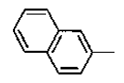 | R | 0.58-1.68 (m, 13H), 3.70 (m, 1H), 7.55-7.70 (m, 3H), 7.74 (d, J=8.4 Hz, 2H), 7.80-7.90 (m, 4H), 7.91-8.04 (m, 5H), 8.18-8.80 (m, 2H), 12.63 (br s, 1H) |
| III-16 | Bn | 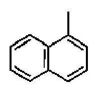 | R | 2.75 (dd, J=9.0, 13.8 Hz, 1H), 2.97 (dd, J=5.7, 13.8 Hz, 1H), 3.92 (m, 1H), 7.10-7.24 (m, 5H), 7.55-7.89 (m, 12H), 8.03 (d, J=8.1 Hz, 2H), 8.36 (br s, 1H), 8.42 (d, J=8.4 Hz, 1H), 12.80 (br s, 1H) |
| III-17 | Me | 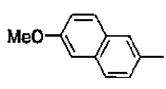 | R | 1.18 (d, J=6.9 Hz, 3H), 3.79 (m, 1H), 3.89 (s, 3H), 7.23 (dd, J=2.6, 8.9 Hz, 1H), 7.37 (d, J= 2.4 Hz, 1H), 7.59 (dd, J=1.8, 8.1 Hz, 1H), 7.71 (d, J=8.4 Hz, 2H), 7.82-7.94 (m, 8H), 8.13 (s, 1H), 8.18 (br s, 1H) |
| III-18 | Me | 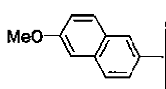 | S | 1.19 (d, J=7.2 Hz, 3H), 3.82 (m, 1H), 3.90 (s, 3H), 7.24 (dd, J=2.4, 8.7 Hz, 1H), 7.38 (d, J= 2.4 Hz, 1H), 7.59 (dd, J=1.5; 8.7 Hz, 1H), 7.71 (d, J=8.4 Hz, 2H), 7.81-7.91 (m, 6H), 7.94 (d, J =8.4 Hz, 2H), 8.14 (s, 1H), 8.22 (br s, 1H), 12.61 (br s, 1H) |
| III-19 | Et | 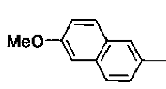 | R | 0.80 (t, J = 7.5Hz, 3H), 1.48 - 1.69 (m, 2H), 3.66 (m, 1H), 3.90 (s, 3H), 7.23 (dd, J = 2.4, 9.0 Hz, 1H), 7.38 (d, J = 2.4 Hz, 1H), 7.59 (dd, J = 1.5, 8.4 Hz, 1H), 7.71 (d, J = 8.4 Hz, 2H), 7.82-7.95 (m, 8H), 8.14 (s, 1H), 8.20 (d, J = 8.7 Hz, 1H) |
| III-20 | i-Pr | 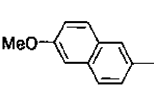 | R | 0.80-0.86 (m, 6H), 1.96 (m, 1H), 3.56 (m, 1H), 3.90 (s, 3H), 7.23 (dd, J = 2.6, 8.7 Hz, 1H). 7.39 (d, J = 2.4 Hz, 1H), 7.59 (dd, J = 1.4, 8.6 Hz, 1H), 7.71 (d, J = 8.4 Hz, 2H), 7.82-7.95 (m, 8H), 8.11 (br d, J = 9.6 Hz, 1H), 8.15 (s, 1H) |

**Table 3**

| Compound No. | R² | R^{A} | | 1H-NMR (DMSO-d₆) |
|---|---|---|---|---|
| III-21 | i-Pr | 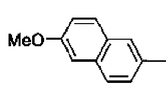 | S | 0.81-0.87 (m, 6H), 1.97 (m, 1H), 3.57 (m, 1H), 3.91 (s, 3H), 7.25 (dd, J = 2.4, 9.0 Hz, 1H), 7.39 (d, J = 2.4 Hz, 1H), 7.60 (dd, J = 1.4, 8.6 Hz, 1H), 7.72 (d, J = 8.4 Hz, 2H), 7.83-7.95 (m, 8H), 8.11 (br d, J = 8.7 Hz, 1H), 8.14 (s, 1H) |
| III-22 | i-Bu | 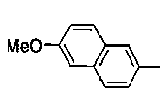 | R | 0.72 (d, J=6.6Hz, 3H), 0.83 (d, J=6.6 Hz, 3H), 1.33-1.50 (m, 2H), 1.60 (m, 1H), 3.70 (m, 1H), 3.90 (s, 3H), 7.23 (dd, J=2.4, 9.3 Hz, 1H), 7.38 (d, J=2.4 Hz, 1H), 7.59 (d, J=8.4 Hz, 1H), 7.71 (d, J=8.4 Hz, 2H), 7.81-7.96 (m, 8H), 8.14 (a, 1H), 8.22 (d, J=8.4 Hz, 1H), 12.60 (br s, 1H) |
| III-23 | s-Bu. | 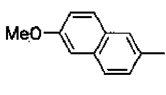 | S | 0.74-0.83 (m, 6H), 1.12 (m, 1H), 1.38 (m, 1H), 1.69 (m, 1H), 3.60 (m, 1H), 3.90 (s, 3H), 7.24 (dd, J = 2.4, 9.0 Hz, 1H), 7.38 (d, J = 2.7 Hz, 1H), 7.59 (dd, J = 1.5, 8.4 Hz, 1H), 7.71 (d, J = 8.1 Hz, 2H), 7.82-7.94 (m, 8H), 8.11 (s, 1H), 8.14 (s, 1H) |
| III-24 | MeS(CH₂)₂- | 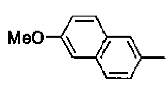 | R | 1.70-1.90 (m, 2H), 1.93 (s, 3H), 2.30-2.42 (m, 2H), 3.90 (s, 4H), 7.24 (dd, J=2.4, 9.0Hz, 1H), 7.38 (d, J=2.1Hz, 1H), 7.59 (dd, J=1.5, 8.4Hz, 1H), 7.71 (d, J=8.4Hz, 2H), 7.82-7.95 (m, 8H), 8,14 (s, 1H), 8.28 (d, J=9.0Hz, 1H), 12.64 (br, 1H) |
| III-25 | MeS(CH₂)₂- | 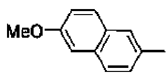 | S | 1.70-1.90 (m, 2H), 1.93 (s, 3H), 2.30-2.43 (m, 2H), 3.90 (s, 4H), 7.23 (dd, J=2.4, 9.9Hz, 1H), 7.38 (d, J=2.1Hz), 7.59 (dd, J=1.5, 8.4Hz, 1H). 7.71 (d, J=8.4Hz, 2H), 7.81-7.96 (m, 8H), 8.14 (s,1H), 8.25 (br, 1H), 12.40 (br, 1H) |
| III-26 | HO₂CCH₂- | 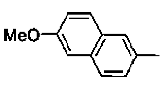 | R | 2.42-2.67 (m, 2H), 3.90 (s, 3H), 4.11 (br m, 1H), 7.24 (dd, J = 2.4, 9.0 Hz, 1H), 7.38 (d, J = 2.4 Hz, 1H), 7.60 (dd, J = 1.5, 8.4 Hz, 1H), 7.71 (d, J= 8.4 Hz, 2H), 7.83-7.95 (m, 8H), 8.14 (s, 1H), 8.30 (br s, 1H) |
| III-27 | HO₂C(CH₂)₂- | 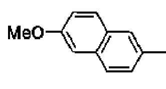 | R | 1.69 (m, 1H), 1.87 (m, 1H), 2.23 (t, J = 7.2 Hz, 2H), 3.82 (m, 1H), 3.90 (s, 3H), 7.24 (dd, J = 2.4, 9.0 Hz, 1H), 7.38 (d, J = 2.4 Hz, 1H), 7.59 (dd, J = 1.5, 8.4 Hz, 1H), 7.71 (d, J = 8.1 Hz, 2H), 7.82-7.94 (m, 8H), 8.14 (s, 1H), 8.22 (br s, 1H) |
| III-28 | Bn | 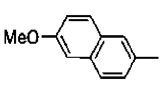 | R | 2.74 (dd, J=9.3, 13.8 Hz, 1H), 2.97 (dd, J=5.7,13.8 Hz, 1H), 3.90 (s, 3H), 3.92 (m, 1H), 7.11-7.21 (m, 5H), 7.24 (dd, J=2.4, 8.7 Hz, 1H), 7.39 (d, J=2.4 Hz, 1H), 7.60 (dd, J=1.8, 8.7 Hz, 1H), 7.64 (d, J=8.7 Hz, 2H), 7.72 (d, J=8.4 Hz, 2H), 7.74-7.84 (m, 4H), 7.88 (dd, J=4.8, 8.7 Hz, 1H), 8.15 (s, 1H), 8.37 (br s, 1H), 12.74 (br s, 1H) |

**Table 4**

| Compound No. | R₂ | R^{A} | * | 1H-NMR (DMSO-d₆) |
|---|---|---|---|---|
| III-29 | Bn | 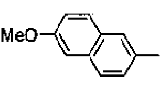 | S | 2.74 (dd, J=9.3, 13.8 Hz, 1H), 2.97 (dd, J=5.6, 13.7 Hz, 1H), 3.90 (s, 4H), 7.12-7.20 (m, 5H), 7.24 (dd, J=2.4, 8.7 Hz, 1H), 7.39 (d, J=2.4 Hz, 1H), 7.58-7.91 (m, 11H), 8.15 (s, 1H), 8.36 (d, J = 8.4 Hz, 1H) |
| III-30 | 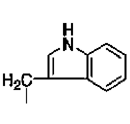 | 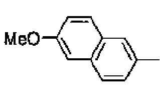 | R | 2.62 (dd, J=6.6, 14.7Hz, 1H), 3.08 (dd, J=6.3, 14.1Hz, 1H), 3.90 (s, 4H), 6.90 (m, 1H), 6.96 (m, 1H), 7.08 (d, J=2.1Hz, 1H), 7.24 (m, 2H), 7.32 (d, J=8.1Hz, 1H), 7.38 (d, J=2.4Hz, 1H), 7.59-7.64 (m, 3H), 7.71 (d, J=9.0Hz, 2H), 7.79 (d, J=8.4Hz, 2H), 7.88 (d, J=8.4Hz, 1H), 7.89 (d, J=9.0Hz, 1H), 8.14 (s, 1H), 8.25 (br, 1H), 10.29 (s, 1H), 12.70 (br, 1H) |
| III-31 | 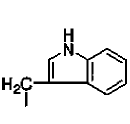 | 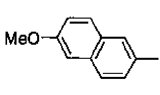 | S | 2.89 (dd, J=8.1, 14.4Hz, 1H), 3.09 (dd, J=8.1, 14.4Hz, 1H), 3.90 (s, 3H), 3.95 (m, 1H), 6.90 (t, J=7.8Hz, 1H), 6.99 (t, J=7.5Hz, 1H), 7.10 (d, J=2.1Hz, 1H), 7.22-7.25 (m, 2H), 7.31 (d, J=8.1Hz, 1H), 7.38 (d, J=2.1Hz, 1H), 7.58-7.80 (m, 9H), 7.88 (dd, J=5.1, 9.0Ha, 2H), 8.15 (s, 1H), 8.33 (d, J=8.7Hz, 1H), 10.80 (s, 1H), 12.60 (br, 1H) |
| III-32 | HO₂CCH₂OBn | 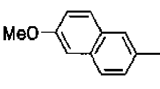 | R | 2.68 (dd, J=9.0, 13.5 Hz, 1H), 2.91 (dd, J=5.1, 13.5 Hz, 1H), 3.84 (m, 1H), 3.91 (s, 3H), 4.56 (s, 2H), 6.71 (d, J=8.4 Hz, 2H), 7.04 (d, J=8.4 Hz, 2H), 7.24 (dd, J=2.1, 8.7 Hz, 1H), 7.39 (d, J=2.1 Hz, 1H), 7.57-7.64 (m, 3H), 7.71 (d, J=8.4 Hz, 2H), 7.75-7.85 (m, 4H), 7.89 (dd, J=4.2, 8.7 Hz, 1H), 8.15 (s, 1H), 8.33 (br s, 1H), 12.60 (br s, 2H) |
| III-33 | Me | 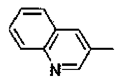 | S | 1.19 (d, J=7.2 Hz, 3H), 3.82 (m, 1H), 7.65-7.98 (m, 10H), 8.06 (t, J=8.7 Hz, 2H), 8.23 (d, J=8.1 Hz, 1H), 8.67 (d, J=2.1 Hz, 1H), 9.04 (d, J=2.1 Hz, 1H), 12.80 (br s, 1H) |
| III-34 | i-Pr | 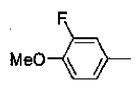 | S | 0.81 (d, J=6.9Hz, 3H), 0.84 (d, J=6.9Hz, 3H), 1.96 (m, 1H), 3.55 (m, 1H), 3.90 (s, 3H), 7.23 (m, 1H), 7.40 (m, 1H), 7.47 (dd, J=2.1, 12.0Hz, 1H), 7.66 (d, J=8.4Hz, 2H), 7.81-7.93 (m, 6H), 8.10 (d, J=9.0Hz, 1H), 12.50 (br, 1H) |
| III-35 | Bn | 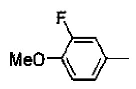 | R | 2.74 (dd, J=9.0, 13.8Hz, 1H), 2.96 (dd, J=5.7, 13.8Hz, 1H), 3.89 (s, 4H), 7.10-7.26 (m, 6H), 7.40 (m, 1H), 7.47 (dd, J=2.1, 12.0Hz, 1H), 7.61-7.68 (m, 4H), 7.75-7.80 (m, 4H), 8.33 (d, J=9.0Hz, 1H), 12.70 (br, 1H) |

**Table 5**

| Compound No. | R² | R^{A} | * | 1H-NMR (DMSO-d₆) |
|---|---|---|---|---|
| III-36 | 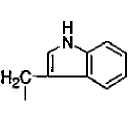 | 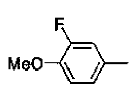 | S | 2.66 (dd, J=8.1, 14.4Hz, 1H), 2.86 (dd, J=6.3, 14.7Hz, 1H), 3.66 (s, 3H), 3.70 (br, 1H), 6.67 (m, 1H), 6.76 (m, 1H), 6.86 (d, J=2.1Hz, 1H), 7.00 (d, J=8.7Hz, 2H), 7.07 (t, J=7.8Hz, 1H), 7.19 (m, 1H), 7.26 (dd, J=2.1, 11.7Hz, 1H), 7.39 (d, J=8.4Hz, 2H), 7.44 (d, J=6.3Hz, 2H), 7.47 (d, J=6.0Hz, 2H), 7.54 (d, J=8.7Hz, 2H), 8.08 (m, 1H), 10.57 (s, 1H) |
| III-37 | MeS(CH₂)₂- | 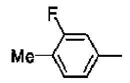 | S | 1.60-1.64 (m, 2H), 1.73 (s, 3H), 2.10 (s, 3H), 2.12-2.14 (m, 2H), 7.09-7.18 (m, 3H), 7.45 (d, J=8.4Hz, 2H), 7.58 (d, J=8.1Hz, 2H), 7.63-7.70 (m, 5H) |
| III-38 | Bn | 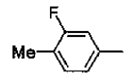 | S | 2.24 (s, 3H), 2.90 (m, 2H), 7.08-7.16(m, 5H), 7.28-7.37 (m, 3H), 7.65 (d, J=8.4Hz, 2H), 7.72-7.82 (m, 6H) |
| III-39 | 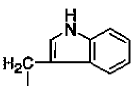 | 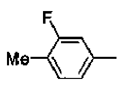 | S | 2.28 (s, 3H), 3.10 (m, 2H), 3.35 (m, 1H), 6.90 (m, 1H), 6.98 (m, 1H), 7.06 (s, 1H), 7.24 (d, J=8.1Hz, 1H), 7.31-7.41 (m, 3H), 7.53 (d, J=7.2Hz, 1H), 7.67 (d, J=7.8Hz, 2H), 7.73-7.78 (m, 7H), 10.68 (s, 1H) |
| III-40 | Bn | 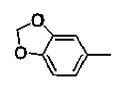 | R | 2.74 (dd, J=9.0, 13.8 Hz, 1H). 2.96 (dd, J=5.4, 13.8 Hz, 1H), 3.92 (m, 1H), 6.10 (s, 2H), 6.99 (d, J=8.1 Hz, 1H), 7.10-7.22 (m, 7H), 7.60-7.68 (m, 4H), 7.73-7.80 (m, 4H), 8.36 (d, J=9.0 Hz, 1H), 12.79 (br s, 1H) |
| III-41 | Bn | 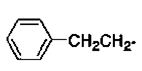 | R | 2.69-2.77 (m, 3H), 2.85-2.99 (m, 3H), 3.90 (m, 1H), 7.11-7.27 (m, 6H), 7.33 (d, J = 4.2 Hz, 4H), 7.46 (d, J = 8.1 Hz, 2H), 7.60 (d, J = 8.4 Hz, 2H), 7.67-7.72 (m, 4H), 8.34 (d, J =9.0 Hz, 1H), 12.74 (br s, 1H) |
| III-42 | n-Pr | 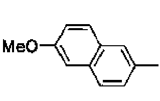 | S | 0.77 (t, J=7.2 Hz, 3H), 1.18-1.32 (m, 2H), 1.45-1.60 (m, 2H), 3.70 (m, 1H), 3.90 (s, 3H), 7.23 (dd, J=2.1, 8.7 Hz, 1H), 7.38 (d, J=2.4 Hz, 1H), 7.59 (dd, J=1.5, 8.7 Hz, 1H), 7.71 (d, J=8.4 Hz, 2H), 7.83-7.95 (m, 8H), 8.14 (s, 1H), 8.19 (d, J=9.0 Hz, 1H), 12.63(brs, 1H) |
| III-43 | n-Bu | 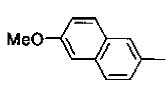 | S | 0.75 (t, J=6.6 Hz, 3H), 1.10-1.22 (m, 4H), 1.45-1.62 (m, 2H), 3.68 (m,1H), 3.90 (s, 3H), 7.23 (dd, J=2.4, 9.6 Hz, 1H), 7.38 (d, J=2.4 Hz, 1H), 7.59 (dd, J=1.8, 8.4 Hz, 1H), 7.71 (d, J=8.4Hz, 2H), 7.82-7.95 (m, 8H), 8.14 (s, 1H), 8.19 (d, J=8.7 Hz, 1H), 12.63 (br s, 1H) |
| III-44 | HOCH₂- | 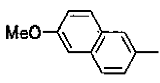 | R | 3.50-3.60 (m, 2H), 3.80 (m, 1H), 3.90 (s, 3H), 7.23 (dd, J=2.4, 8.7 Hz, 1H), 7.38 (d, J=2.4 Hz, 1H), 7.59 (dd, J=1.5, 8.4 Hz, 1H), 7.71 (d, J=8.4 Hz, 2H), 7.83-7.95 (m, 9H), 8.08 (d, J=8.7 Hz, 1H), 8.14 (s, 1H) |

**Table 6**

| Compound No. | R² | R^{A} | * | 1H-NMR (DMSO-d₆) |
|---|---|---|---|---|
| III-45 | HOCH₂- | 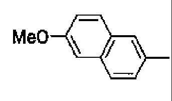 | S | 3.50-3.60 (m, 2H), 3.80 (m, 1H), 3.90 (s, 3H), 5.00 (br, 1H), 7.23 (dd, J=2.4, 9.0 Hz, 1H), 7.38 (d, J=2.4 Hz, 1H), 7.59 (dd, J=1.5, 8.4 Hz, 1H), 7.71 (d, J=8.7 Hz, 2H), 7.83-7.95 (m, 8H), 8.10 (d, J=8.7 Hz, 1H), 8.14 (s, 1H), 12.57 (br, 1H) |
| III-46 | 4-OH-Bn | 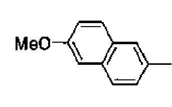 | R | 2.63 (dd, J=8.4, 14.1 Hz, 1H), 2.84 (dd, J=6.3, 14.1 Hz, 1H), 3.80 (m, 1H), 3.90 (s, 3H), 6.59 (d, J=8.4 Hz, 2H), 6.92 (d, J=8.4 Hz, 2H), 7.23 (dd, J=2.4, 9.0 Hz, 1H), 7.38 (d, J=2.4 Hz, 1H), 7.58-7.90 (m, 11H), 8.14 (s, 1H), 8.29 (d, J=9.3 Hz, 1H), 9.22 (s, 1H), 12.65 (br, 1H) |
| III-47 | 4-F-Bn | 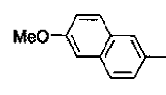 | RS | 2.72 (dd, J=9.6, 13.8 Hz, 1H), 2.97 (dd, J=5.4, 13.5 Hz, 1H), 3.90 (m, 1H), 3.90 (s, 3H), 6.96 (t, J=9.0 Hz, 2H), 7.24 (dd, J=2.4, 9.0 Hz, 2H), 7.39 (d, J=2.4 Hz, 1H), 7.58-7.91 (m, 11H), 8.14 (s, 1H), 8.37 (d, J=8.7 Hz, 1H), 12.82 (s, 1H) |
| III-48 | 4-OMe-Bn | 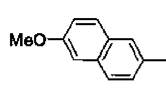 | S | 2.66 (dd, J=9.6, 13.8 Hz, 1H), 2.89 (dd, J=5.1, 13.8 Hz, 1H), 3.62 (s, 3H), 3.82 (m, 1H), 3.90 (s, 3H), 6.71 (d, J=8.7 Hz, 2H), 7.03 (d, J=8.7 Hz, 2H), 7.23 (dd, J=2.4, 8.7 Hz, 1H), 7.38 (d, J=2.1 Hz, 1H), 7.58-7.63 (m, 6H), 7.70-7.82 (m, 6H), 7.88 (dd, J=5.4, 9.3 Hz, 2H), 8.14 (s, 1H), 8.29 (d, J=9.0 Hz, 1H), 12.70 (br, 1H) |
| III-49 | MeSCH₂- | 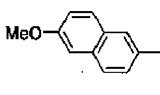 | S | 1.98 (s, 3H), 2.60 (dd, J=7.5, 13.5 Hz, 1H), 2.75 (dd, J=6.5, 13.8 Hz, 1H), 3.90 (m, 1H), 3.90 (s, 3H), 7.23 (dd, J=2.7, 9.0 Hz, 1H), 7.38 (d, J=2.7 Hz, 1H), 7.59 (dd, J=1.8, 8.4 Hz, 1H), 7.71 (d, J=8.7 Hz, 2H), 7.83-7.95 (m, 8H), 8.14 (a, 1H), 8.39 (d, J=8.4 Hz, 1H), |
| III-50 | MeOCH₂- | 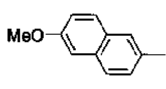 | RS | 3.15 (s, 3H), 3.48-3.55 (m, 2H), 3.90 (s, 3H), 4.00 (m, 1H), 7.23 (dd, J=2.4, 9.0 Hz, 1H), 7.38 (d, J=2.4 Hz, 1H), 7.59 (dd, J=1.5, 8.4 Hz, 1H), 7.71 (d, J=8.7 Hz, 2H), 7.83-7.94 (m, 8H), 8.14 (s, 1H),8.30 (d, J=7.5 Hz, 1H) |
| III-51 | 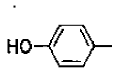 | 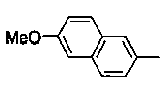 | R | 3.90 (s, 3H), 4.80 (d, J=9.0 Hz, 1H), 6.62 (d, J=8.7 Hz, 2H), 7.07 (d, J=8.7 Hz, 2H), 7.24 (dd, J=2.1, 8.7 Hz, 1H), 7.38 (d, J=2.1 Hz, 1H), 7.60 (dd, J=1.5, 8.4 Hz, 1H), 7.70 (d, J=8.4 Hz, 2H), 7.79-7.90 (m, 8H), 8.14 (s, 1H), 8.63 (d, J=8.7 Hz, 1H), 9.45 (s, 1H), 12.82 (s, 1H) |
| III-52 | 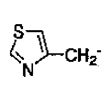 | 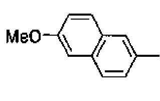 | S | 2.96 (dd, J=9.0, 14.4 Hz,1H), 3.12 (dd, J=5.4, 14.4 Hz, 1H), 3.90 (s, 3H), 4.15 (m, 1H), 7.23 (dd, J=2.7, 9.0 Hz, 1H), 7.31 (m, 1H), 7.38 (d, J=2.7 Hz, 1H), 7.60 (d, J=8.4 Hz, 1H), 7.65 (d, J=8.4 Hz, 2H), 7.71 (d, J=8.1 Hz, 2H), 7.80-7.90 (m, 6H), 8.14 (s, 1H), 8.34 (d, J=8.4 Hz, 1H), 8.89 (s, 1H), 12.78 (brs, 1H) |

**Table 7**

| Compound No. | R² | R^{A} | * | 1H-NMR. (DMSO-d₆) |
|---|---|---|---|---|
| III-53 | Me | 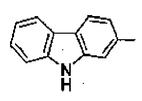 | S | 1.19 (d, J=7.5 Hz, 3H), 3.80 (br, s, 1H), 7.20 (dt, J=1.2, 6.9 Hz, 1H), 7.43 (dt, J=1.2, 8.1 Hz, 1H), 7.50-7.61 (m, 2H), 7.69 (d, J=8.4 Hz, 2H), 7.74-7.95 (m, 6H), 8.20 (d, J=7.8 Hz, 2H), 8.42 (s, 1H), 11.53 (s, 1H). 12.60 (br, s, 1H) |
| III-54 | Me | 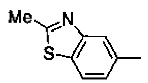 | S | 1.19 (d, J=7.2 Hz, 3H), 2.83 (s, 3H), 3.82 (m, 1H), 7.59 (m, 1H), 7.72 (d, J=8.4 Hz, 2H), 7.89 (m, 6H), 8.12 (m, 2H), 8.23 (d, J=8.4 Hz, 1H) |
| III-55 | HOCH₂- | 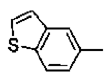 | S | 3.52-3.64 (m, 3H), 3.77-3.85 (m, 2H), 7.51 (d, J=6.3 Hz, 1H), 7.54 (dd, J=1.5, 8.1 Hz, 1H), 7.71 (d, J=8.4 Hz, 2H), 7.82-7.94 (m, 6H), 8.05-8.15 (m, 3H) |
| III-56 | HOCH₂- | 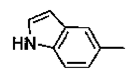 | S | 3.50-3.58 (m, 2H), 3.80 (m, 1H), 6.48 (s, 1H), 7.28 (dd, J=1.5, 8.4 Hz, 1H), 7.42-7.46 (m, 2H), 7.66 (d, J=8.4 Hz, 2H), 7.78-7.84 (m, 3H), 7.86-7.93 (m, 4H), 8.06 (d, J=8.7 Hz, 1H), 11.34 (s, 1H) |
| III-57 | HOCR₂- | 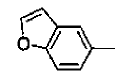 | S | 3.50-3.60 (m, 2H), 3.80 (m, 1H), 7.53 (dd, J=1.5, 8.4 Hz, 1H), 7.67-7.71 (m, 3H), 7.83 (d, J=8.7 Hz, 2H), 7.87-7.94 (m, 6H), 8.08-8.09 (m, 2H), 12.50 (br, 1H) |
| III-58 | Me | 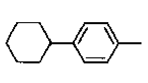 | S | 1.18 (d, J=7.2 Hz, 3H), 1.17-1.48 (m, 5H), 1.63-1.90 (m, 6H), 3.77 (d, J=7.2 Hz, 1H), 7.30 (d, J=8.4 Hz, 1H), 7.50 (d, J=8.1 Hz, 2H), 7.66 (d, J=8.1 Hz, 2H), 7.84-7.94 (m, 6H), 8.16 (br, s, 1H), 12.80 (br, s, 1H) |
| III-59 | Me | 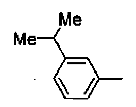 | S | 1.18 (d, J=7.2 Hz, 3H), 1.23 (d, J=7.2 Hz, 6H), 2.93 (m, 1H), 3.70 (m, 1H), 7.36 (m, 3H), 7.46 (s, 1H), 7.67-7.70 (m, 2H), 7.81-7.94 (m, 6H), 8.09 (br, s, 1H) |
| III-60 | Me | 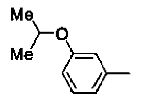 | S | 1.18 (d, J=7.2 Hz, 3H), 1.28 (d, J=6.0 Hz, 6H), 3.80 (br, s, 1H), 4.68 (m, 1H), 6.99 (m, 1H), 7.12 (m, 2H), 7.33 (t, J=7.8 Hz, 1H), 7.67-7.70 (m, 2H), 7.81-7.94 (m, 6H), 8.20 (d, J=6.6 Hz, 1H), 12.67 (br, s, 1H) |
| III-61 | Me | 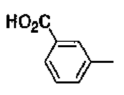 | S | 1.78 (d, 3H), 3.64 (s, 2H), 3.82 (t, J = 6.6Hz, 1H), 7.30-7.42 (m, 4H), 7.69 (d, J = 8.4 Hz, 2H), 7.80-7.95 (m, 6H), 8.23 (m, J = 8.4 Hz, 1H), 12.74 (br s, 2H) |
| III-62 | Me | 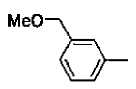 | S | 1.18 (d, 3H), 3.32 (s, 3H), 3.80 (q, J = 7.2, 14 Hz, 1H), 4.45 (s, 2H), 7.36-7.55 (m, 4H), 7.70 (d, J = 5.4 Hz, 2H), 7.81-7.95 (m, 6H), 8.17 (bs, 1H) |

**Table 8**

| Compound No. | R² | R^{A} | * | 1H-NMR (DMSO-d₆) |
|---|---|---|---|---|
| III-63 | iPr | 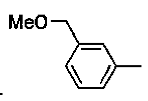 | S | 0.815 (d, J = 6.6 Hz, 3H), 0.846 (d, J = 6.6 Hz, 3H), 1.90-2.01 (m, 1H), 3.32 (s, 3H), 3.56 (t, J = 8.4 Hz, 1H), 4.45 (s, 2H), 7.35-7.55 (m, 4H), 7.70 (d, J = 5.4 Hz, 2H), 7.80-7.94 (m, 6H), 8.10 (d, J = 10 Hz, 1H) |
| III-64 | iBu | 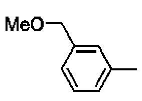 | S | 2004-0305-119-01 0.72 (d, J = 6.6 Hz, 3H), 0.83 (d, J = 6.6 Hz, 3H), 1.38-1.46 (m, 2H), S 1.55-1.66 (m, 1H), 3.32 (s, 3H), 3.69-3.74 (m, 1H), 4.45 (s, 2H), 7.35-7.55 (m, 4H), 7.70 (d, J = 6.6 Hz, 2H), 7.80-7.96 (m, 6H), 8.22 (d, J = 9.0 Hz, 1H), 12.6 (bs, 1H) |

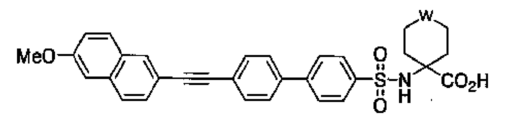

**Table 9**

| Compound No. | W | 1H-NMR (DMSO-d₆) |
|---|---|---|
| III-65 | Bond | 1.40-1.60 (m, 4H), 1.85-2.00 (m, 4H), 3.91 (s, 3H), 7.24 (dd, J=2.4, 8.7 Hz, 1H), 7.39 (d, J=2.6 Hz, 1H), 7.60 (dd, J=1.2, 9.0 Hz, 1H), 7.71 (d, J=8.4 Hz, 2H), 7.83-7.95 (m, 8H), 8.12 (d. J=11.4 Hz, 1H), 12.49 (s, 1H) |
| III-66 | CH₂ | 1.20-1.40 (m, 6H), 1.65-1.88 (m, 4H), 3.90 (s, 3H), 7.23 (dd, J=2.7, 9.0 Hz, 1H), 7.38 (d, J=2.4 Hz, 1H), 7.59 (dd, J=1.2, 8.4 Hz, 1H), 7.71 (d, J=8.7 Hz, 2H), 7.83-7.94 (m, 9H), 8.14 (s, 1H), 12.42 (s, 1H) |
| III-67 | O | 1.80-1.92 (m, 4H), 3.30-3.50 (m, 4H), 3.90 (s, 3H), 7.23 (dd, J=2.4, 8.4 Hz, 1H), 7.38 (d, J=2.7 Hz, 1H), 7.60 (dd, J=1.2, 8.4 Hz, 1H), 7.71 (d, J=8.1 Hz, 2H), 7.84-7.90 (m, 6H), 7.94 (d, J=8.7 Hz, 2H), 8.14 (s, 1H), 8.21 (s, 1H), 12.70 (br, 1H) |

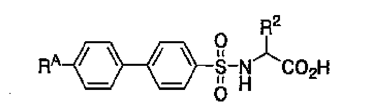

**Table 10**

| Compound No. | R² | R^{A} | * | 1H-NMR (DMSQ-d₆) |
|---|---|---|---|---|
| II-2 | MeS(CH₂)₂- | 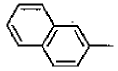 | R | 1.74-1.94 (m, 2H), 1.93 (s, 3H), 2.31-2.50 (m, 2H), 3.93 (s, 4H), 7.53-7.58 (m, 2H), 7.86-8.06 (m, 12H), 8.28 (d, J=9.0Hz, 1H), 8.33 (br, 1H), 12.81 (br, 1H) |
| II-3 | Bn | 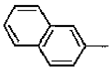 | R | 2.76 (dd, J=9.3, 13.8Hz, 1H), 2.98 (dd, J=5.7, 14.1Hz, 1H), 3.93 (m, 1H), 7.10-7.22 (m, 5H), 7.52-7.67 (m, 4H), 7.79-8.07 (m, 10H), 8.33 (s, 1H), 8.36 (m, 1H), 12.80 (br, 1H) |
| II-4 | Bn | 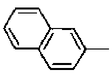 | S | 2.53 (dd, J=9.3, 13.5 Hz, 1H), 2.76 (dd, J=6.0,13.5 Hz, 1H), 3.71 (m, 1H), 6.93-7.01 (m, 5H), 7.34 (m, 2H), 7.50 (d, J=8.4 Hz, 2H), 7.58 (d, J=8.7 Hz, 2H), 7.65 (d, J=8.4 Hz, 2H), 7.70-7.88 (m, 5H), 8.10-8.21 (m, 2H) |
| II-5 | Bn | 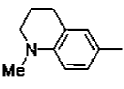 | R | 1.90-1.94 (m, 2H), 2.70-2.81 (m, 3H), 2.88 (s, 3H), 2.97 (dd, J=6.0, 13.0 Hz, 1H), 3.93 (m, 1H), 6.66 (d, 1H, J=9.0 Hz), 7.12-7.19 (m, 5H), 7.32 (s, 1H), 7.41 (d, J=9.0 Hz, 1H), 7.61 (d, J=8.4 Hz, 2H), 7.71-7.75 (m, 6H), 8.33 (d, J=9.0 Hz, 1H), 12.78 (br s, 1H) |

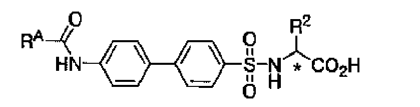

**Table 11**

| Compound No. | R² | R^{A} | * | 1H-NMR (DMSO-d₆) |
|---|---|---|---|---|
| VI-2 | Bn | 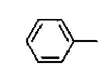 | R | 2.74 (dd, J=9.0, 13.5 Hz, 1H). 2.96 (dd, J=5.1, 13.5 Hz, 1H), 3.91 (m, 1H), 7.10-7.24 (m, 5H), 7.51-7.66 (m, 5H), 7.69-7.79 (m, 4H), 7.91-8.03 (m, 4H), 8.31 (d, J=9.0 Hz, 1H), 10.41 (s, 1H), 12.74 (br s, 1H) |
| VI-3 | Bn | 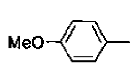 | R | 2.74 (dd, J=9.0, 13.5 Hz, 1H), 2.96 (dd, J=5.4, 13.5 Hz, 1H), 3.85 (s, 3H), 3.90 (m, 1H), 7.05-7.24 (m, 7H), 7.61 (d, J=8.4 Hz, 2H), 7.69-7.76 (m, 4H), 7.93 (d, J=9.0 Hz, 2H), 7.99 (d, J=9.0 Hz, 2H), 8.29 (d, J=8.7 Hz, 1H), 10.25 (s, 1H), 12.79 (br s, 1H) |
| VI-4 | Bn | 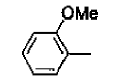 | R | |
| VI-5 | Bn | 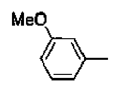 | R | 2.74 (dd, J=9.0, 13.8 Hz, 1H), 2.96 (dd, J=5.7, 13.8 Hz, 1H), 3.85 (s, 3H), 3.91 (m, 1H), 7.11-7.23 (m, 6H), 7.44-7.53 (m, 2H), 7.54-7.64 (m, 3H), 7.70-7.78 (m, 4H), 7.94 (d, J=8.7 Hz, 2H), 8.31 (d, J=7.8 Hz, 1H), 10.38 (s, 1H), 12.70 (br s, 1H) |
| VI-6 | Bn | 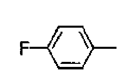 | R | 2.74 (dd, J=9.0, 13.8 Hz, 1H), 2.96 (dd, J=5.4, 13.8 Hz, 1H), 3.91 (m, 1H), 7.10-7.23 (m, 5H), 7.39 (t, J=8.7 Hz, 2H), 7.61 (d, J=8.7 Hz, 2H), 7.69-7.78 (m, 4H), 7.92 (d, J=8.7 Hz, 2H), 8.02-8.11 (m, 2H), 8.29 (d, J=8.1 Hz, 1H), 10.42 (s, 1H), 12.80 (br s, 1H) |
| VI-7 | Bn | 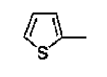 | R | 2.74 (dd, J=9.0, 13.8 Hz, 1H), 2.96 (dd, J=5.7, 13.8 Hz, 1H), 3:91 (m, 1H), 7.10-7.22 (m, 5H), 7.25 (dd, J=4.2, 4.5 Hz, 1H), 7.61 (d, J=8.4 Hz, 2H), 7.69-7.78 (m, 4H), 7.86-7.92 (m, 3H), 8.06 (d, J=3.6 Hz, 1H), 8.31 (d, J=8.1 Hz, 1H), 10.38 (s, 1H), 12.78 (br s, 1H) |
| VI-8 | Bn | 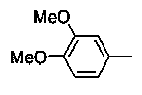 | R | 2.74 (dd, J=9.0, 13.5 Hz, 1H). 2.96 (dd, J=5.4, 13.5 Hz, 1H), 3.85 (s, 3H), 3.86 (s, 3H), 3.91 (m, 1H), 7.08-7.24 (m, 6H), 7.56 (d, J=1.8 Hz, 1H), 7.61 (d, J=8.7 Hz, 2H), 7.66 (dd, J=1.8, 8.4 Hz, 1H), 7.70-7.78 (m, 4H), 7.93 (d, J=8.7 Hz, 2H), 8.31 (d, J=8.4 Hz, 1H). 10.23 (s, 1H), 12.77 (br s, 1H) |
| VI-9 | Bn | 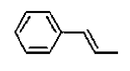 | R | 2.72 (dd, J=9.0, 13.5 Hz, 1H), 2.96 (dd, J=5.1, 13.5 Hz, 1H), 3.89 (m, 1H), 6.87 (d, J=16.2 Hz, 1H), 7.10-7.24 (m, 5H), 7.38-7.51 (m, 3H), 7.57-7.76 (m, 9H), 7.85 (d, J=8.4 Hz, 2H), 8.29 (d, J=8.4 Hz, 1H), 10.39 (s, 1H), 12.77 (br s, 1H) |

**Table 12**

| Compound No. | R² | R^{A} | * | 1H-NMR (DMSO-d₆) |
|---|---|---|---|---|
| VI-10 | Bn | 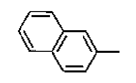 | R | 2.75 (dd, J=9.0, 13.5 Hz, 1H), 2.97 (dd, J=5.1, 13.5 Hz, 1H), 3.92 (m, 1H), 7.10-7.24 (m, 5H), 7.60-7.70 (m, 4H), 7.72-7.81 (m, 4H), 7.96-8.15 (m, 6H), 8.31 (d, J=9.0 Hz, 1H), 10.61 (s, 1H), 12.80 (br s, 1H) |
| VI-11 | Bn | 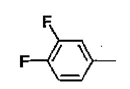 | R | 2.74 (dd, J=9.3, 13.5 Hz, 1H), 2.96 (dd, J=5.7, 13.5 Hz, 1H), 3.91 (m, 1H), 7.09-7.23 (m, 5H), 7.58-7.79 (m, 7H), 7.86-7.95 (m, 3H), 8.07 (ddd, J=1.8, 7.5, 11.1 Hz, 1H), 8.32 (d, J=9.0 Hz, 1H). 10.47 (s, 1H), 12.78 (br s, 1H) |
| VI-12 | Bn | 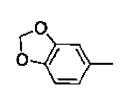 | R | 2.74 (dd, J=9.0, 13.5 Hz, 1H), 2.98 (dd, J=5.1, 13.5 Hz, 1H). 3.90 (m, 1H), 6.15 (s, 2H), 7.05-7.23 (m, 6H), 7.54 (d, J=1.8 Hz, 1H), 7.57-7.64 (m, 3H), 7.69-7.76 (m, 4H), 7.92 (d, J=8.7 Hz, 2H), 8.26 (d, J=8.4 Hz, 1H), 10.22 (s, 1H), 12.76 (br s, 1H) |
| VI-13 | Bn | 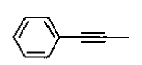 | R | 2.74 (dd, J=9.0, 13.5 Hz, 1H), 2.96 (dd, J=5.4, 13.5 Hz, 1H), 3.90 (m, 1H), 7.10-7.23 (m, 5H), 7.47-7.82 (m, 13H), 8.32 (d, J=8.4 Hz, 1H), 11.05 (s, 1H), 12.60 (br s, 1H) |
| VI-14 | Me | 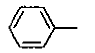 | R | |
| VI-15 | Me | 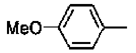 | R | 1.18(d, J=7.2 Hz, 3H), 3.77-3.79 (m, 1H), 3.85 (s, 3H), 7.08(d, J=9.0 Hz, 2H), 7.75-8.00 (m, 10H), 8.14 (br s. 1H), 10.25 (s, 1H) |
| VI-16 | Me | 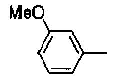 | R | 1.18 (d, J=7.5 Hz, 3H), 3.78-3.83 (m, 1H), 3.85 (s, 3H), 7.18 (d, J=8.4 Hz, 1H), 7.44-7.63 (m, 3H), 7.76-7.95 (m, 8H), 8.17 (d, J=8.4 Hz, 1H), 10.37 (s, 1H), 12.65 (br s, 1H) |
| VI-17 | Me | 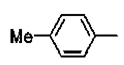 | R | 1.18 (d, J=7.2 Hz, 3H), 2.40 (s, 3H), 3.78-3.85 (m, 1H), 7.36 (d, J=8.4 Hz, 2H), 7.77 (d, J=8.7 Hz, 2H), 7.82-7.95 (m, 10H), 8.17 (d, J=8.1 Hz, 1H), 10.31 (s, 1H), 12.65 (br s, 1H) |
| VI-18 | Me | 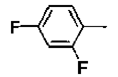 | R | 1.18 (d, J=7.5 Hz, 3H), 3.78-3.83 (m, 1H), 7.23-7.28 (m, 1H), 7.41-7.62 (m, 2H), 7.75-7.90 (m, 8H), 8.18 (d, J=8.4 Hz, 1H), 10.57 (s, 1H), 12.65 (br s, 1H) |
| VI-19 | Me | 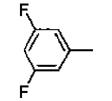 | R | |
| VI-20 | Me | 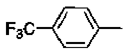 | R | 1.18 (d, J=7.2 Hz, 3H), 3.76-3.86 (m, 1H), 7.78-7.96 (m, 10H), 8.18 (d, J=8.1Hz, 3H), 10.63 (s, 1H), 12.67 (br s, 1H) |
| VI-21 | Me | 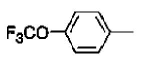 | R | 1.18 (d, J=7.2 Hz, 3H), 3.76-3.86 (m, 1H), 7.56 (d, J=7.8 Hz, 3H), 7.77-7.94 (m, 7H), 8.09-8.12 (m, 2H), 8.18 (d, J=8.1 Hz, 1H), 10.51 (s, 1H), 12.66 (br s, 1H) |

**Table 13**

| Compound No. | R² | R^{A} | * | 1H-NMR (DMSO-d₆) |
|---|---|---|---|---|
| VI-22 | Me | 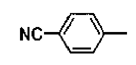 | R | 1.18 (d, J=7.2 Hz, 3H), 3.76-3.83 (m, 1H), 7.78-7.95 (m, 8H), 8.04-8.06 (m, 2H), 8.12-8.15 (m, 2H), 8.18 (d, J=8.4 Hz, 1H), 10.64 (a, 1H), 12.65 (br s, 1H) |
| VI-23 | Me | 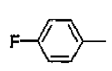 | R | 1.18 (d, J=7.2 Hz, 3H), 3.76-3.86 (m, 1H), 7.39 (t, J=8.7 Hz, 2H), 7.47-7.63 (m, 1H), 7.76-7.94 (m, 7H), 8.04-8.09 (m, 2H), 8.17 (d, J=8.1 Hz, 1H), 10.42 (s, 1H), 12.64 (br s, 1H) |
| YI-24 | Me | 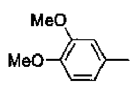 | R | 1.18 (d, J=7.2 Hz, 3H), 3.77-3.82 (m, 1H), 3.85 (s, 3H), 3.86 (s, 3H), 7.10 (d, J=8.4 Hz, 1H), 7.55 (d, J=2.1 Hz, 1H), 7.65 (dd, J=1.8, 8.1 Hz, 1H), 7.75-7.93 (m, 8H), 8.16 (d, J=8.4 Hz, 1H), 10.22 (s, 1H), 12.67 (br s, 1H) |
| VI-25 | Me | 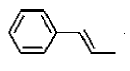 | R | 1.18 (d, J=7.2 Hz, 3H), 3.76-3.85 (m, 1H), 6.86 (d, J=15.6 Hz, 1H), 7.40-7.48 (m, 3H), 7.60-7.65 (m, 3H), 7.74-7.88 (m, 8H), 8.17 (d, J=8.4 Hz, 1H), 10.38 (s, 1H), 12.65 (br s, 1H) |
| VI-26 | Me | 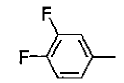 | R | |
| VI-27 | Me | 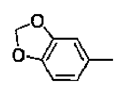 | R | 1.18 (d, J=7.2 Hz, 3H), 3.77-3.79 (m, 1H), 6.14 (s, 2H), 7.07 (d, J=8.1 Hz, 1H), 7.53-7.54 (m, 1H), 7.60 (dd, J=1.5, 8.1 Hz, 1H), 7.74-7.93 (m, 8H), 8.13-8.16 (m, 1H), 10.21 (s, 1H), 12.56 (br s, 1H) |
| VI-28 | i-Bu | 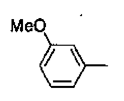 | S | 0.54 (d, J=6.3 Hz, 3H), 0.65 (d, J=6.6 Hz, 3H), 1.23-1.27 (m, 2H), 1.41-1.45(m, 1H), 3.48-3.56 (m, 1H), 3.68 (s, 3H), 7.00 (dd, J=2.7, 8.1 Hz, 1H), 7.27-7.41 (m, 3H), 7.59-7.78 (m, 8H), 8.00 (d, J=8.7 Hz, 1H), 10.20 (s, 1H), 12.43 (br s, 1H) |
| VI-29 | t-Bu | 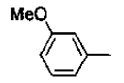 | S | 0.91(s, 9H), 3.45-3.48 (m, 1H), 3.85 (s, 3H), 7.18 (dd, J= 2.7, 8.1 Hz, 1H), 7.44-7.58 (m, 3H), 7.77-7.99 (m, 9H), 10.37 (s, 1H), 12.54 (br s, 1H) |
| VI-30 | Me | 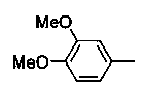 | R | 1.18 (d, J=7.2 Hz, 3H), 3-77-3.82 (m, 1H), 3.85 (s, 3H), 3.86 (s, 3H), 7.10 (d, J= 8.4 Hz, 1H), 7.55 (d, J= 2.1 Hz, 1H), 7.64 (dd, J=2.1, 8.4 Hz, 1H), 7.75-7.93 (m, 8H), 8.16 (d, J=8.4Hz, 1H), 10.22 (s, 1H), 12.67 (br s, 1H) |
| VI-31 | i-Pr | 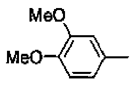 | S | 0.80-0.86 (m, 6H), 1.95 (m, 1H), 3.54 (br, 1H), 3.85 (s, 3H), 3.86 (s, 3H), 7.11 (d, J=8.4 Hz, 1H), 7.55 (d, J=2.1 Hz, 1H), 7.65 (dd, J=1.8, 8.4 Hz, 1H), 7.75-7.93 (m, 8H), 8.02 (br, 1H), 10.23 (s, 1H) |

**Table 14**

| Compound No. | R² | R^{A} | * | 1H-NMR, (DMSO-d₆) |
|---|---|---|---|---|
| VI-32 | i-Bu | 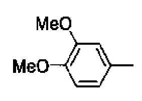 | S | 0.71 (d, J=6.3 Hz, 3H), 0.82 (d, J=6.6 Hz, 3H), 1.38-1.42 (m, 2H), 1.60 (m, 1H), 3.68 (br, 1H), 3.85 (s, 3H), 3.86 (s, 3H), 7.11 (d, J=8.4 Hz, 1H), 7.55 (d, J=2.1 Hz, 1H), 7.65 (dd, J=1.8, 8.4 Hz, 1H), 7.75-7.93 (m, 8H), 8.14 (br, 1H), 10.23 (s, 1H) |
| VI-33 | t-Bu | 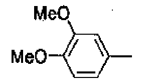 | S | 0.90 (s, 9H), 3.44 (s, 1H), 3.85 (s, 3H), 3.86 (s, 3H), 7.11 (d, J=8.4 Hz, 1H), 7.55 (d, J=2.1 Hz, 1H), 7.65 (dd, J=1.8, 8.4 Hz, 1H), 7.75-7.93 (m, 8H), 10.22 (s, 1H) |
| VI-34 | s-Bu | 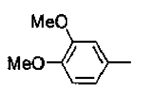 | S | 0.75-0.83 (m, 6H), 1.07-1.17 (m, 1H), 1.34-1.42 (m, 1H), 1.69 (m, 1H), 3.56-3.59 (m, 1H), 3.86 (s, 6H), 7.10 (d, J=8.7 Hz, 1H), 7.48-7.67 (m, 3H), 7.75-7.93 (m, 7H), 8.06 (d, J=9.3 Hz, 1H), 10.22 (s, 1H), 12.59 (br s, 1H) |
| VI-35 | MeS(CH₂)₂- | 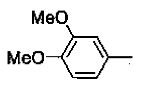 | S | 1.69-1.86 (m, 2H), 1.93 (s, 3H), 2.28-2.46 (m, 2H), 3.86-3.94 (m, 7H), 7.11 (d, J=8.7 Hz, 1H), 7.56 (d, J=2.1 Hz, 1H), 7.65 (dd, J=2.1, 8.4 Hz, 1H), 7.75-7.94 (m, 8H), 8.21 (d, J=8.7 Hz, 1H), 10.22 (s, 1H), 12.74 (br s, 1H) |
| VI-36 | Bn | 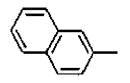 | S | 2.75 (dd, J=9.0, 13.5 Hz, 1H), 2.97 (dd, J=5.1, 13.5 Hz, 1H), 3.92 (br, 1H), 7.10-7.24 (m, 5H), 7.60-7.70 (m, 4H), 7.72-7.81 (m, 4H), 7.96-8.15 (m, 6H), 8.31 (d, J=9.0 Hz, 1H), 10.61 (s, 1H) |
| VI-37 | Me | 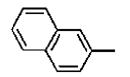 | R | 1.19 (d, J=7.2 Hz, 3H), 3.80 (m, 1H), 7.62-7.69 (m, 2H), 7.79-8.17 (m, 13H), 8.61 (s, 1H), 10.60 (s, 1H), 12.63 (br s, 1H) |
| VI-38 | Me | 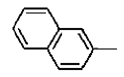 | S | 1.19 (d, J=7.5 Hz, 3H), 3.76-3.86 (m, 1H), 7.62-7.69 (m, 2H), 7.79-7.92 (m, 6H), 7.98-8.20 (m, 7H), 8.62 (s, 1H), 10.60 (s, 1H), 12.66 (br s, 1H) |
| VI-39 | i-Pr | 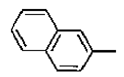 | R | 0.82 (d, J=6.6 Hz, 3H), 0.85 (d, J=6.9 Hz, 3H), 1.91-2.02 (m, 1H), 3.54 (br, 1H), 7.61-7.68 (m ,2H), 7.78-8.13 (m, 14H), 8.61 (s, 1H), 10.60 (s, 1H) |
| VI-40 | i-Pr | 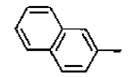 | S | 0.81-0.86 (m, 6H), 1.91-2.02 (m, 1H), 3.55 (m, 1H), 7.61-7.69 (m, 2H), 7.78-7.90 (m, 6H), 7.98-8.12 (m, 7H), 8.61 (s, 1H), 10.59 (s, 1H), 12.62 (br s, 1H) |
| VI-41 | i- Bu | 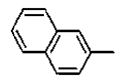 | R | 0.72 (d, J=6.3 Hz, 3H), 0.82 (d, J=6.6 Hz, 3H), 1.38-1.45 (m, 2H), 1.61 (m, 1H), 3.69 (br, 1H), 7.61-7.68 (m ,2H), 7,78-8.13 (m, 14H), 8.16 (br, 1H), 8.61 (s, 1H), 10.60 (s, 1H) |
| VI-42 | i- Bu | 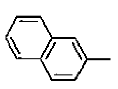 | S | 0.72 (d, J=6.6 Hz, 3H), 0.83 (d, J=6.6Hz, 3H), 1.38 (m, 2H), 1.56-1.63 (m, 1H). 3.66-3.74 (m, 1H), 7.62-7.69 (m, 2H), 7.79-7.91 (m, 6H), 7.98-8.19 (m, 7H), 8.62 (s, 1H), 10.60 (s, 1H), 12.60 (br s, 1H) |

**Table 15**

| Compound No. | R² | R^{A} | * | 1H-NMR (DMSO-d₆) |
|---|---|---|---|---|
| VI-43 | s-Bu | 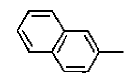 | S | 0.75-0.83 (m, 6H), 1.05-1.17 (m, 1H), 1.36-1.43 (m, 1H), 1.69 (m, 1H), 3.57-3.62 (m, 1H), 7.61-7.69 (m, 2H), 7,78-7,90 (m, 6H), 7.98-8.12 (m, 7H), 8.61 (s, 1H), 10.59 (s, 1H), 12.60 (br s, 1H) |
| VI-44 | MeS(CH₂)₂- | 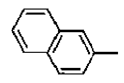 | S | 1.73-1.86 (m, 2H), 1.93 (s, 3H), 2.28-2.44 (m, 2H), 3.88-3.94 (m, 1H), 7.62-7.69 (m, 3H), 7.99-7.92 (m, 6H), 7.98-8.24 (m, 6H), 8.61 (s, 1H), 10.60 (s, 1H), 12.74 (br s, 1H) |
| VI-45 | Bn | 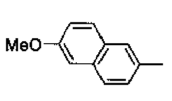 | R | 2.75 (dd, J=9.3, 13.8 Hz, 1H), 2.97 (dd, J=5.7, 13.8 Hz, 1H), 3.91 (m, 1H), 3.93 (s, 3H), 7.10-7.24 (m, 5H), 7.28 (dd, J=2.4, 8.7 Hz, 1H), 7.43 (d, J=2.4 Hz, 1H), 7.62 (d, J=8.4 Hz, 2H), 7.71-7.79 (m, 4H), 7.93-8.05 (m, 5H), 8.31 (d, J=9.0Hz, 1H), 8.54 (s, 1H), 10.50 (s, 1H), 12.76 (br s, 1H) |
| VI-46 | Bn | 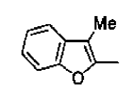 | R | 2.75 (dd, J=9.3, 13.8 Hz, 1H), 2.97 (dd, J=5.7, 13.8 Hz, 1H), 2.63 (s, 3H), 3.92 (m, 1H), 7.11-7.24 (m, 5H), 7.40 (m, 1H), 7.51-7.84 (m, 9H), 8.01 (d, J=8.7 Hz, 2H), 8.32 (d, J=8.7 Hz, 1H), 10.58 (s, 1H), 12.75 (br s, 1H) |
| VI-47 | Bn | 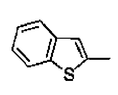 | R | 2.75 (dd, J=9.0, 13.8 Hz, 1H), 2.97 (dd, J=5.7, 13.8 Hz, 1H), 3.91 (m, 1H), 7.10-7.24 (m, 5H), 7.45-7.55 (m, 2H), 7.62 (d, J=8.7 Hz, 2H), 7.70-7.80 (m, 4H), 7.94 (d, J=8.7 Hz, 2H), 8.00-8.11 (m, 2H), 8.30 (d, J=8.4 Hz, 1H), 8.41 (s, 1H), 10.68 (s, 1H), 12.77 (br s, 1H) |
| VI-48 | Bn | 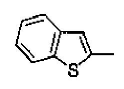 | S | 2.75 (dd, J=9.0, 13.8 Hz, 1H), 2.96 (dd, J=5.1, 13.5 Hz, 1H), 3.90 (brm, 1H), 7.12-7.20 (m, 5H), 7.46-7.54 (m, 2H), 7.62 (d, J=8.4 Hz, 2H), 7.75 (t, J=8.7 Hz, 4H), 7.90 (d, J=12 Hz, 2H), 8.02-8.09 (m, 2H), 8.30 (d, J=9.0 Hz, 1H), 8.41 (s, 1H), 10.68 (s, 1H), 12.79 (br, 1H) |
| VI-49 | t-Bu | 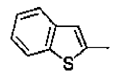 | S | 0.90 (s, 9H), 3.44 (s, 1H), 7.44-7.83 (m, 2H), 7.77-7.93 (m, 8H), 8.01-8.08 (m, 2H), 8.39 (s, 1H), 10.68 (s, 1H) |
| VI-50 | Bn | 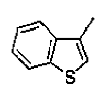 | S | 2.75 (dd, J=9.3, 13.5 Hz, 1H), 2.97 (dd, J=5.4, 13.5 Hz, 1H), 3.92 (m, 1H), 7.11-7.25 (m, 5H), 7.43-7.55 (m, 2H), 7.62 (d, J=8.7 Hz, 2H), 7.71-7.81 (m, 4H), 7.95 (d, J=8.7 Hz, 2H), 8.10 (m, 1H), 8.33 (d, J=8.7Hz, 1H), 8.45 (m, 1H), 8.61 (s, 1H), 10.52 (s, 1H), 12.80 (br s, 1H) |

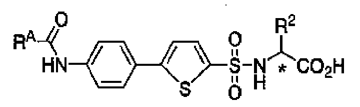

**Table 16**

| Compound No. | R² | R^{A} | * | 1H-NMR (DMSO-de) |
|---|---|---|---|---|
| VI-51 | Bn | 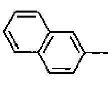 | R | 2.77 (dd, J=9.3, 13.5Hz, 1H), 3.01 (dd, J=5.1, 13.5Hz, 1H), 3.97 (m, 1H), 7.09-7.25 (m, 5H), 7.30 (d, J=3.9 Hz, 1H), 7.37 (d, J=3.9 Hz, 1H), 7.61-7.73 (m, 4H), 7.96 (d, J=9.0 Hz, 2H), 8.00-8.14 (m, 4H), 8.60 (br s, 1H), 8.61 (s, 1H), 10.63 (s, 1H), 12.70 (br s, 1H) |

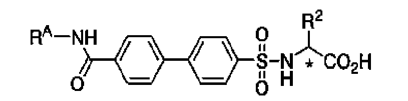

**Table 17**

| Compound No. | R² | R^{A} | * | 1H-NMR(DMSO-d₆) |
|---|---|---|---|---|
| V-1 | Bn | 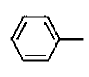 | R | 2.75 (dd, J=9.3, 13.8 Hz, 1H), 2.97 (dd, J=5.7, 13.8 Hz, 1H), 3.92 (m, 1H), 7.09-7.22 (m, 6H), 7.33-7.42 (m, 2H), 7.66 (d, J=8.4 Hz, 2H), 7.77-7.84 (m, 4H), 7.88 (d, J=8.4 Hz, 2H), 8.10 (d, J =8.4 Hz, 2H), 8.36 (d, J=8.7 Hz, 1H), 10.33 (s, 1H), 12.80 (br s, 1H) |

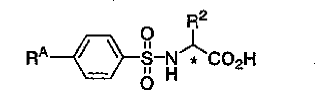

**Table 18**

| Compound No. | R² | R^{A} | * | 1H-NMR (DMSO-d₆) |
|---|---|---|---|---|
| VII-2 | Bn | 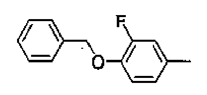 | R | 2.80 (dd, J=7.8, 13.5Hz, 1H), 2.99 (dd, J=6.9, 13.5Hz, 1H), 3.95 (m, 1H), 5.25 (s, 2H), 7.12-7.50 (m, 16H), 7.61 (d, J=12.BHz, 1H), 8.61(br s, 1H), 12.86(br s, 1H) |
| VII-3 | Me | 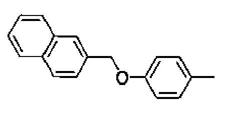 | R | 1.17 (d, J=7.2 Hz, 3H), 3.78 (m, 1H), 5.36 (s, 2H), 7.19 (d, J=8.7 Hz, 2H), 7.49-7.57 (m, 2H), 7.61 (dd, J=1.5, 8.4 Hz, 1H), 7.71 (d, J=8.7 Hz, 2H), 7.81 (s. 4H), 7.88-8.98 (m, 3H), 8.01 (s, 1H), 8.13 (br s, 1H), 12.69 (br s, 1H) |
| VII-4 | Me | 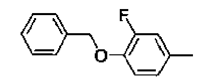 | S | 1.19 (d, J=7.2 Hz, 3H), 3.90 (m, 1H), 5.27 (s, 2H), 7.12-7.45 (m, 16H), 7.61 (d, J=12.6Hz, 1H), 8.59(br s, 1H), 12.85(br s, 1H) |
| VII-5 | Bn | 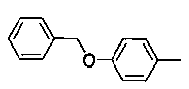 | R | 2.78 (dd, J=7.8, 13.5Hz, 1H), 2.99 (dd, J=6.9, 13.5Hz, 1H), 3.98 (m, 1H), 5.25 (s, 2H), 7.10-7.53 (m, 17H), 7.59 (d, J=12.6Hz, 1H), 8.60(br s, 1H), 12.83(br s, 1H) |
| VII-6 | Me | 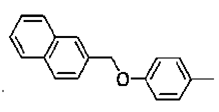 | S | 1.17 (d, J=7.2 Hz, 3H), 3.79 (m, 1H), 5.36 (s, 2H), 7.16 (d, J=8.7 Hz, 2H), 7.50-7.57 (m, 2H), 7.61 (dd, J=1.8, 8.4 Hz, 1H), 7.72 (d, J=8.7 Hz, 2H), 7.82 (s, 4H), 7.90-7.99 (m, 3H), 8.02 (s, 1H), 8.15 (d, J=7.8 Hz, 1H), 12.63 (br s, 1H) |
| VII-7 | s-Bu | 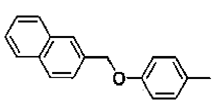 | S | 0.76 (t, J=7.5 Hz, 3H), 0.80 (d, J=6.6 Hz, 3H), 1.10 (m, 1H), 1.36 (m, 1H), 1.67 (m, 1H), 3.57 (m, 1H), 5.36 (s, 2H), 7.19 (d, J=8.4 Hz, 2H), 7.49-7.57 (m, 2H), 7.60 (d, J=8.4 Hz, 1H), 7.71 (d, J=8.7 Hz, 2H), 7.80 (s, 4H), 7.89-7.99 (m, 3H), 8.01 (s, 1H), 8.06 (d, J=8.7 Hz, 1H), 12.60 (br s, 1H) |
| VII-8 | t-Bu | 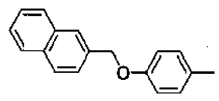 | S | 0.88 (s, 9H), 3.40 (m, 1H), 5.35 (s, 2H), 7.17 (d, J=8.7 Hz, 2H), 7.50-7.53(m, 2H), 7.59 (dd, J=1.5, 8.4 Hz, 1H), 7.69 (d, J=8.7 Hz, 2H), 7.78 (s, 4H), 7.91-8.00 (m, 5H) |
| VII-9 | MeS(CH₂)₂- | 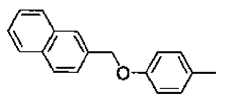 | S | 1.65-1.85 (m, 2H), 1.91 (s, 3H), 2.25-2.44 (m, 2H), 3.88 (m, 1H), 5.36 (s, 2H), 7.19 (d, J=9.0 Hz, 2H), 7.51-7.62 (m, 3H), 7.71 (d, J=8.7 Hz, 2H), 7.77-7.84 (m, 4H), 7.91-8.01 (m, 4H), 8.21 (d, J=8.7 Hz, 1H), 12.79 (br, 1H) |
| VII-10 | Me | 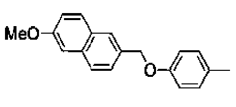 | S | 1.17 (d, J=6.9 Hz, 3H), 3.79 (m, 1H). 3.88 (ms, 3H), 5.30 (s, 2H), 7.14-7.21 (m, 3H), 7.34 (d, J=2.4 Hz, 1H), 7.55 (dd, J=1.8, 8.7 Hz, 1H), 7.71 (d, J=8.7 Hz, 2H), 7.79-7.88 (m, 6H), 7.93 (s, 1H), 8.14 (d, J=8.1 Hz, 1H), 12.65 (br s, 1H) |
| VII-11 | Me | 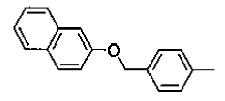 | R | 1.18 (d, J=7.2 Hz, 3H), 3.81 (m, 1H), 5.31 (s, 2H), 7.27 (dd, J=2.4, 9.0 Hz, 1H), 7.36 (m, 1H), 7.43-7.50 (m, 2H), 7.65 (d, J=8.4 Hz, 2H), 7.77-7.92 (m, 9H), 8.20 (br s, 1H), 12.60 (br s, 1H) |

**Table 19**

| Compound No. | R² | R^{A} | * | 1H-NMR (DMSO-d₆) |
|---|---|---|---|---|
| VII-12 | iPr | 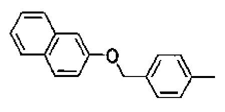 | R | 0.81(d, J=6.9 Hz, 3H), 0.84 (d, J=6.9 Hz, 3H), 1.96 (m, 1H), 3.56 (dd, J=6.0, 8.1 Hz, 1H), 7.27 (dd, J=2.4, 9.0 Hz, 1H), 7.36 (m, 1H), 7.44-7.49 (m, 2H), 7.64 (d, J=8.1 Hz, 2H), 8.07 (d, J=9.6 Hz, 1H), 12.63 (brs, 1H) |

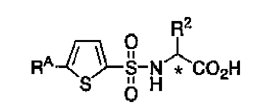

**Table 20**

| Compound No. | R² | R^{A} | * | 1H-NMR (DMSO-d₆) |
|---|---|---|---|---|
| VII-13 | Me | 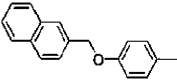 | S | 1.16 (d, J=7.2 Hz, 3H), 3.76 (m, 1H), 7.10 (dd, J=2.4, 8.7 Hz, 1H), 7.26 (d, J=2.4 Hz, 1H), 7.38-7.40 (m, 3H), 7.63-7.65 (m, 3H), 7.72 (dd, J=3.3, 8.7 Hz, 1H). 7.82 (s, 4H), 8.14 (br-s, 1H) |
| VII-14 | iPr | 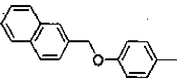 | S | 0.80-0.87 (m, 6H), 1.95-2.03 (m, 1H), 3.56-3.58 (m, 1H), 5.35 (s, 2H), 7.15 (d, J=8.7 Hz, 2H), 7.40 (d, J=3.9 Hz, 1H), 7.47-7.67 (m, 6H), 7.92-8.00 (m, 4H), 8.24 (br s, 1H), 12.64 (br s, 1H) |

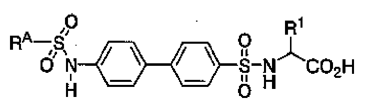

**Table 21**

| Compound No. | R² | R^{A} | * | 1H-NMR (DMSO-d₆) |
|---|---|---|---|---|
| VIII-1 | Bn | 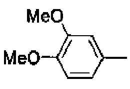 | R | 2.72 (dd, J=9.0, 13.8 Hz, 1H), 2.94 (dd, J=5.7, 13.8 Hz, 1H), 3.76 (s, 3H), 3.78 (s, 3H), 3.87 (m, 1H), 7.04-7.27 (m, 8H), 7.29 (d, J=2.1 Hz, 1H), 7.37 (dd, J=2.1, 8.7 Hz, 1H), 7.53-7.65 (m, 6H), 8.28 (d, J=8.7 Hz, 1H), 10.31 (s, 1H), 12.70 (br s, 1H) |

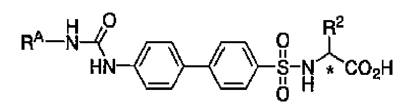

**Table 22**

| Compound No. | R² | R^{A} | * | 1H-NMR (DMSO-d₆) |
|---|---|---|---|---|
| IX-1 | Bn | 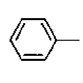 | R | 2.74 (dd, J=9.0, 13.5 Hz, 1H), 2.96 (dd, J=5.4, 13.5 Hz, 1H), 3.90 (m, 1H), 6.98 (t, J=7.2 Hz, 1H), 7.10-7.23 (m, 5H), 7.29 (t, J=7.5 Hz, 1H), 7.47 (d, J=7.8 Hz, 2H), 7.56-7.72 (m, 6H), 8.28 (d, J=7.2 Hz, 1H), 8.76 (s, 1H), 8.89 (s, 1H), 12.70 (br s, 1H) |
| IX-2 | Bn | 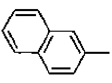 | R | 2.74(d, J=9.3, 13.8 Hz, 1H), 2.96 (dd, J=6.0, 13.8 Hz, 1H), 3.91 (m, 1H), 7.10-7.24 (m, 5H), 7.33-7.73 (m, 11H), 7.78-7.89 (m, 3H), 8.13 (d, J=1.8 Hz, 1H), 8.29 (d, J=8.4Hz, 1H), 8.99 (s, 2H), 12.80 (br s, 1H) |

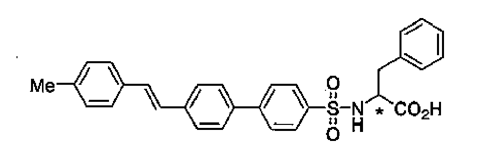

**Table 23**

| Compound , No. | * | 1H-NMR (DMSO-d₆) |
|---|---|---|
| X-1 | R | 2.32 (s, 3H), 2.74 (d, J=8.7, 13.8 Hz, 1H), 2.96 (d, J=5.7, 13.8 Hz, 1H), 3.91 (m, 1H), 7.10-7.37 (m, 9H), 7.54 (d, J=8.1 Hz, 2H), 7.62 (d, J=8.1 Hz, 2H), 7.69-7.79 (m, 6H), 8.33 (d, J=8.4 Hz, 1H), 12.80 (br s, 1H) |
| X-2 | S | 2.31 (s, 3H), 2.74 (d, J=8.8, 13.8 Hz, 1H), 2.95 (d, J=6.0, 13.8 Hz,.1H), 3.91 (m, 1H), 7.10-7.39 (m, 9H), 7.54 (d, J=8.1 Ha, 2H), 7.61 (d, J=8.1 Hz, 2H), 7.70-7.80 (m, 6H), 8.33 (d, J=8.4 Hz, 1H), 12.80 (br s, 1H) |

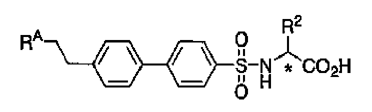

**Table 24**

| Compound No. | R^{A} | R¹ | * | 1H·NMR (DMSO-d₆) |
|---|---|---|---|---|
| XI-1 | Me | 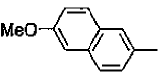 | S | 1.16 (d, J=7.2 Hz, 3H), 3.02 (s, 3H), 3.84 (s, 4H), 7.10 (dd, J=2.4, 8.7 Hz, 1H), 7.26 (d, J=2.4 Hz, 1H), 7.38-7.40 (m, 3H), 9.63-7.65 (m, 3H), 7.72 (dd, J=3.3, 8.7 Hz, 1H), 7.82 (s, 4H), 8.14 (br-s, 1H) |
| XI-2 | iPr | 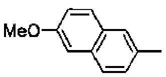 | S | 0.78-0.84 (m, 6H), 1.91 (s, 1H), 3.02 (s, 3H), 3.84 (s, 4H), 7.10 (dd, J=2.4, 8.7 Hz, 1H), 7.26 (d. J=2.4 Hz, 1H), 7.35-7.41 (m, 3H), 7.62-7.64 (m, 3H), 7.72 (dd, J=3.3, 9 Hz, 1H), 7.81 (s, 4H), 8.01 (br-s, 1H) |

Test Example 1 Isolation and Purification ofMMP
MMP-2 was purchased from Calbiachem-Novabiochem International, Inc.
In regard to MMP-8, catalytic domain (⁹⁹Phe~²⁶²Gly) was amplified with PCR using commercial available Human Bone Marrow cDNA. This was cloned in *Escherichia coli* expression vector pTrc99AHE inserted with His-tag sequence and enterokinase digestion-site, induced and expressed by IPTG (Isopropyl-β-D-thiogalactopyranoside) and expressed in a insoluble fraction (Thau F. Ho, M. Walid Qoronfleh, Robert C. Wahl, Trica A. Pulvino, Karen J. Vavra, Joe Falvo, Tracey M. Banks, Patricia G. Brake and Richard B. Ciccarelli: Gene expression, purification and characterization of recombinant human neutrophil collagenase. Gene 146, (1994) 297-301. It was prepared by the method in a slight modification). Isolation of MMP-8 from an insoluble fraction was carried out by dissolving in modifier (6 M urea) by a usual method and purification with metal chelate chromatography. And then removing modifier (6 M urea) with dialysis and refolding of the enzyme spontaneously gave activated MMP-8.
MMP-9 was isolated and purified by the procedure of Y. Okada et al. (Yasunori Okada, Yukio Gonoji, Katsumi Naka, Katsuro Tomita, Isao Nakanishi, Kazushi Iwata, Kyoko Yamashita, and Taro Hayakawa: Matrix metalloproteinase 9 (92-kDa gelatinase/type IV collagenase) from HT1080 human fibrosarcoma cells. Purification and activation of the precursor and enzymic properties, J. Biol. Chem., 1992, 267, 21712-21719) in combination with other following methods: 1) Yasunori Okada, Tatsuhisa Morodomi, Jan J, Enghild, Ko Suzuki, Atsushi Yasui, Isao Nakanishi, Guy Salvesen and Hideaki Nagase: Matrix metalloproteinase 2 from human rheumatoid synovial fibroblasts. Purification and activation of the precursor and enzymic properties, Eur. J. Biochem., 1990, 194, 721-730; 2) Robin V Ward, Rosalind M Hembry, John J Reynolds and Gillian Murphy: The purification of tissue inhibitor of metallaprateinase-2 from its 72 kDa progelatinase complex. Biochem. J., 1991, 278, 179-187. In detail, human fibrosarcoma ATCC HT1080 cell line was cultured to confluent in Dulbecco's Modified Medium (DMEM) containing 10% fetal-calf serum at 37°C for 48 hours. Subsequently, the medium of confluent culture was changed to serum-free DMEM medium. To obtain MMP-9, phorbol-12-myristate-13-acetate (TPA) must be added to this serum-free DMEM medium at a concentration of 50 ng/ml. The TPA-treated medium was centrifuged at 3000 rpm for 15 min, and the supernatant was concentrated to 450 ml by a Toyo-Roshi UP-20 apparatus with an ultrafiltration membrane. Then, proMMP-9 in this concentrated solution was purified by using columns of Gelatin-Sepharose and Concanavalin A-Sepharose. The pool containing proMMP-9 was dialyzed, concentrated (Toyo-Roshi UP-20) and applied to columns of Sephacryl S-200 and Green A matrix for the separation from TIMPs. The obtained proMMP-9 fraction was activated by TPCK-Trypsin (Final conc, 3 µg/50 µl React Mix.).

In regard to MMP-12, catalytic domain (¹⁰⁰Phe~²⁶³Gly) was amplified with RT-PCR from Human Placenta Total RNA. This was cloned in *Escherichia coli* expression vector pTrc99AHE inserted with His-tag sequence and enterokinase digestion-site, induced and expressed by IPTG (Isopropyl-β-D-thiogalactopyranoside) and expressed in an insoluble fraction. Isolation of MMP- 12 from an insoluble fraction was carried out by dissolving in modifier (6 M urea) by a usual method and purification with metal chelate chromatography (Ni Chelateing Sepharose). And then removing modifier (6 M urea) with dialysis and refolding of the enzyme spontaneously gave activated MMP-12.
In regard to MMP-13, mRNA was prepared from carcinoma cell SW1353 derived from human cartilage stimulate by IL-1 and TNF, and catalytic domain (¹⁰⁴Tyr~²⁶⁷Gly) was amplified with RT-PCR. This was cloned in *Escherichia coli* expression vector pTrc99AHE inserted with His-tag sequence and enterokinase digestion-site, induced and expressed by IPTG (Isopropyl-β-D-thiogalactopyranoside) and expressed in an insoluble fraction. Isolation of MMP-13 from an insoluble fraction was carried out by dissolving in modifier (6 M urea) by a usual method and purification with metal chelate chromatography (Ni Chelateing Sepharose). And then removing modifier (6 M urea) with dialyze and refolding of the enzyme spontaneously gave activated MMP-13.

Test example 2 Assay for inhibitory activities on various type of MMPs
The enzymatic activity on MMPs was analyzed by the method described in "C. Graham Knight, Frances Willenbrock and Gillian Murphy: A novel coumarin-labelled peptide for sensitive continuous assays of the matrix metalloproteinases: FEBS LETT., 296, (1992), 263-266". The substrate: MOCAc-Pro-Leu-Gly-Leu-A₂Pr(DNP)-Ala-Arg-NH₂ was purchased from Peptide Institute, Inc., Osaka, Japan. The measurement of the inhibitory activities (IC₅₀) was carried out by the following four methods;
(A) Reaction with substrate, enzyme (MMPs) and inhibitor
(B) Reaction with substrate and inhibitor, without enzyme
(C) Reaction with substrate and enzyme (MMPs), without inhibitor
(D) Reaction with substrate only
   IC₅₀ values were calculated by using the following formula and each fluorescence values of above four methods (A to D).
   % inhibition = {1-(A-B)/(C-D)}× 100
   IC₅₀ means the concentration required to inhibit 50% of the enzyme activity.
   The value of inhibitory activity against MMP-2, MMP-8, MMP-9, MMP-12, and MMP-13 measured by above mentioned methods are shown in Table 25 and 26.

**Table 25**

| Compound No. | MMP inhibitory activity IC₅₀ (µM) | | | | |
|---|---|---|---|---|---|
| | MMP-13 | MMP-2 | MMP-8 | MMP-9 | MMP-12 |
| III-3 | 0.00786 | > 10 | 0.0563 | > 10 | 0.0709 |
| III-4 | 0.00454 | 0.0726 | 0.0401 | > 10 | 0.0474 |
| III-6 | 0.0151 | 0.499 | 0.261 | > 10 | 0.363 |
| III-7 | 0.00432 | 0.327 | 0.0131 | > 10 | 0.0202 |
| III-10 | 0.00286 | 0.374 | 0.0259 | > 10 | 0.0297 |
| III-11 | 0.00992 | 0.163 | 0.128 | > 10 | 2.00 |
| III-12 | 0.0357 | 8.991 | 0.270 | > 10 | 0.888 |
| III-15 | 0.225 | > 10 | 7.683 | > 10 | > 10 |
| III-17 | 0.00560 | > 10 | > 10 | > 10 | > 10 |
| III-18 | 0.0199 | > 10 | > 10 | > 10 | > 10 |
| III-20 | 0.0150 | 0.167 | 0.154 | > 10 | 0.128 |
| 111-21 | 0.00573 | > 4 | 0.0369 | > 10 | 0.0583 |
| III-22 | 0.0557 | > 10 | 5.710 | > 10 | 9.678 |
| III-23 | 0.0163 | 0.602 | 0.0416 | > 10 | 0.0507 |
| III-24 | 0.00632 | 0.239 | 0.190 | > 10 | 0.146 |
| III-25 | 0.0242 | 2.852 | 0.0893 | > 10 | 0.117 |
| m-26 | 0.00385 | 5.413 | 0.519 | > 10 | > 10 |
| III-27 | 0.00271 | 0.0589 | 0.0333 | >10 | 0.0368 |
| III-28 | 0.0157 | > 5 | > 5 | > 5 | 2.204 |
| III-29 | 0.0788 | > 10 | 5.302 | > 10 | > 10 |
| III-31 | 0.0201 | >10 | 4.455 | > 10 | 0.901 |
| III-44 | 0.00349 | 5.01 | >10 | >10 | 5.294 |
| III-45 | 0.0193 | >10 | >10 | >10 | >10 |
| III-46 | 0.00912 | 0.0575 | | | |
| III-47 | 0.143 | >10 | >10 | >10 | >10 |
| III-48 | 0.100 | >10 | >10 | >10 | >10 |
| III-49 | 0.0508 | >10 | 0.109 | >10. | 0.118 |
| III-50 | 0.0138 | 0.0506 | 0.243 | >10 | 0.125 |
| III-51 | 0.128 | >10 | 0.508 | >10 | 0.536 |
| III-54 | 0.0113 | 0.147 | | | |
| III-55 | 0.0109 | 0.494 | | | |
| III-56 | 0.00605 | 0.0657 | | | |
| III-60 | 0.0978 | 1.17 | | | |
| III-61 | 0.00617 | 0.349 | | | |
| III-62 | 0.0189 | 0.402 | | | |
| III-63 | 0.0095 | 0.144 | | | |
| III-64 | 0.0211 | 0.261 | | | |
| III-65 | 0.172 | >10 | | | |
| III-66 | 0.0424 | >10 | | | |
| VI-2 | 0.0736 | 0.371 | > 10 | > 10 | > 10 |
| VI-3 | 0.0326 | 0.239 | > 10 | > 10 | 1.103 |
| VI-5 | 0.0501 | 0.660 | 0.623 | > 10 | 1.228 |
| VI-6 | 0.0394 | 0.235 | > 10 | > 10 | > 10 |
| VI-7 | 0.0333 | 0.156 | 2.353 | 4.338 | 0.611 |
| VI-8 | 0.0272 | 1.049 | 0.723 | > 10 | 0.839 |
| VI-9 | 0.0446 | 0.178 | > 10 | > 10 | > 10 |
| VI-10 | 0.0214 | >10 | >10 | >10 | >10 |

**Table 26**

| Compound No. | MMP inhibitory activity IC₅₀(µM) | | | | |
|---|---|---|---|---|---|
| | MMP-13 | MIO-2 | MMP-8 | MMP-9 | MMP-12 |
| VI-11 | 0.0388 | 0.139 | >10 | >10 | 9.008 |
| VI-12 | 0.0311 | 0.197 | 1.742 | >10 | 0.334 |
| VI-13 | 0.0272 | 0.167 | 0.641 | >10 | 0.503 |
| VI-15 | 0.0555 | 0.222 | >10 | >10 | 0.801 |
| VI-16 | 0.0771 | 0.553 | 1.096 | >10 | 1.024 |
| VI-17 | 0.0356 | 0.127 | >10 | >10 | 0.952 |
| VI-21 | 0.0368 | 0.105 | >10 | >10 | 0.522 |
| VI-24 | 0.0501 | 0.839 | 1.517 | >10 | 0.443 |
| VI-30 | 0.0501 | 0.839 | 1.517 | >10 | 0.443 |
| VI-35 | 0.0687 | 2.22 | 0.215 | >10 | 0.232 |
| VI-36 | 0.0651 | >10 | >10 | >10 | 7.974 |
| VI-37 | 0.0381 | 0.726 | >10 | >10 | >10 |
| VI-39 | 0.0811 | 0.664 | 5.401 | >10 | >10 |
| VI-41 | 0.1785 | >10 | >10 | >10 | >10 |
| VI-43 | 0.0137 | 0.243 | 0.0632 | >10 | 0.0669 |
| VI-44 | 0.0484 | 0.502 | 0.252 | >10 | 0.162 |
| VI-45 | 0.0613 | >10 | >10 | >10 | >10 |
| VI-46 | 0.0965 | 0.747 | | | |
| VI-47 | 0.136 | >10 | >10 | >10 | >10 |
| VI-48 | 0.0473 | >10 | | | |
| VI-49 | 0.01 | 0.241 | 0.158 | >10 | 0.0988 |
| VI-51 | 0.0222 | 0.292 | >10 | >10 | >10 |
| VII-8 | 0.00793 | 0.0910 | | | |
| VII-9 | 0.0265 | 0.188 | | | |
| VII-10 | 0.0165 | >10 | | | |
| VII-11 | 0.0162 | >10 | | | |
| VII-12 | 0.0262 | 3.72 | 0.14 | >10 | 0.0957 |
| VII-13 | 0.119 | >10 | >10 | >10 | >10 |
| XI-1 | 0.0124 | >10 | | | |

Test example 3 The inhibitory activities of the compounds on the disease progression in the rat osteoarthritis model.
Rat osteoarthritis model was prepared according to the procedure reported by Meacock et al. (J. Exp. Path. 71, 279-293, 1990), by medial meniscectomy and collateral ligament transection on the right knee of 12 weeks old female SD rats (Charles River Japan). 0.5% methylcellulose or compounds (II-3) or (III-11) (10 mg/kg) were orally administered twice daily from the following day of the surgery for 6 weeks.
On the next day of final administration, tibial plateaus were removed. The surfaces of the tibial plateau were stained with India ink (Kuretake), and then photographed using a digital camera (Nikon). The progressive level of OA lesion was scored using a photographic image thereof. As a result of evaluation, the compounds (II-3) and (III-11) showed 56.9 and 77.2% of inhibitory activities, respectively.
This result showed that the compounds (II-3) and (III-11) were effective on the treatment of osteoarthritis.

### Formulation example

Formulation example 1
Granules are prepared using the following ingredients.

| | | |
|---|---|---|
| Ingredients | Active ingredient | 10 mg |
| | Lactose | 700 mg |
| | Corn starch | 274 mg |
| | HPC-L | 16 mg |
| | | 1000 mg |

The active ingredient and lactose are made pass through a 60 mesh sieve. Corn starch is made pass through a 120 mesh sieve. They are mixed by a twin shell blender. An aqueous solution of HPC-L (low mucosity hydroxypropylcellulose) is added to the mixture and the resulting mixture is kneaded, granulated (by the extrusion with pore size 0.5 to 1 mm mesh), and dried. The dried granules thus obtained are sieved by a swing sieve (12/60 mesh) to yield the granules.

Formulation example 2
Powders for filling capsules are prepared using the following ingredients.

| | | |
|---|---|---|
| Ingredients | Active ingredient | 10 mg |
| | Lactose | 79 mg |
| | Corn starch | 10 mg |
| | Magnesium stearate | 1 mg |
| | | 100 mg |

The active ingredient and lactose are made pass through a 60 mesh sieve. Corn starch is made pass through a 120 mesh sieve. These ingredients and magnesium stearate are mixed by a twin shell blender. 100 mg of the 10-fold trituration is filled into a No. 5 hard gelatin capsule.

Formulation example 3
Tablets are prepared using the following ingredients.

| | | |
|---|---|---|
| Ingredients | Active ingredient | 10 mg |
| | Lactose | 90 mg |
| | Microcrystal cellulose | 30 mg |
| | CMC-Na | 15 mg |
| | Magnesium stearate | 5 mg |
| | | 150 mg |

The active ingredient, lactose, microcrystal cellulose, and CMC-Na (carboxymethylcellulose sodium salt) are made pass through a 60 mesh sieve and then mixed. The resulting mixture is mixed with magnesium stearate to obtain the mixed powder for the tablet formulation. The mixed powder is compressed to yield tablets of 150 mg.

Formulation Example 4
An aerosol solution is prepared containing the following components:

| | Weight |
|---|---|
| Active ingredient | 0.25 |
| Ethanol | 25.75 |
| Propellant 22 (chlorodifluoromethane) | 74.00 |
| Total | 100.00 |

The active compound is mixed with ethanol and the admixture added to a portion of the propellant 22, cooled to -30 °C and transferred to filling device. The required amount is then fed to stainless steel container and diluted with the reminder of the propellant. The valve units are then fitted to the container.

Formulation Example 5
An intravenous formulation may be prepared as follows:

| | |
|---|---|
| Active ingredient | 100 mg |
| Saline | 1000 mL |

The solution of the above ingredients generally is administered intravenously to a subject at a rate of 1 mL per minute.

Formation example 6
Endermatic formulation is prepared using the following ingredients.

| | |
|---|---|
| Active ingredient | 10 mg |
| Isopropyl myristate | 990 mg |
| | 1000 mg |

The compound represented by the formula (I) is dispersed in isopropyl myristate. The mixture is mixed with acrylic adhesive formulation (e.g. nikazole) and is attached plastered to a support to yield endermatic formulation.

Formulation example 7
Ointment was prepared using the following ingredients.

| | |
|---|---|
| Active ingredient | 10 mg |
| Liquid paraffin | 75 mg |
| White petrolatum | 925 mg |
| | 1000 mg |

The compound represented by the formula (I) is dispersed in liquid paraffin and kneaded with white petrolatum to yield the ointment.

### Industrial Applicability

. The sulfonamide derivatives having selective inhibitory activities against the MMP-13 of the present invention are useful for the treating or preventing agent for diseases related to MMP-13, particularly osteoarthritis

## Claims

1. A compound represented by the general formula (I): 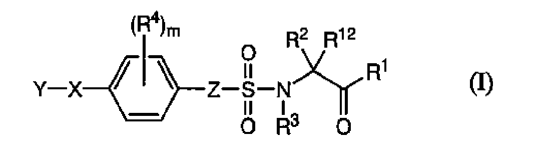 wherein Z is 1,4-phenylene or thiophen-2,5-diyl;
R¹ is hydroxy, lower alkyloxy, or -NHOH;
R² is hydrogen atom, optionally substituted lower alkyl, optionally substituted aralkyl, optionally substituted heteroarylalkyl, optionally substituted aryl, or optionally substituted heteroaryl;
R¹² is hydrogen atom; or
R² and R¹² taken together with the adjacent carbon atom may form a 5 to 6 membered non-aromatic carbocyclic group or a 5 to 6 membered non-aromatic heterocyclic group;
R³ is hydrogen atom, optionally substituted lower alkyl, optionally substituted aralkyl, optionally substituted heteroarylalkyl, optionally substituted aryl, or optionally substituted heteroaryl;
R⁴ is halogen, lower alkyl, cycloalkyl, lower alkenyl, lower alkynyl, lower alkyloxy, lower alkenyloxy, lower alkylthio, halo(lower)alkyl, halo(lower)alkyloxy, halo(lower)alkylthio, hydroxy, hydroxy(lower)alkyl, mercapto, carboxy, lower alkyloxycarbonyl, lower alkylsulfonyl, acyl, acyloxy, nitro, cyano, optionally substituted amino, or optionally substituted aminocarbonyl;
m is 0, 1, or 2;
X is a bond, -CH₂CH₂-, -CH=CH-, -C≡C-, -(CH₂)p¹-C≡C-, -X¹-C(=O)-N(R⁵)-, -X²-N(R⁶)-C(=O)-, -X³-O-X⁴-, -SO-N(R⁹)-, or -N(R¹⁰)-C(=O)-N(R¹¹)-;
X¹ and X² are each independently a bond, -CH=CH-, or -C≡C-;
X³ and X⁴ are each independently a bond or -(CH₂)p²-;
R⁵, R⁶, R⁹, R¹⁰, and R¹¹ are each independently hydrogen atom or lower alkyl;
p¹ and p² are each independently an integer from 1 to 5;
Y is optionally substituted aryl, optionally substituted heteroaryl, an optionally substituted non-aromatic carbocyclic group, or an optionally substituted non-aromatic heterocyclic group;
provided Y is not unsubstituted phenyl when R² is isopropyl or benzyl and X is a bond; Y is not unsubstituted naphthyl when R² is methyl or isopropyl and X is a bond; and Y is not unsubstituted naphthyl when R² is benzyl and X is -C=C-;
its optically active substance, their pharmaceutically acceptable salt, or a solvate thereof.

2. A compound according to claim 1 wherein the compound is represented by the general formula (II): 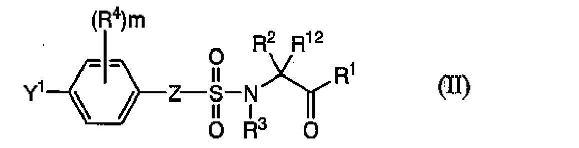 wherein R¹, R², R³, R⁴, R¹², Z, and m are as defined in claim 1;
Y¹ is a group represented by the formula: 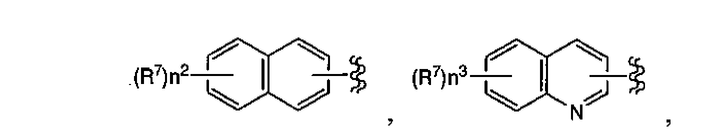 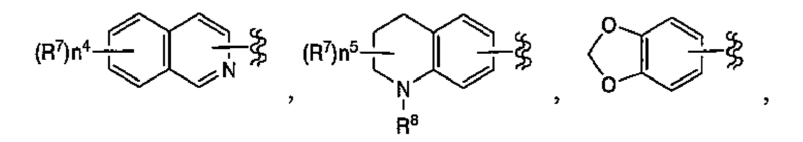 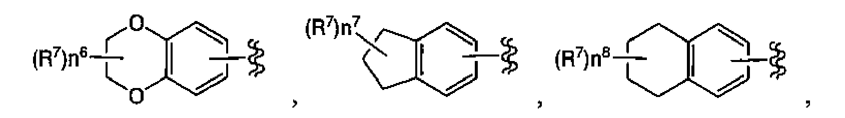 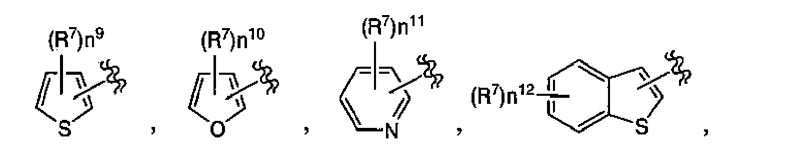 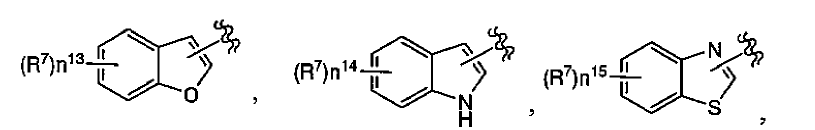 or 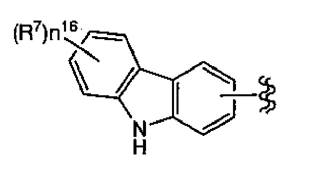 wherein R⁷ is halogen, lower alkyl, lower alkyloxy, cycloalkyl, lower alkenyl, lower alkynyl, lower alkenyloxy, lower alkylthio, halo(lower)alkyl, halo(lower)alkyloxy, halo(lower)alkylthio, hydroxy, hydroxy(lower)alkyl, mercapto, carboxy, lower alkyloxycarbonyl, lower alkylsulfonyl, acyl, acyloxy, nitro, cyano, optionally substituted amino, optionally substituted aminocarbonyl, optionally substituted aryl, optionally substituted heteroaryl, optionally substituted aryloxy, optionally substituted aralkyloxy, lower alkyloxy(lower)alkyl, optionally substituted aryloxy(lower)alkyl, optionally substituted ureide, optionally substituted pyrolizinyl, optionally substituted morpholinyl, or optionally substituted piperidinyl,;
R⁸ is hydrogen atom or lower alkyl;
n² to n¹⁶ are each independently an integer from 0 to 3;
provided n² is an integer from 1 to 3 when R² is methyl or isopropyl,
its optically active substance, their pharmaceutically acceptable salt, or a solvate thereof.

3. A compound according to claim 1 wherein the compound is represented by the general formula (III): 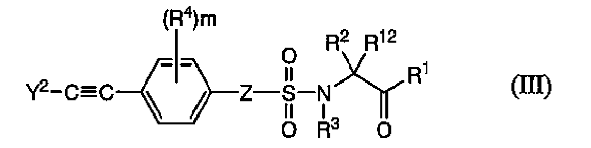 wherein R¹, R², R³, R⁴, R¹², Z, and m are as defined in claim 1;
Y² is a group represented by the formula: 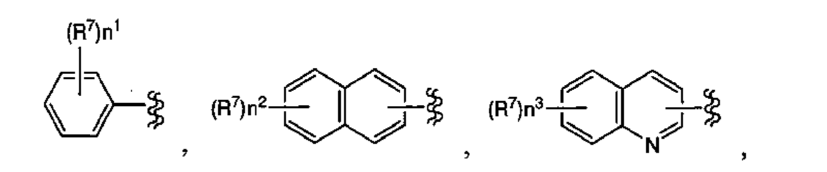 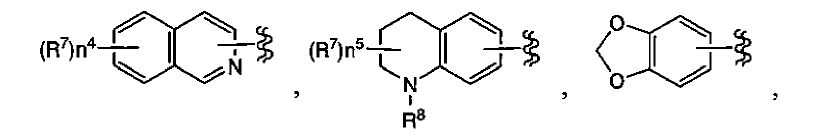 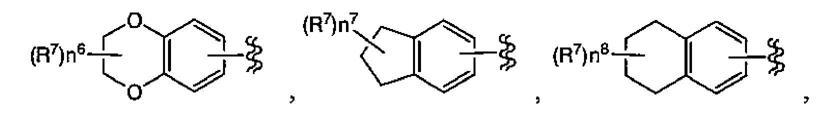 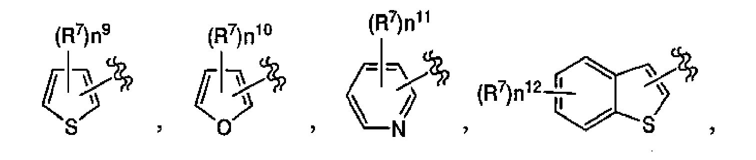 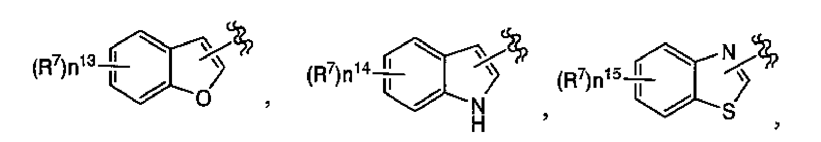 or 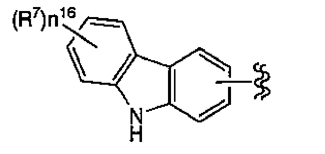 wherein R⁷, R⁸, and n¹ to n¹⁶ are as defined in claim 2;
provided n² is an integer from 1 to 3 when R² is benzyl,
its optically active substance, their pharmaceutically acceptable salt, or a solvate thereof.

4. A compound according to claim 1 wherein the compound is represented by the general formula (IV): 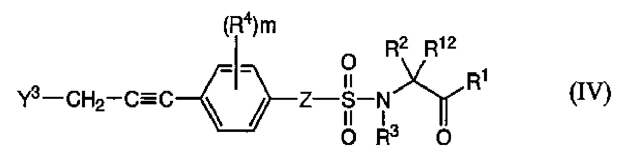 wherein R¹, R², R³, R⁴, R¹², Z, and m are as defined in claim 1;
Y³ is a group represented by the formula: 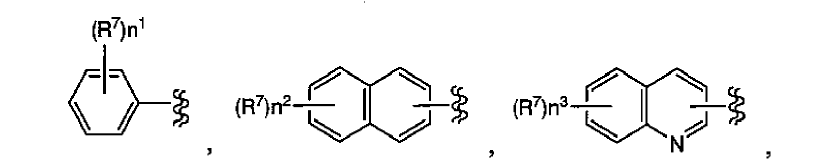 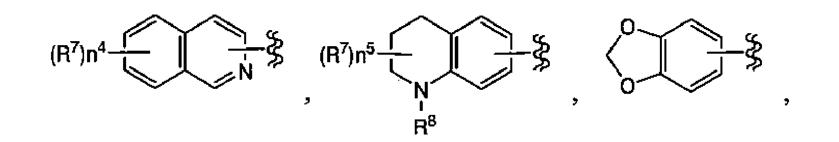 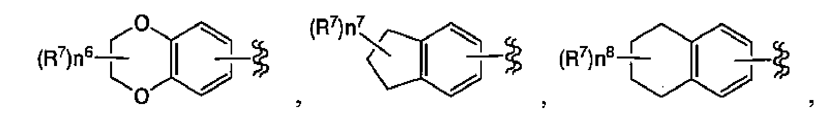 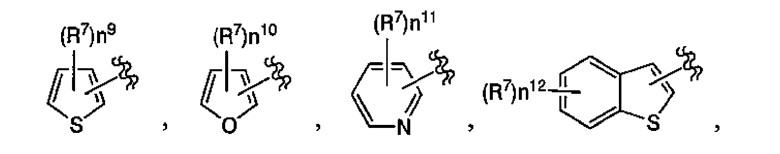 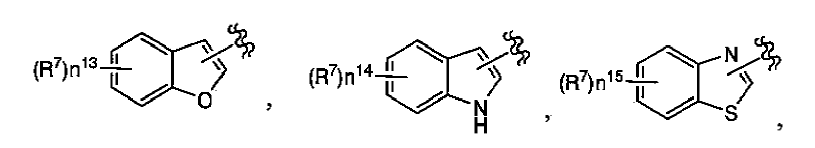 or 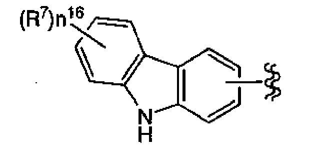 wherein R⁷, R⁸, and n¹ to n¹⁶ are as defined in claim 2,
its optically active substance, their pharmaceutically acceptable salt, or a solvate thereof.

5. A compound according to claim 1 wherein the compound is represented by the general formula (V): 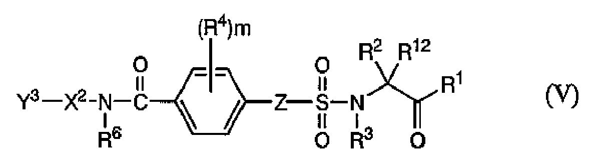 wherein R¹, R², R³, R⁴, R⁶, R¹², X², Z, and m are as defined in claim 1;
and Y³ is as defined in claim 4,
its optically active substance, their pharmaceutically acceptable salt, or a solvate thereof.

6. A compound according to claim 1 wherein the compound is represented by the general formula (VI): 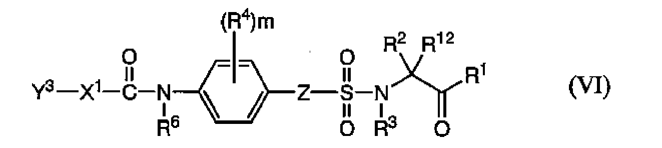 wherein R¹, R², R³, R⁴, R⁶, R¹², X¹, Z, and m are as defined in claim 1;
and Y³ is as defined in claim 4,
its optically active substance, their pharmaceutically acceptable salt, or a solvate thereof.

7. A compound according to claim 1 wherein the compound is represented by the general formula (VII): 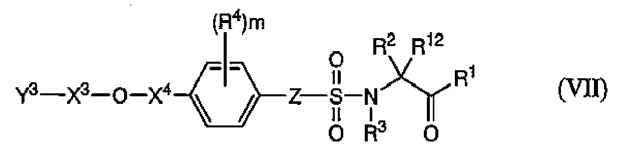 wherein R¹, R², R³, R⁴, R¹², X³, X⁴, Z, and m are as defined in claim 1;
and Y³ is as defined in claim 4,
its optically active substance, their pharmaceutically acceptable salt, or a solvate thereof

8. A compound according to claim 1 wherein the compound is represented by the general formula (VIII): 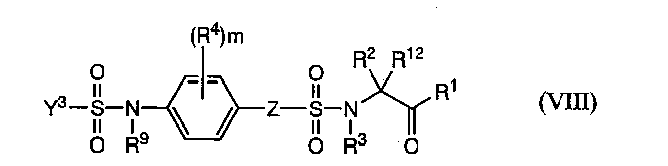 wherein R¹, R², R³, R⁴, R⁹, R¹², Z, and m are as defined in claim 1;
and Y³ is as defined in claim 4,
its optically active substance, their pharmaceutically acceptable salt, or a solvate thereof

9. A compound according to claim 1 wherein the compound is represented by the general formula (IX): 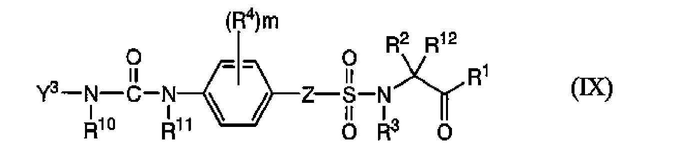 wherein R¹, R², R³, R⁴, R¹⁰, R¹¹, R¹², Z, and m are as defined in claim 1;
and Y³ is as defined in claim 4,
its optically active substance, their pharmaceutically acceptable salt, or a solvate thereof

10. A compound according to claim 1 wherein the compound is represented by the general formula (X): 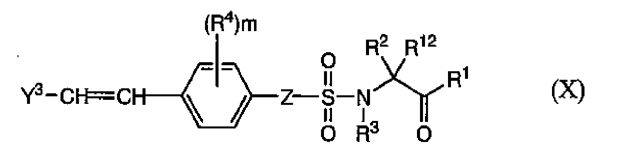 wherein R¹, R², R³, R⁴, R¹², Z, and m are as defined in claim 1;
and Y³ is as defined in claim 4,
its optically active substance, their pharmaceutically acceptable salt, or a solvate thereof

11. A compound according to claim 1 wherein the compound is represented by the general formula (XI): 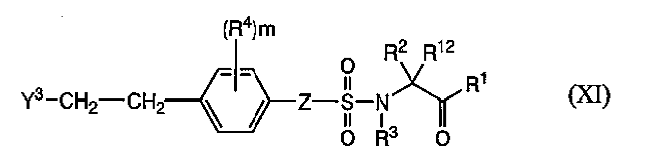 wherein R¹, R², R³, R⁴, R¹², Z, and m are as defined in claim 1;
and Y³ is as defined in claim 4,
its optically active substance, their pharmaceutically acceptable salt, or a solvate thereof

12. A compound according to any one of claims 1 to 11 wherein R¹ is hydroxy, its optically active substance, their pharmaceutically acceptable salt, or a solvate thereof.

13. A compound according to any one of claims 1 to 12 wherein R² is hydrogen atom, methyl, ethyl, n-propyl, isopropyl, n-butyl, s-butyl, isobutyl, t-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, indol-3-ylmethyl, thiazol-4-ylmethyl, carboxymethyloxybenzyl, or cyclopropylmethyh
R¹² is hydrogen atom; or
R² and R¹² taken together with the adjacent carbon atom may form a group represented by the formula: 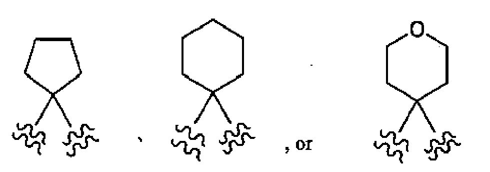 its optically active substance, their pharmaceutically acceptable salt, or a solvate thereof.

14. A compound according to any one of claims 1 to 13 wherein R³ is hydrogen atom, its optically active substance, their pharmaceutically acceptable salt, or a solvate thereof.

15. A pharmaceutical composition which contains a compound of any one of claims 1 to 14 as an active ingredient.

16. A method for treating a disease caused by or related to MMP-13 of a mammal comprising administering to said mammal including human a therapeutically effective amount of a compound of any one of claims 1 to 14.

17. Use of a compound of any one of claims 1 to 14 for the preparation of a medicament for treating a disease caused by or related toMMP-13.
